# FASCICULE DE BREVET EUROPEEN

(11) **EP 2 102 204 B1**
(45) Date de publication et mention de la délivrance du brevet: **05.05.2010**
(21) Numéro de dépôt: 08761737.9
(22) Date de dépôt: 04.01.2008
(51) Int. Cl.: C07D 471/04, C07D 471/14, C07D 471/16, A61K 31/5025, A61K 31/4375, A61P 25/22, A61P 25/24

(54) **NOUVEAUX DÉRIVÉS AMINOPYRROLO[1,2- A]INDOLE ET AMINOPYRIDAZINO[1,6- A]INDOLE, LEUR PROCÉDÉ DE PRÉPARATION ET LES COMPOSITIONS PHARMACEUTIQUES QUI LES CONTIENNENT**
NEUE AMINOPYRROLO-[1,2-A]-INDOL- UND AMINOPYRIDAZINO-[1,6-A]-INDOL-DERIVATE, VERFAHREN ZU IHRER HERSTELLUNG UND PHARMAZEUTISCHE ZUSAMMENSETZUNGEN DAMIT
NEW AMINOPYRROLO[1,2- A]INDOLE ET AMINOPYRIDAZINO[1,6-A]INDOLE DERIVATIVES, METHOD FOR PREPARING THE SAME AND PHARMACEUTICAL COMPOSITIONS CONTAINING THE SAME

(30) Priorité: 05.01.2007 FR 0700047
(43) Date de publication de la demande: 23.09.2009
(73) Titulaire: Les Laboratoires Servier, 92284 Suresnes Cedex (FR); Centre National de la Recherche Scientifique, 75794 Paris Cedex 16 (FR); Université de Paris-Sud, 91405 Orsay Cedex (FR)
(72) Inventeur: BRION, Jean-Daniel, F-95320 Saint Leu la Fôret (FR); GALTIER, Christophe, F-94600 Choisy-le-Roi (FR); HERVET, MAUD, F-92330 Sceaux (FR); LE STRAT, Frédéric, F-27180 Saint Sébastien de Morsent (FR); MOREAU, Anne, 22300 Lannion (FR); RENKO, Zafiarisoa, Dolor, F-91940 Les Ulis (FR); LE RIDANT, Alain, F-92200 Neuilly Sur Seine (FR); HARPEY, Catherine, F-75016 Paris (FR)
(86) Numéro de dépôt international: PCT/FR2008/000013
(87) Numéro de publication internationale: WO 2008/099082

(56) Documents cités:
- EP-A1- 0 658 557
- FR-A1- 2 713 643
- MELNYK P ET AL: "Synthesis of New Annelated Indole Systems: New Entry in the E-azaeburnane series." TETRAHEDRON, vol. 51, no. 7, 1995, pages 1941-1952, XP002439190
- SIUTA J G ET AL: "Studies Directed toward a Mitomycin Synthesis" J. ORG. CHEM., vol. 39, no. 25, 1974, pages 3739-3744, XP002439192
- DATABASE CAPLUS CHEMICAL ABSTRACTS SERVICE, COLUMBUS, OHIO, US; XP002439595 extrait de STN accession no. 1982:163001 Database accession no. 96:163001 & HETEROCYCLES, vol. 19, no. 1, 1982, pages 83-87,

## Description

La présente invention concerne de nouveaux dérivés aminopyrrolo[1,2-*a*]indole et aminopyridazino[1,6-*a*]indole, leur procédé de préparation et les compositions pharmaceutiques qui les contiennent.

La littérature fournit de nombreux exemples de composés possédant une structure éburnane. C'est le cas notamment du brevet US 3 454 583 traitant de la vincamine ((3α,14β,16αa)-(14,15-dihydro-14-hydroxy-éburnaménine-14-carboxylate de méthyle) et de ses dérivés pour leurs propriétés vasodilatatrices. Les demandes de brevets FR 2 433 528 et FR 2 381 048 présentent de nouveaux dérivés de 20,21-dinorébumaménine et la demande EP 0 287 468, de nouveaux dérivés des 17-aza-20,21-dinoréburnaménine. La demande de brevet EP 0 658 557 décrit des dérivés de l'éburnane modifiés en position 14 et 15 du squelette éburnane. La demande de brevet EP 0 563 916 décrit des dérivés de 1*H*-indole-cyclohexanecarboxamide.

Les composés de la présente invention outre le fait qu'ils soient nouveaux, présentent des propriétés pharmacologiques très intéressantes. Ils se révèlent notamment être de puissants inducteurs de tyrosine hydroxylase, de manière sélective ou non.

Plus particulièrement, la présente invention concerne les composés de formule (I) : 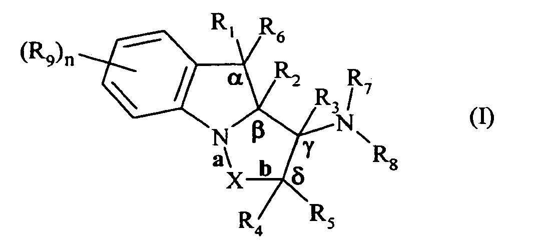 dans laquelle :
- X représente CO ou 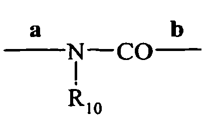 R₁ représente hydrogène, alkyle (C₁-C₆) linéaire ou ramifié, aminoalkyle (C₁-C₆) linéaire
   ou ramifié, hydroxyalkyle (C₁-C₆) linéaire ou ramifié, arylalkyle (C₁-C₆) linéaire ou ramifié,
   R₂ représente hydrogène, alkyle (C₁-C₆) linéaire ou ramifié, aminoalkyle (C₁-C₆) linéaire
   ou ramifié, hydroxyalkyle (C₁-C₆) linéaire ou ramifié,
   ou R₁ et R₂ ensemble avec les atomes de carbone qui les portent forment une liaison carbone carbone,
   R₃ représente hydrogène, alkyle (C₁-C₆) linéaire ou ramifié, aminoalkyle (C₁-C₆) linéaire
   ou ramifié, hydroxyalkyle (C₁-C₆) linéaire ou ramifié,
   R₄ représente hydrogène, alkyle (C₁-C₆) linéaire ou ramifié, aminoalkyle (C₁-C₆) linéaire
   ou ramifié, hydroxyalkyle (C₁-C₆) linéaire ou ramifié,
   ou R₃ et R₄ ensemble avec les atomes de carbone qui les portent forment une liaison carbone carbone,
   R₅ représente hydrogène, alkyle (C₁-C₆) linéaire ou ramifié,
   R₆ représente hydrogène, alkyle (C₁-C₆) linéaire ou ramifié,
   R₇, R₈ représentent un atome d'hydrogène, un groupement alkyle (C₁-C₆) linéaire ou ramifié, arylalkyle (C₁-C₆) linéaire ou ramifié, alkényle (C₂-C₆) linéaire ou ramifié, alkynyle (C₂-C₆) linéaire ou ramifié, une chaîne alkyle (C₁-C₆) linéaire ou ramifiée substituée par un ou plusieurs hydroxy, cyano, alkoxy (C₁-C₆) linéaire ou ramifié, NR₁₃R₁₄, une chaîne alkényle (C₁-C₆) linéaire ou ramifiée substituée par les mêmes substituants que ceux définis pour la chaîne alkyle ou, une chaîne alkynyle (C₁-C₆) linéaire ou ramifiée substituée par les mêmes substituants que ceux définis pour la chaîne alkyle,
   ou,
   soit R₅ et R₈ ensemble avec les atomes de carbone et d'azote qui les portent, forment un hétérocycle à 5, 6 ou 7 chaînons, éventuellement substitué par un groupement R₁₂,
   soit R₆ et R₇ ensemble avec les atomes de carbone et d'azote qui les portent, forment un hétérocycle à 5, 6 ou 7 chaînons, éventuellement substitué par un groupement R₁₁,
   étant entendu que nécessairement un, mais un seul des deux groupements "R₅ et R₈" ou "R₆ et R₇" ensemble avec les atomes de carbone et d'azote qui les portent, forment un hétérocycle à 5, 6 ou 7 chaînons, éventuellement substitué par un groupement R₁₁,
   R₉ représente hydrogène, halogène, alkyle (C₁-C₆) linéaire ou ramifié, alkoxy (C₁-C₆) linéaire ou ramifié, hydroxy, cyano, nitro, polyhalogénoalkyle (C₁-C₆) linéaire ou ramifié, NR₁₅R₁₆, ou une chaîne alkyle (C₁-C₆) linéaire ou ramifiée substituée par un ou plusieurs halogène, un ou plusieurs hydroxy, alkoxy (C₁-C₆) linéaire ou ramifié, ou NR₁₅R₁₆,
   n représente un entier 0, 1, 2, 3 ou 4,
   R₁₀ représente hydrogène, alkyle (C₁-C₆) linéaire ou ramifié, alkényle (C₂-C₆) linéaire ou ramifié, arylalkyle (C₁-C₆) linéaire ou ramifié, polyhalogénoalkyle (C₁-C₆) linéaire ou ramifié, ou une chaîne alkyle (C₁-C₆) linéaire ou ramifié substituée par un ou plusieurs atomes d'halogène, un ou plusieurs groupements hydroxy, alkoxy (C₁-C₆) linéaire ou ramifié, ou NR₁₅R₁₆,
   R₁₁, R₁₂, identiques ou différents, représentent un groupement -COOT ou -CH₂O-U dans lesquels T et U, identiques ou différents, représentent un atome d'hydrogène ou un groupement alkyle (C₁-C₆) linéaire ou ramifié,
   R₁₃, R₁₄, identiques ou différents, représentent un atome d'hydrogène ou un groupement alkyle (C₁-C₆) linéaire ou ramifié ou, forment ensemble avec l'atome d'azote qui les porte, un hétérocycle de 4 à 8 chaînons éventuellement substitué, contenant éventuellement une double liaison au sein de l'hétérocycle et contenant éventuellement au sein du système cyclique un second hétéroatome choisi parmi l'atome d'oxygène et l'atome d'azote,
   R₁₅, R₁₆, identiques ou différents, représentent un atome d'hydrogène ou un groupement alkyle (C₁-C₆) linéaire ou ramifié, alkényle (C₂-C₆) linéaire ou ramifié,
   leurs énantiomères, diastéréoisomères, N-oxydes, ainsi que leurs sels d'addition à un acide
   ou à une base pharmaceutiquement acceptable,
   étant entendu que :
   - la (3a*SR*,4*SR*)-3-benzyl-4-éthyl-2,3,3a,4-tétrahydrobenzo[*b*]pyrido[2,3,4-*gh*]pyrrolizin-5(1*H*)-one,
   - la pyrido[3',2':4,5]pyridazino[1,6-*a*]indol-5(6*H*)-one,
   - la 6-méthylpyrido[3',2':4,5]pyridazino[1,6-*a*]indol-5(6*H*)-one,
   - la 6-méthyl-1,2,3,4-tétrahydropyrido[3',2':4,5]pyridazino[1,6-*a*]indol-5(6*H*)-one,
   - la (4a*R*,12b*R*)-6-méthyl-1,2,3,4,4a,12b-hexahydropyrido[3',2':4,5]pyridazino[1,6-*a*] indol-5(6*H*)-one,
   - la 1-méthyl-3a, 10b-dihydro[1,2,3]triazolo[4',5':3,4]pyrrolo[1,2-*a*]indol-4(1*H*)-one,
   - la 1-benzyl-3a, 10b-dihydro[1,2,3]triazolo[4',5':3,4]pyrrolo[1,2-*a*]indol-4(1*H*)-one, ne font pas partie de l'invention,
   par aryle, on entend un groupement phényle ou naphtyle, les deux étant éventuellement substitués par un ou plusieurs atomes d'halogène, groupements nitro, amino, alkyle (C₁-C₆) linéaire ou ramifié, ou alkoxy (C₁-C₆) linéaire ou ramifié,
   par hétérocycle à 5, 6 ou 7 chaînons, on entend un groupement monocyclique saturé ou insaturé contenant 5, 6 ou 7 côtés, et possédant un ou deux hétéroatomes choisis parmi azote et oxygène. On peut nommer la pyrrolidine, la pipéridine, l'azépane et la pyridine,
   par hétérocycle de 4 à 8 chaînons éventuellement substitué, contenant éventuellement une
   ou plusieurs doubles liaisons au sein de l'hétérocycle et contenant éventuellement au sein du système cyclique, un second hétéroatome choisi parmi l'atome d'oxygène ou l'atome d'azote, on peut nommer à titre non limitatif la pyrrolidine, la pipéridine, l'azépane, la pipérazine, la morpholine, ces hétérocycles pouvant éventuellement être substitués, y compris sur le deuxième atome d'azote de la pipérazine par un ou plusieurs groupements, identiques ou différents, choisis parmi alkyle (C₁-C₆) linéaire ou ramifié, hydroxyalkyle (C₁-C₆) linéaire ou ramifié, alkoxy (C₁-C₆)alkyle(C₁-C₆) linéaire ou ramifié, aminoalkyle (C₁-C₆) linéaire ou ramifié, la partie amino étant éventuellement substituée par un ou deux groupements identiques ou différents, alkyle (C₁-C₆) linéaire ou ramifié,
   par α, β, γ et δ on entend les centres chiraux éventuellement présents sur les composés de formule (I).

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la *tert*-butylamine, la lysine, etc...

Une variante avantageuse concerne les composés de formule (I) pour lesquels R₁ et R₄, identiques ou différents, représentent chacun un atome d'hydrogène ou un alkyle (C₁-C₆) linéaire ou ramifié.

Une autre variante avantageuse concerne les composés de formule (I) pour lesquels R₂ et R₃ représentent chacun un atome d'hydrogène.

Un autre aspect particulier de l'invention concerne les composés de formule (I) pour lesquels R₁ et R₂ ensemble avec les atomes de carbone qui les portent forment une liaison carbone carbone.

R₉ représente avantageusement un atome d'hydrogène.

n représente avantageusement un entier 0.

Les composés préférés de l'invention sont les composé de formule (I) pour lesquels R₅ et R₈ ensemble avec les atomes de carbone et d'azote qui les portent, forment un hétérocycle à 5, 6 ou 7 chaînons et plus particulièrement un hétérocycle à 6 chaînons.

Une autre variante avantageuse de l'invention concerne les composé de formule (I) pour lesquels R₆ et R₇ ensemble avec les atomes de carbone et d'azote qui les portent, forment un hétérocycle à 5, 6 ou 7 chaînons et plus particulièrement un hétérocycle à 6 chaînons.

Encore plus particulièrement, l'invention concerne les composés de formule (I) qui sont :
(4a*SR*,11a*SR*,11b*RS*)-1-Méthyl-1,2,3,4,4a,11,11a,11b-octahydropyrido[2',3':3,4]pyrrolo [1,2-*a*]indol-5-one,
(4a*SR*,11a*RS*,11b*SR*)-1-Allyl-1,2,3,4,4a,11,11a,11b-octahydropyrido[2',3':3,4]pyrrolo[1,2-*a*]indol-5-one,
(4a*SR*,11a*SR*,11b*RS*)-1-Méthyl-1,2,3,4,4a,11,11a,11b-octahydropyrido[2',3':3,4]pyrrolo [1,2-*a*]indol-5-one,
(4a*SR*,11a*SR*,11b*RS*)-1,2,3,4,4a,11,11a,11b-Octahydro-1,6,6a-triazabenzo[*a*]fluorén-5-one,
(4a*SR*,11a*SR*,11b*RS*)-(5-Oxo-3,4,4a,11,11a,11b-hexahydro-2*H*,5*H*-pyrido[2',3' :3,4]pyrrolo[1,2-*a*]indol-1-yl)-acétonitrile,
(3a*SR*,6a*RS*,10c*RS*)-4-Propyl-(3a,4,5,6,6a,10c-hexahydro)-3*H*-1,4,10b-triazafluoranthén-2-one,
(3a*RS*,6a*SR*,10c*RS*)-4-Propyl-(3a,4,5,6,6a,10c-hexahydro)-3*H* 1,4,10b-triazafluoranthén-2-one,
(4a*SR*,11a*RS*,11b*SR*)-1-Allyl-9-méthoxy-1,2,3,4,4a,11,11a,11b-octahydropyrido [2',3':3,4]pyrrolo[1,2-*a*]indol-5-one,
(4a*SR*,11a*SR*,11b*RS*)-9-Fluoro-1,2,3,4,4a,11,11a,11b-octahydropyrido[2',3':3,4]pyrrolo [1,2-*a*]indol-5-one,
(4a*SR*,11a*SR*,11b*RS*)-9-Chloro-1-méthyl-1,2,3,4,4a,1,1a,11b-octahydropyrido [2',3':3,4]pyrrolo[1,2-*a*]indol-5-one,
1,9-Diméthyl-1,2,3,4-tétrahydropyrido[2',3':3,4]pyrrolo[1,2-*a*]indol-5-one,
(11a*RS*)-1,9-Diméthyl-1,2,3,4,11,11a-hexahydropyrido[2',3':3,4]pyrrolo[1,2-*a*]indol-5-one,
(4a*S*,11a*R*,11b*S*)1-Allyl-1,2,3,4,4a,11,11a,11b-octahydropyrido[2',3':3,4]pyrrolo[1,2-*a*] indol-5-one,
(4a*R*,11a*S*,11b*R*)1-Allyl-1,2,3,4,4a,11,11a,11b-octahydropyrido[2',3':3,4]pyrrolo[1,2-*a*] indol-5-one,
(3a*RS*,10b*SR*,10c*RS*)-3-Benzyl-2,3,3a,4,10b,10c-hexahydrobenzo[*b*]pyrido[2,3,4-*gh*] pyrrolizin-5(1*H*)-one,
(4a*S*,11a*S*,11b*R*)-1-Méthyl-1,2,3,4,4a,11,11a,11b-octahydropyrido[2',3':3,4]pyrrolo[1,2-*a*]indol-5-one,
(4a*SR*,11a*RS*,11b*SR*)-1,2,3,4,4a,11a,11b-Octahydropyrido[2',3':3,4]pyrrolo[1,2-*a*]indol-5-one,
(4a*R*,11a*R*,11b*S*)-1-Méthyl-1,2,3,4,4a,11,11a,11b-octahydropyrido[2',3':3,4]pyrrolo[1,2-*a*]indol-5-one.

Les énantiomères, diastéréoisomères, N-oxydes, ainsi que les sels d'addition à un acide ou à une base, pharmaceutiquement acceptables, des composés préférés font partie intégrante de l'invention.

La notation (3a*RS*,10b*SR*,10c*RS*) suivi du nom du composé signifie que le produit obtenu est un mélange racémique et donc, que les deux configurations sont possibles.
A titre d'exemple :
(3a*RS*, 10b*SR*,10c*RS*)-3-benzyl-2,3,3a,4,10b,10c-hexahydrobenzo[*b*]pyrido[2,3,4-*gh*]-pyrrolizin-5(1*H*)-one signifie que le produit obtenu, le mélange racémique, contient le (3a*R*,10b*S*, 10c*R*)-3-benzyl-2,3,3a,4,10b,10c-hexahydrobenzo[*b*]pyrido[2,3,4-*gh*]pyrrolizin-5(1*H*)-one et le (3a*S*,10b*R*,10c*S*)-3-benzyl-2,3,3a,4,10b,10c-hexahydrobenzo[*b*]pyrido [2,3,4-*gh*]pyrrolizin-5(1*H*)-one.

La notation (3a*R*,10b*S*,10c*R*) ou (3a*S*,10b*R*,10c*S*) suivi du nom du composé signifie que le produit obtenu est un énantiomère optiquement pur.
A titre d'exemple :
(3a*R*,10b*S*,10c*R*) ou (3a*S*,10b*R*,10c*S*)-3-benzyl-2,3,3a,4,10b,10c-hexahydrobenzo[*b*] pyrido-[2,3,4-*gh*]pyrrolizin-5(1*H*)-one signifie que le produit obtenu, l'énantiomère optiquement pur, est le (3a*R*,10b*S*,10c*R*)-3-benzyl-2,3,3a,4,10b,10c-hexahydrobenzo [*b*]pyrido-[2,3,4-*gh*]-pyrrolizin-5(1*H*)-one ou le (3a*S*,10b*R*,10c*S*)-3-benzyl-2,3,3a,4,10b,10c-hexahydrobenzo-[*b*]pyrido-[2,3,4-*gh*]pyrrolizin-5(1*H*)-one.

Par énantiomère α et énantiomère β, on entend les énantiomères optiquement purs du mélange racémique correspondant.

La présente invention concerne également le procédé de préparation des composés de formule (I) caractérisé en ce que l'on utilise comme produit de départ un composé de formule (II) : 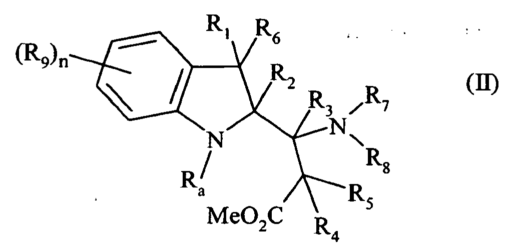 dans laquelle R₁, R₂, R₃, R₄, R₅, R₆, R₇, R₈, R₉ et n sont tels que définis dans la formule (I), Rₐ représente un atome d'hydrogène, un groupement nitroso, amino ou carboxylate de *tert-*butyle,
composé de formule (II) que l'on soumet à une réaction de cyclisation en milieu basique pour conduire aux composés de formule (I), qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

Une variante avantageuse concerne le procédé de préparation des composés de formule (I/a), cas particulier des composés de formule (I) : 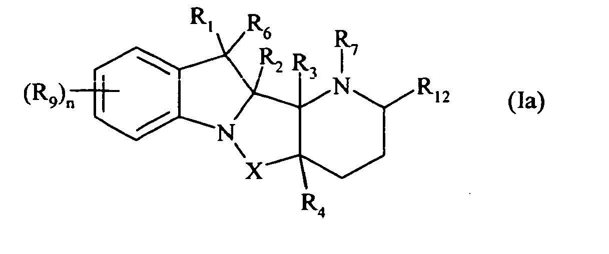 dans laquelle R₁, R₂, R₃, R₄, R₆, R₇, R₉, R₁₂ et n sont tels que définis dans la formule (I),
caractérisé en ce que l'on utilise comme produit de départ un composé de formule (III) : 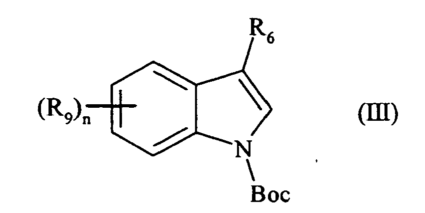 dans laquelle R₆, R₉ et n sont tels que définis dans la formule (I) et Boc représente le groupement carboxylate de *tert*-butyle, qui est mis en réaction avec du borate de triisopropyle en milieu anhydre pour conduire au composé de formule (IV) : 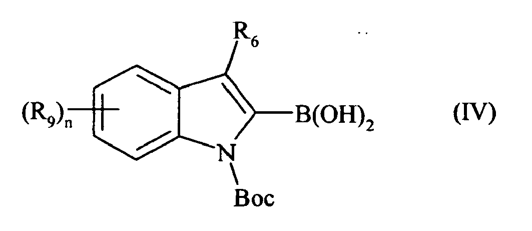 dans laquelle R₆, R₉, n et Boc sont tels que définis précédemment, que l'on fait réagir avec un composé de formule (V) : 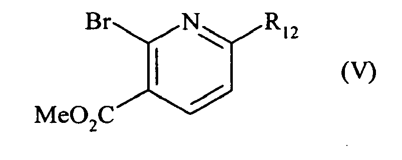 dans laquelle R₁₂ est tel que défini dans la formule (I),
en présence de base et de tétrakis(triphénylphosphine)palladium pour conduire au composé de formule (VI) : 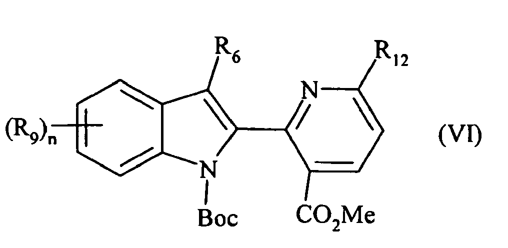 dans laquelle R₆, R₉, R₁₂, n et Boc sont tels que défini précédemment,
composé de formule (VI) que l'on soumet,
- soit à une réaction d'alkylation avec un composé de formule (VII) :

   R'₇-Hal (VII)
dans laquelle R'₇ prend toutes les définitions de R₇ tel que défini dans la formule (I) à l'exception de l'atome d'hydrogène et Hal représente un atome d'halogène,
pour conduire au composé de formule (VIII) : 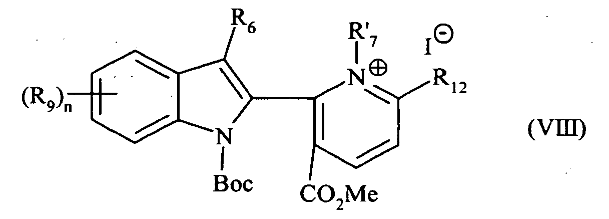 dans laquelle R₆, R'₇, R₉, R₁₂, n et Boc sont tels que définis précédemment,
que l'on soumet à une réaction de réduction partielle avec de l'hydrogène en présence de triéthylamine et d'oxyde de platine pour conduire au composé de formule (IX) : 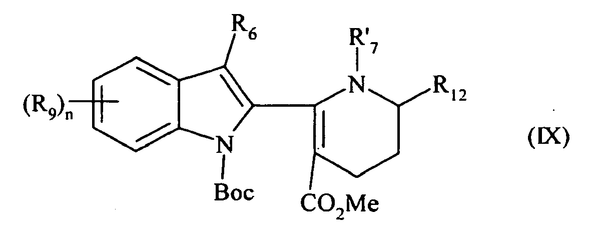 dans laquelle R₆, R'₇, R₉, R₁₂, n et Boc sont tels que définis précédemment,
que l'on soumet à une réduction en présence d'acide chlorhydrique et de cyanoborohydrure de sodium pour conduire au composé de formule (X), de stéréochimie *trans :* 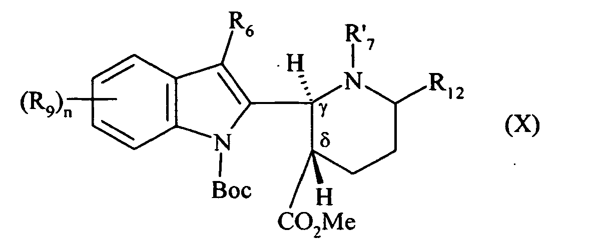 dans laquelle R₆, R'₇, R₉, R₁₂, n et Boc sont tels que définis précédemment, γ et δ sont tels
que définis dans la formule (I), et Hγ et Hδ sont de stéréochimie *trans,* qui subit une réaction de déprotection en présence d'acide trifluoroacétique pour conduire au composé de formule (XI), 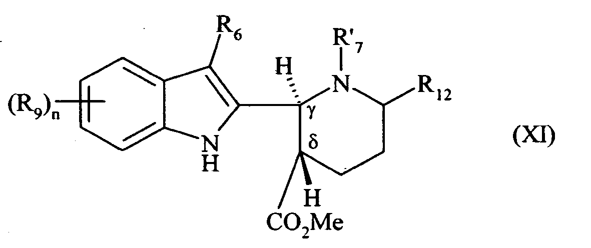 dans laquelle R₆, R'₇, R₉, R₁₂ et n sont tels que définis précédemment,
qui subit une réaction de cyclisation en présence d'un complexe borane-THF et d'acide trifluoroacétique pour conduire au composé de formule (Ia₁), cas particulier des composés de formule (I) : 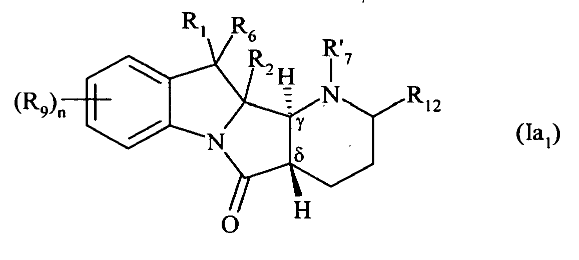 dans laquelle R₆, R'₇, R₉, R₁₂ et n sont tels que définis précédemment, et R₁ et R₂ sont tels que définis dans la formule (I),
- soit à une réaction de réduction avec de l'hydrogène en présence d'acide acétique et d'un catalyseur au rhodium pour conduire aux composés de formules (XIIa) et (XIIb), de stéréochimie (*trans, cis*) et (*cis,cis*) respectivement : 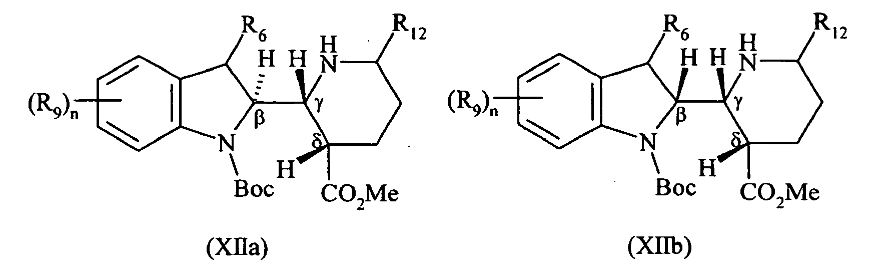
dans lesquelles R₆, R₉, R₁₂, n et Boc sont tels que définis précédemment, β, γ et δ sont tels
que définis dans la formule (I) et H_{β} et H_{γ} sont de stéréochimie *trans* dans le composé de formule (XIIa) et de stéréochimie *cis* dans le composé de formule (XIIb) et H_{γ} et H_{δ} sont de stéréochimie *cis* dans les composés de formules (XIIa) et (XIIb),
composés de formule (XIIa) et (XIIb) qui sont :
- soit mis à réagir avec un composé de formule (VII) tel que défini précédemment en présence de base pour conduire aux composés de formules (XIIIa) et (XIIIb) : 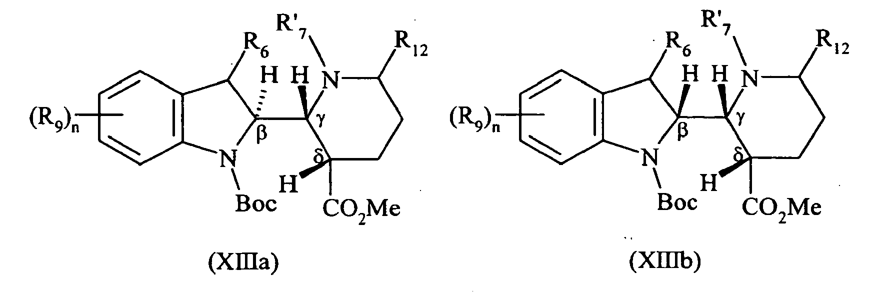
dans laquelle R₆, R'₇, R₉, R₁₂, n et Boc sont tels que définis précédemment,
composés de formules (XIIIa) et (XIIIb) qui sont :
□ soit mis en présence d'acide trifluoroacétique pour conduire aux composés de formules (Iₐ₂) et (XIVb), composés de formule (Iₐ₂), cas particulier des composés de formule (I) : 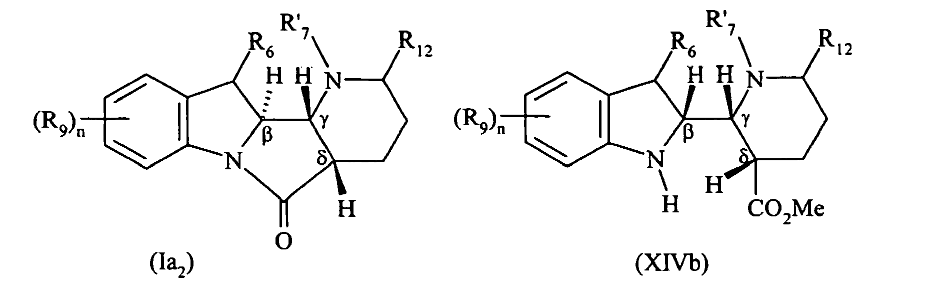
dans lesquelles R₆, R'₇, R₉, R₁₂, et n sont tels que définis précédemment,
composé de formule (XIVb) qui subit une réaction de cyclisation en présence d'hydrure de sodium pour conduire au composé de formule (Iₐ₃), cas particulier des composés de formule (I) : 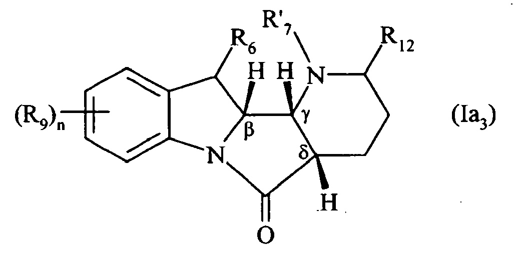 dans lesquelles R₆, R'₇, R₉, R₁₂, et n sont tels que définis précédemment,
composé de formule (XIIIa) qui est :
□ soit mis en présence de méthylate de sodium dans du méthanol pour conduire au composé de formule (XV) : 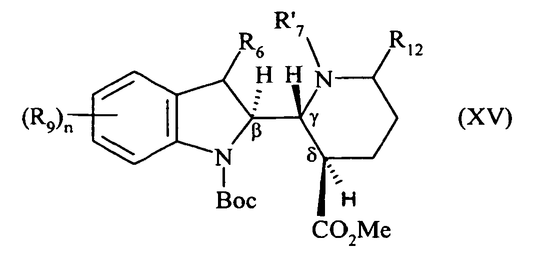
dans lesquelles R₆, R'₇, R₉, R₁₂, n et Boc sont tels que définis précédemment,
qui subit une réaction de cyclisation en présence d'acide trifluoroacétique pour conduire au composé de formule (Iₐ₄), cas particulier des composés de formule (I) : 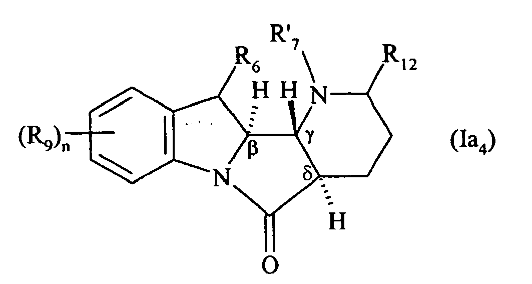 dans laquelle R₆, R'₇, R₉, R₁₂ et n sont tels que définis précédemment,
composé de formule (XIIa) qui est :
- soit soumis aux mêmes conditions que les composés de formules (XIIIa) et (XIIIb) pour conduire au composé de formule (XVI) : 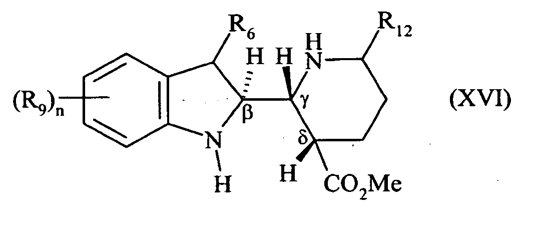
dans laquelle R₆, R₉, R₁₂ et n sont tels que définis précédemment,
composé de formule (XVI) qui est :
* soit dissous dans un mélange d'acide acétique et d'eau et une solution de nitrite de sodium pour conduire au composé de formule (XVII) : 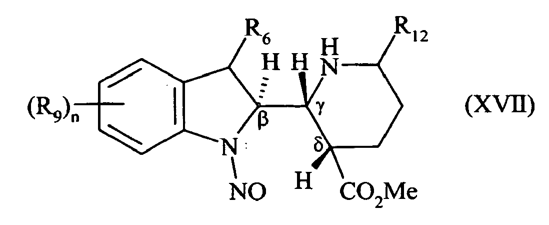
dans laquelle R₆, R₉, R₁₂ et n sont tels que définis précédemment,
qui est successivement mis à réagir avec un composé de formule (VII) tel que défini précédemment, puis du zinc et du carbonate d'ammonium pour conduire au composé de formule (Iₐ₅), cas particulier des composés de formule (I) : 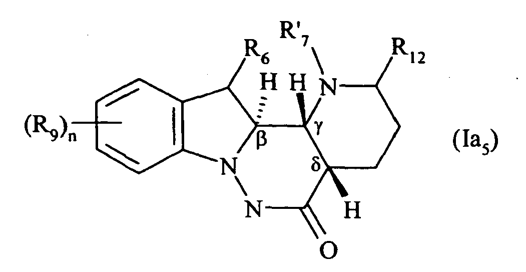 dans laquelle R₆, R'₇, R₉, R₁₂ et n sont tels que définis précédemment,
composé de formule (XVI) qui est:
* soit soumis à une réaction de cyclisation en présence de carbonate de potassium pour conduire au composé de formule (Iₐ₆), cas particulier des composés de formule (I) : 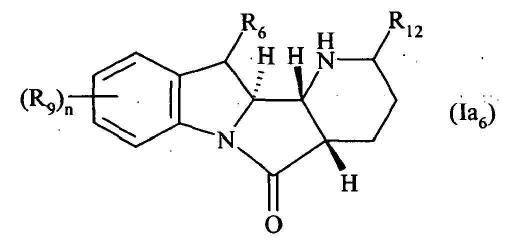
dans laquelle R₆, R₉, R₁₂ et n sont tels que définis précédemment,
qui subit une réaction d'alkylation avec un composé de formule (VII) tel que défini précédemment en milieu basique pour conduire au composé de formule (Iₐ₇), cas particulier des composés de formule (I) : 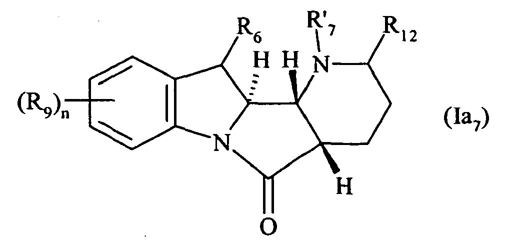 dans laquelle R₆, R'₇, R₉, R₁₂ et n sont tels que définis précédemment,
les composés de formules (Iₐ₁) à (Iₐ₇) forment les composés de formule (Ia), qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

Une seconde variante avantageuse concerne le procédé de préparation des composés de formule (Ib), cas particulier des composés de formule (I) : 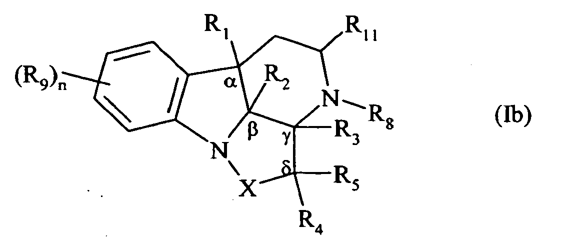 dans laquelle R₁, R₂, R₃, R₄, R₅, R₈, R₉, R₁₁, X et n sont tels que définis dans la formule (I)
caractérisé en ce que l'on utilise comme produit de départ un composé de formule (XVIII) : 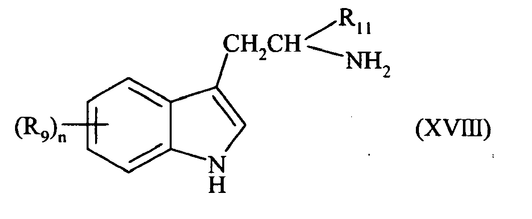 dans laquelle R₉, R₁₁ et n sont tels que définis précédemment,
qui est mis en réaction avec du cyanoacétate de méthyle en présence de Pd/C dans de l'acide acétique glacial pour conduire au composé de formule (XIX) : 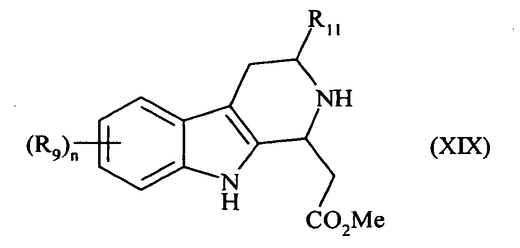 dans laquelle R₉, R₁₁ et n sont tels que définis précédemment,
qui est mis en réaction avec un composé de formule (XX) :

R"₇-CHO (XX)

dans laquelle R"₇ prend toutes les définitions de R₇ tel que défini dans la formule (I) à l'exception de l'atome d'hydrogène, suivi d'une réduction avec du borohydrure de sodium
ou du cyanoborohydrure de sodium pour conduire au composé de formule (XXI) : 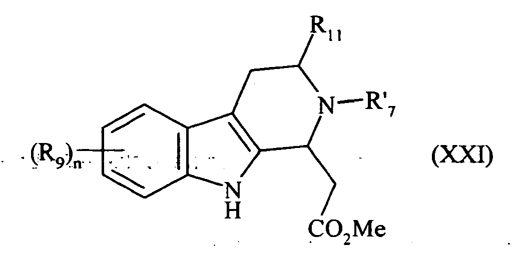 dans laquelle R₉, R₁₁ et n sont tels que définis précédemment et R'₇ prend toutes les définitions de R₇ tel que défini dans la formule (I) à l'exception de l'atome d'hydrogène,
composé de formule (XXI) qui est :
- soit mis à réagir avec un complexe BH₃-THF dans du THF puis mis en solution avec de l'acide trifluoroacétique pour conduire aux composés de formules (XXIIa) et (XXIIb), de stéréochimie (*trans, cis*) et (*trans, trans*) respectivement : 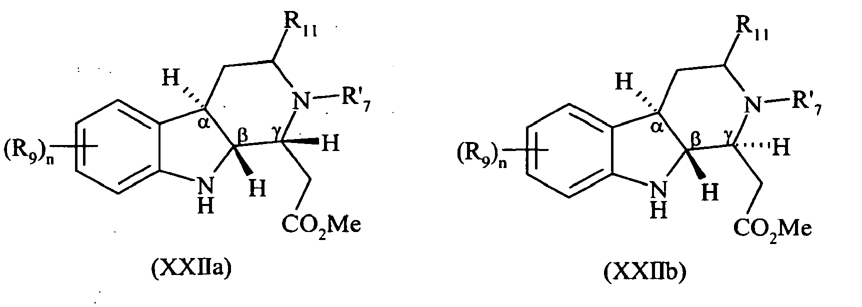
dans lesquelles R'₇, R₉, R₁₁ et n sont tels que définis précédemment, et H_{α} et H_{β} sont de stéréochimie *trans* dans les composés de formules (XXIIa) et (XXIIb) et H_{β} et H_{γ} sont de stéréochimie *cis* dans le composé de formule (XXIIa) et de stéréochimie *trans* dans le composé de formule (XXIIb),
qui sont dissous dans un mélange d'acide acétique et d'eau et une solution de nitrite de sodium pour conduire aux composés de formules (XXIIIa) et (XXIIIb) : 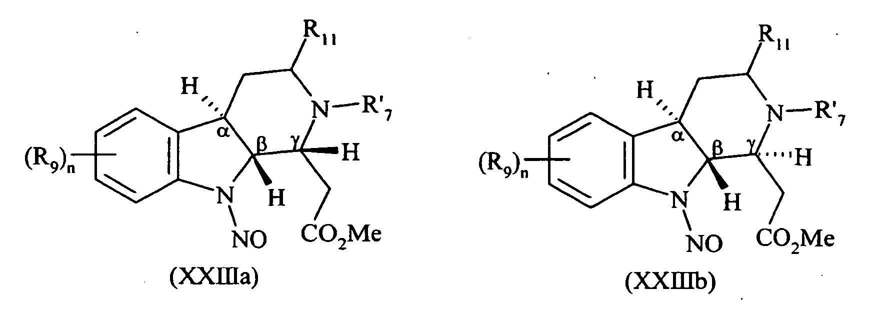 dans lesquelles R'₇, R₉, R₁₁ et n sont tels que définis précédemment,
qui sont mis en présence de carbonate d'ammonium et de zinc pour conduire aux composés de formulés (XXIVa) et (XXIVb) : 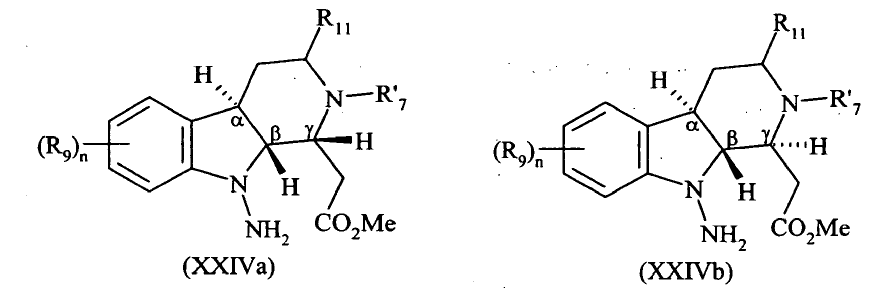 dans lesquelles R'₇, R₉, R₁₁ et n sont tels que définis précédemment,
qui subissent une réaction de cyclisation en présence d'hydrure de sodium ou de triméthylaluminium en solution dans l'hexane pour conduire aux composés de formules (I_{b1}) et (I_{b2}), cas particuliers des composés de formule (I) : 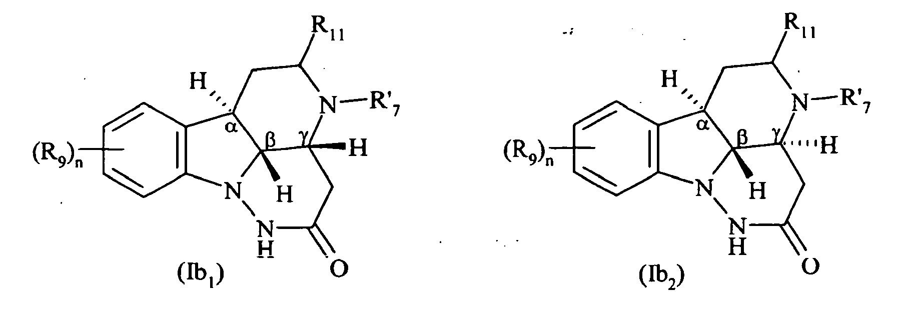 dans lesquelles R'₇, R₉, R₁₁ et n sont tels que définis précédemment,
composé de formule (XXI) qui est :
- soit mis à réagir avec l'anhydride de l'acide di-*tert*-butyloxycarbonique et du diméthylaminopyridine pour conduire au composé de formule (XXV) : 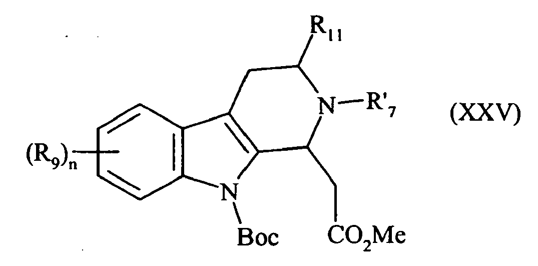
dans laquelle R'₇, R₉, R₁₁, et n sont tels que définis précédemment et Boc représente le
groupement carboxylate de *tert*-butyle, qui subit une réaction de réduction en présence d'oxyde de platine et d'hydrogène pour conduire aux composés de formules (XXVIa) et (XXVIb), de stéréochimie (*cis, cis*) et (*cis, trans*) respectivement : 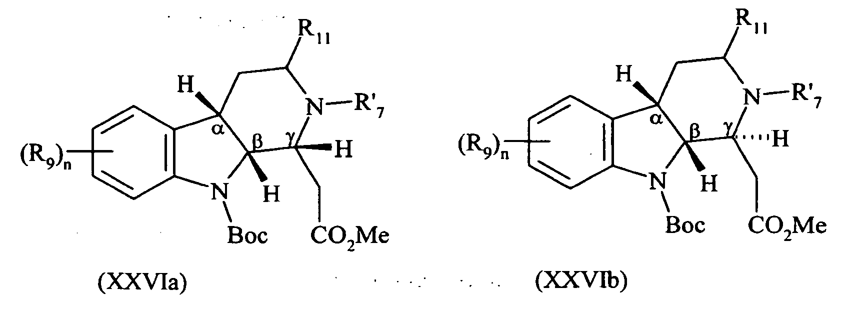 dans lesquelles R'₇, R₉, R₁₁, n et Boc sont tels que définis précédemment,
et H_{α} et H_{β} sont de stéréochimie *cis* dans les composés de formules (XXVIa) et (XXVIb) et H_{β} et H_{γ} sont de stéréochimie *cis* dans le composé de formule (XXVIa) et de stéréochimie *trans* dans le composé de formule (XXVIb),
qui sont mis en présence d'acide trifluoroacétique dans un milieu anhydre pour conduire aux composés de formules (XXVIIa) et (XXVIIb) : 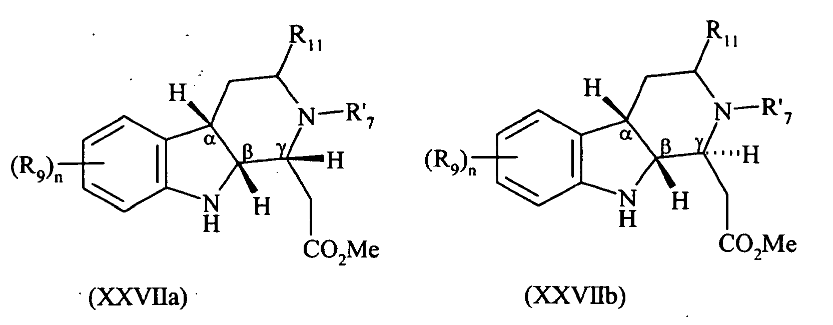 dans lesquelles R'₇, R₉, R₁₁ et n sont tels que définis précédemment,
qui subissent :
- soit une réaction de cyclisation dans les mêmes conditions que les composés de formules (XXIVa) et (XXIVb) pour conduire aux composés de formules (I_{b3}) et (I_{b4}), cas particulier des composés de formule (I) : 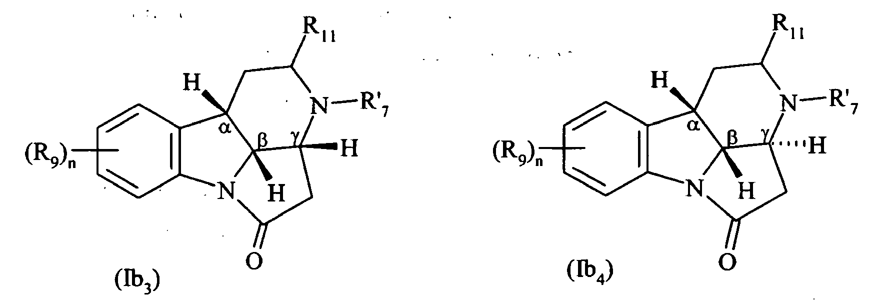
dans lesquelles R'₇, R₉, R₁₁ et n sont tels que définis précédemment,
- soit sont soumis aux mêmes conditions que les composés de formules (XXIIa) et (XXIIb) pour conduire aux composés de formule (XXVIIIa) et (XXVIIIb) : 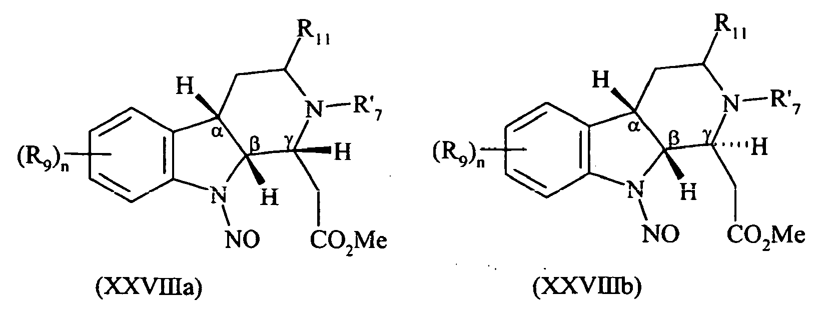
dans lesquelles R'₇, R₉, R₁₁ et n sont tels que définis précédemment,
qui sont soumis aux mêmes conditions que les composés de formules (XXIIIa) et (XXIIIb) pour conduire aux composés de formules (I_{b5}) et (I_{b6}), cas particulier des composés de formule (I) : 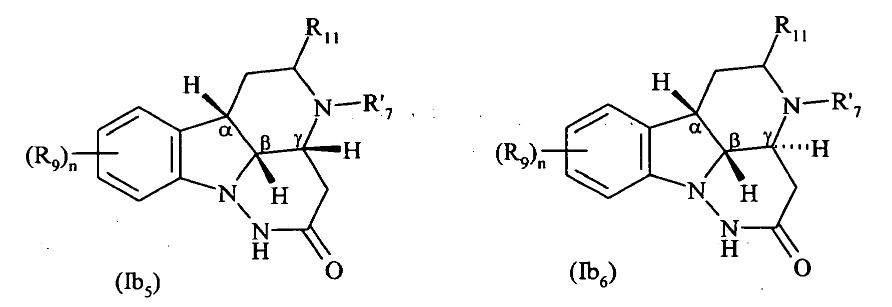 dans lesquelles R'₇, R₉, R₁₁ et n sont tels que définis précédemment,
les composés de formules (I_{b1}) à (I_{b6}) forment les composés de formule (Ib), qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

Les composés de formules (III), (V), (VII), (XVIII) et (XX) sont soit des produits commerciaux, soit obtenus selon des méthodes classiques de la synthèse organique bien connues de l'homme du métier.

Les composés de formule (I) possèdent d'intéressantes propriétés pharmacologiques et notamment celle d'être de puissants inducteurs de tyrosine hydroxylase (TH). On sait que la tyrosine hydroxylase est une enzyme limitante qui contrôle particulièrement la synthèse des neurotransmetteurs dans les neurones catécholaminergiques et dopaminergiques centraux. La vitesse de synthèse de ces neurotransmetteurs est notamment reliée à l'apparition de dysfonctions toniques du cerveau que sont de nombreuses pathologies comportementales chez l'homme, telles que l'anxiété, les psychoses, les dépressions, le stress, etc...

Grâce à leur capacité d'induction de tyrosine hydroxylase, les composés de l'invention trouvent donc leur utilisation thérapeutique dans le traitement des dépressions, de l'anxiété, des troubles de la mémoire au cours de la sénescence et/ou de maladies neurodégénératives, et dans le traitement palliatif de la maladie de Parkinson et pour l'adaptation au stress.

La présente invention a également pour objet les compositions pharmaceutiques renfermant comme principe actif au moins un composé de formule (I), ses énantiomères, diastéréoisomères, N-oxydes ou un de ses sels d'addition à une base ou un acide pharmaceutiquement acceptable, seul ou en combinaison avec un ou plusieurs excipients ou véhicules inertes, non toxiques pharmaceutiquement acceptables.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par voies orale, rectale, intramusculaire ou parentérale.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires ou ovules, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 0,1 mg à 100 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples et dans les préparations ont été déterminées selon les techniques spectrométriques usuelles (infrarouge, résonance magnétique nucléaire, spectrométrie de masse, ...).

Les points de fusion ont été déterminés sur appareil TOTTOLI (sans correction de colonne émergente). Lorsque le composé existe sous forme de sel, le point de fusion correspond à celui du produit salifié.

### PREPARATION 1: (2SR,3SR)-2-(1H-Indol-2-yl)-1-méthylpipéridine-3-carboxylate de méthyle

### Stade A : Acide 1-(tert-butoxycarbonyl)-1H-indol-2-yl-2-boronique

À une solution de 25 g de 1*H*-indole-1-carboxylate de *tert*-butyle et de 32,4 g de borate de triisopropyle dans le 120 ml de THF anhydre sous atmosphère d'azote, est ajoutée à 0 °C une solution de 75 ml de LDA 2 M goutte à goutte. L'agitation est maintenue pendant 40 min avant d'ajouter 200 ml d'une solution aqueuse d'acide chlorhydrique 2 M. Le pH est ajusté à pH = 7 en ajoutant une solution concentrée d'ammoniaque. Après séparation des phases, la phase aqueuse est extraite par de l'acétate d'éthyle (2 x 100 ml). Les phases organiques sont rassemblées, lavées avec une solution saturée en chlorure de sodium (100 ml), séchées sur sulfate de sodium, filtrées et concentrées sous pression réduite. De l'eau est ajoutée au résidu et le mélange est trituré jusqu'à la précipitation de l'acide boronique qui est filtré et lavé à quatre reprises avec du pentane pour obtenir 29,27 g du produit attendu.
*Point de fusion: 96-98°C*

### Stade B : 2-[(3-Méthoxycarbony/)pyridin-2-yl]indo/e-1-carboxy/ate de tert-butyle

À une solution dégazée de 9,94 g de 2-bromonicotinate de méthyle dans 170 ml de DME à 85 °C, sont successivement ajoutés 13,46 g d'une solution de carbonate de sodium dans 50 ml d'eau et 4,77 g de tétrakis(triphénylphosphine) palladium. Une solution de 12,96 g du composé du stade A précédent dans 50 ml d'éthanol est alors ajoutée goutte à goutte. L'agitation est maintenue pendant 6 h à 85 °C avant de revenir à température ambiante et d'ajouter 200 ml d'eau. Après séparation des phases, la phase aqueuse est extraite par de l'éther diéthylique (2 x 100 ml). Les phases organiques sont rassemblées, lavées avec une solution saturée en chlorure de sodium, séchées sur sulfate de sodium, filtrées et concentrées sous pression réduite. Une chromatographie sur colonne de gel de silice (cyclohexane/acétate d'éthyle : 9/1) permet d'obtenir 14,11 g du produit attendu.
*Point de fusion : 83°C*
*Spectrométrie de masse (ES* +, *m*/*z) :* 375 *(M+Na)⁺ ;* 353 *(M+H)⁺*
*Analyse élémentaire :*

| | *C%* | *H%* | *N%* |
|---|---|---|---|
| *Calculé:* | *68,17* | *5,72* | *7,95* |
| *Trouvé :* | *68,03* | *5,85* | *7,85* |

### Stade C : Iodure de 2-(1-tert-butoxycarbonyl-1H-indol-2-yl)-3-(méthoxycarbonyl)-1-méthylpyridinium

Un mélange de 1,40 g du composé du stade B précédent et 5 ml d'iodométhane est agité à température ambiante pendant 4 j. Le mélange réactionnel est alors concentré sous pression réduite et le résidu, repris dans l'éther diéthylique, est filtré pour obtenir 1,73 g du produit attendu.
*Point de fusion : Dégradation à 120 °C*
*Spectrométrie de masse (ES +, m*/*z) : 367 (M-I)*
*Analyse élémentaire :*

| | *C%* | *H%* | *N%* |
|---|---|---|---|
| *Calculé :* | *51,02* | *4,69* | *5,67* |
| *Trouvé:* | *50,79* | *4,88* | *5,71* |

### Stade D : 2-[3-(Méthoxycarbonyl)-1-méthyl-1,4,5,6-tétrahydropyridin-2-y]-1H-indole-1-carboxylate de tert-butyle

Un mélange de 1,01 g du composé du stade C précédent, de 0,63 ml triéthylamine et de 100 mg d'oxyde de platine dans 10 ml de méthanol, est agité sous atmosphère d'hydrogène pendant 3 h. Le mélange réactionnel est alors filtré sur célite et le filtrat, concentré. Le résidu est repris par du dichlorométhane et lavé avec de l'eau. La phase organique est séchée sur sulfate de sodium, filtrée et concentrée sous pression réduite pour donner 0,73 g du composé attendu.
*Infrarouge (ν_{cm-1}):* 2943 (ν_{C-H}) ;1730 (ν_{C=O} ester) ; 1686 (ν_{C=O} carbamate) ; 1597, 1581, 1545 (ν_{C=C}) ; 1365 (δ_{C-H} *t*Bu) ; 1338 (ν_{C-N}) ; 1259, 1117 (δ_{C-H}).

### Stade E 2-[(2SR,3SR)-3-(Méthoxycarbonyl)-1-méthylpipéridin-2-yl]-1H-indole-1-carboxylate de tert-butyle

À une solution de 1,36 g du composé du stade D précédent dans 22 ml de méthanol et 2 ml d'acide chlorhydrique aqueux 37 %, sont ajoutés à température ambiante, 2,0 g de cyanoborohydrure de sodium par portions de 0,5 g toutes les 5 min. L'agitation est maintenue pendant 30 min avant d'ajouter une solution aqueuse saturée en carbonate de potassium. Le méthanol est évaporé sous pression réduite. La phase aqueuse est extraite par du dichlorométhane. La phase organique est séchée sur sulfate de sodium, filtrée et concentrée sous pression réduite pour donner 1,21 g du composé attendu.
*Spectrométrie de masse (ES +, m*/*z) : 373 (M+H)⁺ ; 317.*
*Infrarouge (ν_{cm-1}) :* 2945 et 2785 (ν_{C-H}) ; 1729 (ν_{C=O}) ; 1452 (ν_{C=C}) ; 1369 (δ_{C-H} *t*Bu) ; 1322 (ν_{C-N}) ; 1157 (δ_{C-H}).

### Stade F : (2SR,3SR)-2-(1H-Indol-2-yl)-1-méthylpipéridine-3-carboxylate de méthyle

À une solution de 0,57 mg du composé du stade E précédent dans 5 ml de dichlorométhane, sont ajoutés 15 ml d'acide trifluoroacétique. Le mélange est agité à température ambiante pendant 6 h et concentré sous pression réduite. Le résidu est repris par de l'eau et du dichlorométhane. Du carbonate de potassium est ajouté jusqu'à ce que le pH de la phase aqueuse soit basique. La phase aqueuse est extraite par du dichlorométhane à deux reprises. Les phases organiques réunies sont séchées sur sulfate de sodium, filtrées et concentrées sous pression réduite. Une chromatographie sur colonne de gel de silice (dichlorométhane/méthanol : 98/2) permet d'obtenir 365 mg du composé attendu.
*Spectrométrie de masse* (ES +, m/z) : 273 (M+H)⁺.
*Infrarouge (ν_{cm-1}) :* 3386 (ν_{N-H}) 2946 et 2789 (ν_{C-H}) ; 1721 (ν_{C=O}) ; 1456 et 1435 (ν_{C=C}) ; 1162(δ_{C-H}).

### PREPARATION 2 : (SR)-2-[(2RS,3SR)-3-(Méthoxycarbonyl)-1-méthylpipéridin-2-yl]indoline-1-carboxylate de tert-butyle

### Stade A : (SR)-2-[(2RS,3SR)-3-(Méthoxycarbonyl)pipéridin-1-yl]indoline-1-carboxylate de tert-butyle

Un mélange de 6,00 g du composé du stade B de la préparation 1 et de 7,0 g de rhodium 5 % sur alumine dans 60 ml d'acide acétique, est agité à température ambiante sous 15 bars de pression d'hydrogène pendant 20 h. Le milieu réactionnel est alors filtré sur papier. Le papier est alors rincé avec du méthanol. Le filtrat est concentré sous pression réduite et le résidu est repris par du dichlorométhane et de l'eau. Du carbonate de potassium est ajouté jusqu'à pH basique de la phase aqueuse. Les phases sont séparées et la phase aqueuse est extraite à deux reprises par du dichlorométhane. Les phases organiques sont réunies, séchées sur sulfate de sodium, filtrées et concentrées sous pression réduite. Une chromatographie sur colonne de gel de silice (dichlorométhane/méthanol : 95/5) permet d'obtenir 3,49 g du composé attendu.
*Point de fusion : 79-80 °C*
*Spectrométrie de masse (ES +, m*/*z) : 383 (M+Na)⁺; 361 (M+H)⁺; 305.*
*Analyse élémentaire :*

| | *C%* | *H%* | *N%* |
|---|---|---|---|
| *Calculé :* | *66, 64* | *7,83* | *7,77* |
| *Trouvé :* | *66,45* | *7,96* | *7, 67* |

### Stade B: (SR)-2-[(2RS,3SR)-3-(Méthoxycarbonyl)-1-méthylpipéridin-2-yl]indoline-1-carboxylate de tert-butyle

À une solution de 0,50 g du composé du stade A précédent dans 15 ml d'acétonitrile à température ambiante, est ajouté 0,15 ml d'une solution aqueuse de formaldéhyde 37 %. La solution est agitée à température ambiante pendant 40 min puis, 1,5 ml d'acide acétique est ajouté. Après 10 min d'agitation, 100 mg de borohydrure de sodium est ajouté par petites portions. Après l'addition de la dernière portion, le mélange réactionnel est agité pendant 1 h à température ambiante puis, de l'eau et du carbonate de potassium sont ajoutés jusqu'à pH basique de la solution. La solution est alors extraite par de l'acétate d'éthyle à trois reprises. Les phases organiques sont rassemblées, séchées sur sulfate de sodium, filtrées et concentrées sous pression réduite pour donner 0,50 g du produit attendu.
*Infrarouge (ν_{cm-1}):* 2931 (ν_{C-H}) 1736 (ν_{C=O} ester) ; 1702 (ν_{C=O} carbamate) ; 1482 (ν_{C=C}) ; 1368 (δ_{C-H}).

### PREPARATION 3 : (RS)-2-[(2RS,3SR)-3-(Méthoxycarbonyl)pipéridin-2-yl]indoline-1-carboxylate de tert-butyle

Le produit est obtenu lors de la synthèse du composé du stade A de la préparation 2.
Une chromatographie sur colonne de gel de silice (dichlorométhane/méthanol : 95/5) permet d'obtenir 0,43 g du produit attendu.
*Infrarouge (ν_{cm-1}) :* 3335 (ν_{N-H}) ; 2934 (ν_{C-H}) ; 1725 (ν_{C=O} ester) ; 1697 (ν_{C=O} carbamate) ; 1483 (ν_{C=C}) ; 1391 et 1368 (δ_{C-H}).

### PREPARATION 4 : (2RS,3SR)-2-[(SR)-1-Nitrosoindolin-2-yl]pipéridine-3-carboxylate de méthyle

### Stade A : (2RS,3SR) 2-[(SR)-Indolin-2-yl]pipéridine-3-carboxylate de méthyle

À une solution de 927 mg du composé du stade A de la préparation 2 dans 15 ml de dichlorométhane, sont ajoutés 15 ml d'acide trifluoroacétique à température ambiante. Le mélange est agité à température ambiante pendant 2 h et concentré sous pression réduite. Le résidu est repris avec du dichlorométhane et de l'eau. Du carbonate de potassium est ajouté jusqu'à pH basique de la phase aqueuse. La phase aqueuse est extraite par du dichlorométhane. Les phases organiques sont réunies, séchées sur sulfate de sodium et concentrées sous pression réduite pour donner 633 mg du produit attendu.
*Infrarouge (ν_{cm-1}) :* 3369 (ν_{N-H}) ; 2942 et 2857 (ν_{C-H}) ; 1720 (ν_{C=O}) ; 1485 (ν_{C=C}) ; 1249, 1197 et 1165 *(ν_{N-C}).*

### Stade B : (2RS,3SR)-2-[(SR)-1-Nitrosoindolin-2-yl]pipéridine-3-carboxy/ate de méthyle

633 mg du composé du stade A précédent sont dissous dans un mélange de 6 ml d'acide acétique et 6 ml d'eau à 0 °C et une solution de 168 mg de nitrite de sodium dans 4 ml d'eau est ajoutée goutte à goutte. Le mélange est agité 20 min à 0 °C avant d'ajouter du dichlorométhane. Du carbonate de potassium est ajouté jusqu'à pH basique de la phase aqueuse. La phase aqueuse est extraite par du dichlorométhane à deux reprises. Les phases organiques sont rassemblées, séchées sur sulfate de sodium et concentrées sous pression réduite. Une chromatographie sur colonne de gel de silice (dichlorométhane/méthanol : 99/1) permet d'isoler 538 mg du produit attendu.
*Infrarouge (ν_{cm-1})* : 3314 (ν_{N-H}) ; 2939 et 2857 (ν_{C-H}) ; 1721 (ν_{C=O}) ; 1485 et 1477 (ν_{C=C}) ; 1430 (ν_{N=O}) ; 1168 (ν_{C-N}).

### PREPARATION 5 (SR)-2-[(2RS,3SR)-3-(Méthoxyvcarbonyl)-1-allylpipéridin-2-yl]lindoline-1-carboxylate de tert-butyle

À une solution de 440 mg du composé du stade A de la préparation 2 dans 10 ml d'acétonitrile, sont successivement ajoutés à température ambiante 0,3 ml de bromure d'allyle puis, 800 mg de carbonate de potassium. Le mélange est agité à température ambiante pendant 3 h puis, de l'eau est ajoutée. La phase aqueuse est extraite par du dichlorométhane. Les phases organiques réunies sont séchées sur sulfate de sodium, filtrées et concentrées sous pression réduite. Une chromatographie sur colonne de gel de silice (cyclohexane/acétate d'éthyle : 8/2) permet d'isoler 382 mg du produit attendu.
*Point de fusion: 119-121 °C*
*Analyse élémentaire :*

| | *C%* | *H%* | *N%* |
|---|---|---|---|
| *Calculé :* | *68,97* | *8,05* | *6,99* |
| *Trouvé :* | *68, 85* | *8,15* | *7, 04* |

### PREPARATION 6 : (RS)-2-[(2SR,3SR)-3-(Méthoxycarbonyl)-1-allylpipéridin-2-yl]indoline-1-carboxylate de tert-butyle

0,80 g d'une solution du composé de la préparation 5 dans 10 ml de THF, est ajouté à une solution de méthylate de sodium dans le méthanol (préparée en ajoutant par petites portions 0,23 g de sodium dans 10 ml de méthanol à 0°C). Le mélange réactionnel est porté au reflux du solvant pendant 3 h. Après refroidissement du milieu réactionnel à température ambiante, 15 ml d'eau est ajoutée ; le méthanol et le THF sont éliminés par évaporation sous pression réduite. La solution résultante est extraite par de l'acétate d'éthyle à deux reprises. Les phases organiques sont réunies, séchées sur sulfate de sodium, filtrées et concentrées sous pression réduite. Une chromatographie sur colonne de gel de silice (cyclohexane/éther diéthylique : 9/1) permet d'isoler 0,58 g du produit attendu. *Infrarouge (ν_{cm-1}) :* 2929 (ν_{C-H}) 1732 (ν_{C=O} ester) ; 1698 (ν_{C=O} carbamate) ; 1483 (ν_{C=C}) ; 1369 (δ_{C-H} *t*Bu*).*

### PREPARATION 7 : (2RS,3SR)-2-[(RS)-Indolin-2-yl]-1-méthylyipéridine-3-carboxylate de méthyle

### Stade A (RS)-2-[(2RS,3SR)-3-(Méthoxycarbonyl)-1-méthylpipéridin-2-yl]indoline-1-carboxylate de tert-butyle

À une solution de 0,43 g du composé de la préparation 3 dans 10 ml d'acétonitrile à température ambiante, est additionnée 0,12 ml d'une solution aqueuse de formaldéhyde 37%. La solution est agitée à température ambiante pendant 40 min puis, 1,0 ml d'acide acétique est ajouté. Après 10 min d'agitation, 75 mg de borohydrure de sodium sont ajoutés par petites portions. Après l'addition de la dernière fraction, le mélange réactionnel est agité pendant 1 h à température ambiante puis, de l'eau est ajoutée. Du carbonate de potassium est ajouté jusqu'à pH basique de la solution. La solution est extraite trois fois par du dichlorométhane. Les phases organiques sont réunies, séchées sur sulfate de sodium, filtrées et concentrées sous pression réduite pour donner 0,37 g du produit attendu.
*Point de fusion: 100-104°C.*
*Infrarouge (ν_{cm-1}) :* 2948 et 2773 (ν_{C-H}); 1736 (ν_{C=O} ester); 1694 (ν_{C=O} carbamate); 1485 (ν_{C=C}); 1390 (δ_{C-H} *t*Bu).

### Stade B : (2RS,3SR)-2-[(RS)-Indolin-2-yl]-1-méthylpipéridine-3-carboxylate de méthyle

Le produit est obtenu selon le procédé du stade A de la préparation 4 en remplaçant le composé du stade A de la préparation 2 par le composé du stade A précédent.
*Infrarouge (ν_{cm-1}) :* 3396 (ν_{N-H)}; 2939 et 2858 (ν_{C-H)}; 1727(ν_{C=O)}; 1485 (ν_{C=C}).

### PREPARATION 8 : (1RS,4aRS,9aRS)-(2-Propyl-2,3,4,4a,9,9a-hexahydro-1H-β-carbolin-1-yl)acétate de méthyle

### Stade A : (RS)-(2,3,4,9-Tétrahydro-1H-β-carbolin-1-yl)acétate de méthyle

À une solution de 8 g de tryptamine dans 150 ml d'acide acétique glacial, sont ajoutés 4,4 ml de cyanoacétate de méthyle puis, 550 mg de Pd/C à 10 %. Après dégazage sous pression réduite, le mélange réactionnel est placé sous atmosphère d'hydrogène à température ambiante puis, agité 72 heures. La suspension est filtrée sur célite (élution : méthanol). La solution est évaporée sous pression réduite et le résidu, repris par du dichlorométhane et par de l'eau. La phase aqueuse est alcalinisée jusque pH 10 par l'ajout de carbonate de sodium. La phase organique est récupérée et la phase aqueuse, extraite par du dichlorométhane. Les phases organiques réunies sont séchées sur sulfate de sodium, filtrées puis évaporées sous pression réduite. Une chromatographie éclair sur gel de silice (acétate d'éthyle/méthanol : 95/5) permet d'obtenir 7,7 g du produit attendu.
*Point de fusion : 98°C*
*Analyse élémentaire :*

| | *C%* | *H%* | *N%* |
|---|---|---|---|
| *Calculé :* | *68,83* | *6,60* | *11,47* |
| *Trouvé :* | *68,69* | *6,74* | *11,34* |

### Stade B : (RS)-(2-Propyl-2,3,4,9-tétrahydro-1H-β-carbolin-1-yl)acétate de méthyle

À une solution de 2,1 g de composé du stade A précédent dans 40 ml d'un mélange méthanol/acide acétique 95/5 à 0 °C. sont ajoutés 1,32 ml de propionaldéhyde puis 1,62 g de cyanoborohydrure de sodium par portions pendant trente minutes à 0 °C. La solution est agitée 1h 30 et diluée par du dichlorométhane et de l'eau ; la phase aqueuse est alcalinisée par l'addition de carbonate de sodium. La phase organique est séparée et la phase aqueuse, extraite par du dichlorométhane. Les phases organiques réunies sont séchées sur sulfate de sodium, filtrées puis, évaporées sous pression réduite. Une chromatographie éclair sur gel de silice (cyclohexane/acétate d'éthyle : 85/15 jusque 8/2) permet d'obtenir 2,2 g du produit attendu.
*Infrarouge* (ν_{cm-1}) : 3403; 3351 (ν_{N-H}); 3042 (ν_{=C-H}); 2957; 2926; 2848 (ν_{C-H}); 1722 (ν_{C=O}); 1608 (ν_{C=C}); 1456; 1436 (δ_{C-H}); 1360 (ν_{N-C}); 1231 (ν_{C-O}).
*Analyse élémentaire:*

| | *C%* | *H%* | *N%* |
|---|---|---|---|
| *Calculé :* | *71,30* | *7,74* | *9,78* |
| *Trouvé :* | *71,03* | *7,95* | *9,59* |

### Stade C : (1RS,4aRS,9aRS)-(2-Propyl-2,3,4,4a,9,9a-herahvdro-1H-β-carbolin-1-vl)acétate de méthyle

À une solution de 1,52 g du composé du stade B précédent dans 40 ml de THF anhydre refroidie à 0 °C, sont ajoutés 5,56 ml du complexe BH₃-THF (1M dans le THF). La solution est agitée 1h 30 à 0 °C puis, est diluée par du dichlorométhane et de l'eau. La phase aqueuse est alcalinisée jusqu'à pH 10 et extraite deux fois par du dichlorométhane. Les phases organiques réunies sont séchées sur sulfate de sodium, filtrées et évaporées sous pression réduite. 1,6 g d'amineborane est obtenu. Cet amineborane est dissous dans 16 ml de TFA et la solution est agitée pendant 1h à température ambiante. Le solvant est éliminé par évaporation sous pression réduite puis, le résidu est repris par du dichlorométhane et par de l'eau. La phase aqueuse, alcalinisée jusqu'à pH 10 par du carbonate de sodium, est extraite deux fois par du dichlorométhane. Les phases organiques réunies sont séchées sur sulfate de sodium, filtrées puis, évaporées sous pression réduite. Une chromatographie éclair sur gel de silice (cyclohexane/acétate d'éthyle : 85/15) permet d'obtenir 305 mg du produit attendu.
*Point de fusion :* 41°C.
*Infrarouge* (ν_{cm-1}) : 3261 (ν_{N-H}); 3033 (ν_{=C-H}); 2961; 2937; 2887; 2869; 2833 (ν_{C-H}); 1724 (ν_{C=O}); 1612 (ν_{C=C}); 1460; 1433 (δ_{C-H}); 1357; 1339 (ν_{C-N}); 1252; 1239 (ν_{C-O}).
*Analyse élémentaire*

| | *C%* | *H%* | *N%* |
|---|---|---|---|
| *Calculé :* | *70,80* | *8,39* | *9,71* |
| *Trouvé :* | *70,70* | *8,16* | *9,63* |

### PREPARA TION 9 : (1SR,4aRS,9aRS)-(9-Amino-2-propyl-2,3,4,4a,9,9a-hexahydro-1H-β-carbolin-1-yl)acétate de méthyle

### Stade A : (1SR,4aRS,9aRS)-(2-Propyl-2,3,4,4a,9,9a-hexahydro-1H-β-carbolin-1-yl)acétate de méthyle

Le produit est obtenu lors de la synthèse du composé du stade C de la préparation 8.
Une chromatographie sur colonne de gel de silice (cyclohexane/acétate d'éthyle : 8/2) permet d'obtenir 760 mg du produit attendu.
*Point de fusion :* 85 °C.
*Spectrométrie de masse* (ESI +, m/z) *:* 289,2.(M + H+); 311,2 (M+Na+).
*Analyse élémentaire:*

| | *C%* | *H%* | *N%* |
|---|---|---|---|
| *Calculé:* | *70,80* | *8,39* | *9,71* |
| *Trouvé :* | *70,77* | *8,52* | *9,60* |

### Stade B : (1SR,4aRS,9aRS)-(9-Nitroso-2-propyl-2,3,4,4a,9,9a-hexahydro-1H-β-carbolin-1-yl)acétate de méthyle.

À une solution de 660 mg du composé du stade A précédent dans 10 ml d'un mélange acide acétique/eau 1/1 à 0 °C, est ajoutée goutte à goutte une solution de 160 mg de NaNO₂ dans 3 ml d'eau. Le mélange réactionnel est ensuite agité 30 min à 0 °C puis, dilué par du dichlorométhane. La phase aqueuse est alcalinisée à pH 10 par du carbonate de sodium. La phase organique est séparée tandis que la phase aqueuse est extraite deux fois par du dichlorométhane. Les phases organiques réunies sont séchées sur sulfate de sodium, filtrées puis évaporées. Une chromatographie éclair sur gel de silice (cyclohexane/acétate d'éthyle 8/2) permet d'obtenir le produit attendu.

*Point de fusion :* 76 °C.
*Infrarouge* (ν_{cm-1}) : 2955; 2929; 2877; 2840; 2806 (v _{C-H}); 1732 (v _{C=O}); 1474; 1455 (δ _{C-H}); 1370; 1356 (v _{N-C}); 1242; 1204 (v _{C-O}).
*Analyse élémentaire*

| | *C%* | *H%* | *N%* |
|---|---|---|---|
| *Calculé :* | *64,33* | *7,30* | *13,24* |
| *Trouvé :* | *64, 07* | *7,43* | *13,05* |

### Stade C : (1SR,4aRS,9aRS)-(9-Amino-2-propyl-2,3,4,4a,9,9a-hexahydro-1H-β-carbolin-1-yl)acétate de méthyle

À une solution de 700 mg du composé du stade B précédent dans 22 ml d'éthanol à 0 °C, sont ajoutés 10 ml d'eau, 1,89 g de carbonate d'ammonium et 1,28 g de zinc. Le mélange réactionnel est agité 1h 30 à 0 °C. La suspension est filtrée sur fritté (l'insoluble est lavé par du dichlorométhane et de l'eau). La phase organique est récupérée tandis que la phase aqueuse est extraite par du dichlorométhane. Les phases organiques réunies sont séchées sur sulfate de sodium, filtrées puis, évaporées sous pression réduite. Une chromatographie éclair sur gel de silice (cyclohexane/acétate d'éthyle : 8/2 puis 7/3) permet d'obtenir 250 mg du produit attendu.
*Point de fusion :* 82 °C.
*Infrarouge* (ν_{cm-1}) : 3307 (ν_{N-H}); 2957; 2922; 2860 (ν_{C-H}); 1731 (v _{C=O}); 1641; 1606 (ν_{C=C}); 1469; 1456 (δ _{C-H}); 1372 (ν_{N-C}); 1242; 1194 (v _{C-O}).
*Analyse élémentaire* :

| | *C%* | *H%* | *N%* |
|---|---|---|---|
| *Calculé :* | *67,30* | *8,31* | *13,85* |
| *Trouvé :* | *67,29* | *8,43* | *13,79* |

### PREPARATION 10 : (1RS, 4aSR, 9aRS)-(2-Propyl-2,3,4,4a,9,9a-herahydro-1H-β-carbolin-1-yl)acétate de méthyle

### Stade A : (RS)-(2-Propyl-9-tert-butyloxycarbonyl-2,3,4,9-tétrahydro-1H-β-carbolin-1-yl) acétate de méthyle.

À 370 mg d'une solution du composé du stade B de la préparation 8 dans 7 ml de dichlorométhane anhydre, sont ajoutés 425 mg de Boc₂O et 16 mg de DMAP. Le mélange réactionnel est agité trois heures à température ambiante puis, évaporé sous pression réduite. Une chromatographie éclair sur gel de silice (cyclohexane/acétate d'éthyle 85/15) permet d'obtenir 400 mg du produit attendu.
*infrarouge* (ν_{cm-1}) : 2930; 2873; 2849 (v _{C-H}); 1726 (v _{C=O}); 1607 (v _{C=C}); 1478; 1455 (δ _{C-H}); 1368 (ν_{N-C}); 1243; 1167 (ν_{C-O}).
*Analyse élémentaire* :

| | *C %* | *H %* | *N %* |
|---|---|---|---|
| *Calculé :* | *68,37* | *7,82* | *7,37* |
| *Trouvé :* | *68,45* | *7,98* | *7,25* |

### Stade B : (1RS,4aSR,9aRS)-(2-Propyl-9-tert-butyloxycarbonyl-2,3,4,4a,9,9α-hexahydro-1H-β-carbolin-1-yl)acétate de méthyle.

À une solution de 200 mg du composé du stade A précédent dans 4,5 ml de méthanol, sont ajoutés 20 mg d'oxyde de platine. Le système est dégazé deux fois, placé sous pression d'hydrogène puis, laissé sous agitation sous une pression de 4 bars pendant 24 heures. 20 mg d'oxyde de platine sont à nouveau ajoutés et le milieu réactionnel est agité 24 heures supplémentaires. La suspension est filtrée sur célite (éludant : dichlorométhane) puis, la solution est évaporée sous pression réduite. Une chromatographie éclair sur gel de silice (cyclohexane/acétate d'éthyle : 9/1) permet d'obtenir 100 mg du produit attendu.
*Infrarouge* (ν_{cm-1}) : 2956; 2933; 2873 (v _{C-H}); 1734; 1703 (v _{C=O}); 1603 (v _{C=C}); 1479; 1459 (δ _{C-H}); 1367 (ν _{N-C}); 1254; 1147 (v _{C-O}).

### Stade C : (1RS,4aSR,9aRS)-(2-Propyl-2,3,4,4α,9,9α-hexahydro-1H-β-carbolin-1-yl)acétate de méthyle.

À une solution de 120 mg du composé du stade B précédent dans 1,5 ml de dichlorométhane anhydre à 0 °C, est ajouté 1,5 ml d'acide trifluoroacétique. La solution est agitée 30 minutes à 0 °C puis, 30 minutes à température ambiante. Le mélange réactionnel est concentré sous pression réduite puis, le résidu est repris par du dichlorométhane et de l'eau. La phase aqueuse est alcalinisée par du carbonate de sodium jusqu'à pH 10. La phase organique est récupérée et la phase aqueuse extraite par du dichlorométhane. Les phases organiques réunies sont séchées sur sulfate de sodium, filtrées puis, évaporées sous pression réduite pour donner 85 mg du produit attendu.
*Infrarouge* (ν_{cm-1}) : 3366 (v _{N-H}); 2932; 2872 (ν _{C-H}); 1731 (v c=o); 1610 (v _{C=C}); 1543 (δ _{N-H}); 1484; 1464 (δ _{C-H}); 1371 (v _{N-C}); 1250; 1200 (v _{C-O}).

### PREPARATION 11 : (1SR,4aSR,9aRS)-(2-Propyl-9-tert-butyloxycarbonyl 2,3,4,4a,9,9a-hexahydro-1H-β-carbolin-1-yl)acétate de méthyle

Le produit est obtenu lors de la synthèse du composé du stade B de la préparation 10. Une chromatographie sur colonne de gel de silice (cyclohexane/acétate d'éthyle: 9/1) permet d'obtenir 30 mg du produit attendu.
*Infrarouge* (ν_{cm-1}) : 2933; 2872 (v _{C-H}); 1737; 1690 (v _{C=O}); 1603 (v _{C=C}); 1479; 1460 (δ _{C-H}); 1383; 1367 (ν _{N-C}); 1253; 1165 (v _{C-O}).

### PREPARATION 12 : (1RS, 4αSR, 9αRS)-(9-Nitroso-2-propyl-2,3,4,4α,9,9α-hexahydro-1H-β-carbolin-1-yl)acétate de méthyle.

Le produit est obtenu selon le procédé du stade B de la préparation 9 en remplaçant le composé du stade A de la préparation 9 par le composé du stade C de la préparation 10.
Une chromatographie éclair sur gel de silice (cyclohexane/acétate d'éthyle : 85/15) permet d'obtenir 105 mg du produit attendu.
*Infrarouge* (ν_{cm-1}): 2953 ; 2872 (v _{C-H}); 1735 (v _{C=O}); 1613 (v _{C=C}); 1596 (ν _{N=O}); 1482 ; 1466 (δ _{C-H}); 1363 (ν_{N-C}); 1218 ; 1192 (v _{C-O}).

### PREPARATION 13 : (1RS,4aRS,9aRS)-(9-Amino-2-propyl-2,3,4,4a,9,9a-hexahydro-1H-β-carbolin-1-yl)acétate de méthyle

### Stade A : (1RS, 4aRS, 9aRS)-(9-Nitroso-2-propyl-2,3,4,4a,9,9a-hexahydro-1H-β-carbolin-1-yl)acétate de méthyle.

Le produit est obtenu selon le procédé du stade B de la préparation 9 en remplaçant le composé du stade A de la préparation 9 par le composé du stade C de la préparation 8. Une chromatographie éclair sur gel de silice (cyclohexane/acétate d'éthyle : 85/15) permet d'obtenir 150 mg du produit attendu.
*Infrarouge* (ν_{cm-1}) : 2955; 2932; 2872 (v _{C-H}); 1737 (v _{C=O}); 1612 (v _{C=C}); 1435 (δ _{C-H}); 1367 (v _{C-N}); 1270; 1236 (v _{C-O}).

### Stade B : (1RS,4aRS,9aRS)-(9-Amino-2-propyl-2,3,4,4a,9,9a-hexahydro-1H-carbolin-1-yl)acétate de méthyle.

Le produit est obtenu selon le procédé du stade C de la préparation 9 en remplaçant le composé du stade B de la préparation 9 par le composé du stade A précédent. Une chromatographie éclair sur gel de silice (cyclohexane/acétate d'éthyle : 7/3) permet d'obtenir 90 mg du produit attendu.
*Point de fusion :* 57 °C. *Infrarouge* (ν_{cm-1}) : 3353 (ν_{N-H}); 2955; 2931; 2870; 2809 (ν_{C-H}); 1725 (v _{C=O}); 1458; 1436 (δ _{C-H}); 1360 (ν_{C-N}); 1264; 1235 (v _{C-O}).
*Analyse élémentaire :*

| | *C%* | *H%* | *N%* |
|---|---|---|---|
| *Calculé :* | *67,30* | *8,31* | *13,85* |
| *Trouvé :* | *67,15* | *8,32* | *13,76* |

### PREPARATION 14 : (2SR)-5-Méthoxy-2-[(2RS,3SR)-3-(Méthoxycarbonyl)pipéridin-2-yl]indoline-1-corboxylate de tert-butyle

### Stade A : 5-Méthoxy-1H-indole-1-corboxylate de tert-butyle

À une solution de 7 g de 5-méthoxyindole dans 90 ml de TH sont additionnés 15,57 g de carbonate de di*tert*-butyle, puis, par petites portions, 8,72 g de 4-(diméthylamino)pyridine. La solution est ensuite agitée pendant deux heures à température ambiante puis le mélange réactionnel est hydrolysé avec 100 ml d'eau. La phase organique est ensuite séparée et extraite avec de l'éther diéthylique (3 × 70 ml). Les phases organiques sont rassemblées, lavées avec une solution d'acide chlorhydrique 1 M (150 ml) puis avec une solution saturée de chlorure de sodium. Après séchage sur sulfate de sodium et évaporation sous vide des solvants, on obtient 11,76 g de l'indole protégé.

### Point de fusion : 74-76 °C.

### Stade B : Acide [1-(tert-butoxycarbonyl)-5-méthoxy-1H-indol-2-yl]boronique

À une solution de 10 g de 5-méthoxy-1*H*-indole-1-carboxylate de *tert*-butyle et de 11,41 g de borate de triisopropyle dans 27 ml de THF anhydre est ajoutée, goutte à goutte, sous atmosphère d'argon et à 0 °C, une solution de 26,3 ml de diisopropylamidure de lithium 2 M. Le mélange réactionnel est ensuite amené à température ambiante l'agitation étant maintenue pendant une heure. Une solution aqueuse d'acide chlorhydrique 2 M (70 ml) est alors ajoutée au mélange réactionnel, puis le pH de la phase aqueuse est ensuite ajusté à une valeur de 7 au moyen d'une solution concentrée d'ammoniac. Les phases sont séparées et la phase aqueuse est extraite avec de l'acétate d'éthyle (3 × 70 ml). Les phases organiques sont rassemblées, puis concentrées sous pression réduite. De l'eau (50 ml) et du pentane (50 ml) sont ajoutés à l'huile rouge résiduelle et le ballon est placé dans une cuve à ultrasons pendant une heure. Le précipité ainsi formé est alors filtré puis lavé avec du pentane pour obtenir 10,88 g de l'acide boronique du titre.
*Point de fusion : 110-112 °C.*

### Stade C : 5-Méthoxy-2-[3-(méthoxycarbonyl)pyridin-2-yl]indole-1-carboxylate de tert-butyle

À une solution dégazée de 3,09 g de 2-bromonicotinate de méthyle dans du 1,2-diméthoxyéthane (58 ml) à 85 °C, sont successivement ajoutés une solution de 4,65 g de carbonate de sodium dans l'eau (18 ml) et 1,65 g de tétrakis(triphénylphosphine)palladium. Le mélange réactionnel est maintenu à une température de 85 °C, température à laquelle une solution contenant 5,00 g de l'acide obtenu au stade B dans 18 ml de 1,2-diméthoxyéthane est alors ajoutée goutte à goutte. L'agitation est maintenue pendant 6 heures à 85 °C avant de revenir à température ambiante et d'ajouter de l'eau (60 ml). Les phases sont séparées et la phase aqueuse est extraite avec de l'éther diéthylique (2 × 60 ml) puis avec de l'acétate d'éthyle (2 × 60 ml). Les phases organiques sont rassemblées, lavées avec une solution saturée de chlorure de sodium, séchées sur sulfate de sodium, filtrées et concentrées sous pression réduite. Le résidu est purifié par chromatographie sur colonne de silice en utilisant comme éluant un mélange acétate d'éthyle / cyclohexane (25 / 75) pour donner le produit de couplage attendu sous la forme d'une huile.
*Infrarouge (ν_{cm-1}) : 2980 (ν_{C-H}) ; 1726 (ν_{C=O}).*

### Stade D : (2SR)-5-Méthoxy-2-[(2RS,3SR)-3-(méthoxycarbonyl)pipéridin-2-yl]indoline-1-carboxylate de tert-butyle

Un mélange contenant 5,40 g de 5-méthoxy-2-[3-(méthoxycarbonyl)pyridin-2-yl]indole-1-carboxylate de *tert*-butyle et 5,90 g de rhodium 5% sur alumine dans l'acide acétique glacial (50 ml) est agité à température ambiante sous une pression de 16 bars d'hydrogène pendant 16 heures. Le mélange réactionnel est ensuite filtré sur célite qui est lavée avec du méthanol. Le filtrat est alors concentré sous pression réduite puis le résidu est repris par de l'éther diéthylique (60 ml) et de l'eau (100 ml). Du carbonate de potassium est ajouté jusqu'à pH basique. Les phases sont séparées et la phase aqueuse est extraite avec de l'éther diéthylique (60 ml) puis avec de l'acétate d'éthyle (2 × 60 ml). Les phases organiques sont rassemblées, séchées sur sulfate de sodium, filtrées et concentrées sous pression réduite. Le résidu est ensuite purifié par chromatographie sur colonne de silice en utilisant comme éluant l'éther diéthylique pour conduire à 3,65 g du produit du titre.
*Infrarouge (ν_{cm-1}) : 2934 (ν_{C-H}) 1725 (ν_{C=O}) ; 1693 (ν_{C=O}).*

### PREPARATION 15 :(2RS)-5-Méthoxy-2-[(2RS,3SR)-3-(méthoxycarbonyl)-pipéridin-2-yl]indoline-1-carboxylate de tert-butyle

Le produit est obtenu lors de la synthèse du composé du stade D de la préparation 14.
*Infrarouge* (ν_{cm-1}) : 2936 (ν_{C-H}) ; 1725 (ν_{C=O}) ; 1691 (ν_{C=O}).

### PREPARATION 16 : (2SR)-5-Fluoro-2-[(2RS,3SR)-3-(méthoxycarbonyl)pipéridin-2-yl]indoline-1-carboxylate de tert-butyle

Le produit est obtenu suivant un procédé analogue à celui décrit à la préparation 14 en remplaçant le 5-méthoxyindole par le 5-fluoroindole.
*Infrarouge* (ν_{cm-1}) : 2983 (ν_{C-H}) ; 1735 (ν_{C=O}).

### PREPARATION 17: (2RS)-5-Fluoro-2-[(2RS,3SR)-3-(méthoxycarbonyl)pipéridin-2-yl]indoline-1-carboxylate de tert-butyle

Le produit est obtenu lors de la synthèse du composé du stade D de la préparation 16.
*Point de fusion :* 107-108 °C.

### PREPARATION 18 : 5-Chloro-2-[3-(méthoxycarbonyl)pyridin-2-yl]indole-1-carboxylate de tert-butyle

Le produit est obtenu suivant un procédé analogue à celui décrit aux stades A, B, et C de la préparation 14 en remplaçant le 5-méthoxyindole par le 5-chlororoindole.
*Spectroscopie de masse :* [ES+] m/z = 409 [M+Na]⁺.

### PREPARATION 19 : 2-[3-(Méthoxycarbonyl)-1-méthyl-1,4,5,6-tétrahydropyridin-2-yl]-3-méthylindole-1-carboxylate de tert-butyle

### STADE A : 3-Méthyl-1H-indole-1-carboxylate de tert-butyle

À une solution de 15 g de 3-méthylindole dans 180 ml de tétrahydrofurane sont additionnés 50 g de carbonate de di*tert*-butyle puis 21 g de 4-(diméthylamino)pyridine. La solution est laissée 1 heure sous agitation sous atmosphère d'argon. Le mélange réactionnel est alors hydrolysé avec de l'eau (300 ml), la phase organique est séparée et lavée avec une solution 1 M d'acide chlorhydrique puis une solution saturée de chlorure de sodium. Après séchage sur sulfate de sodium puis évaporation sous vide du solvant, on obtient le produit attendu.
*Point de fusion :* 106 °C.

### STADE B : Acide [1-(tert-butyloxycarbonyl)-3-méthyl-1H-indol-2-yl]boronique

A une solution de 2,1 g du composé obtenu à l'étape précédente dans 25 ml de tétrahydrofurane anhydre, sous atmosphère d'argon, refroidie à -78°C, est ajoutée une solution 1,6 M de n-butyllithium dans 6 ml d'hexane. L'agitation est maintenue pendant 30 min avant d'ajouter une solution de 1,87 g de borate de triisopropyle dans 10 ml de tétrahydrofurane anhydre. Le mélange est maintenu sous agitation pendant 2 heures à température ambiante avant d'ajouter de l'eau (25 ml). Après séparation des phases, la phase aqueuse est extraite par de l'éther diéthylique (2 × 30ml). Les phases organiques sont rassemblées, lavées avec une solution d'acide chlorhydrique 1M séchées sur sulfate de sodium. Le résidu obtenu est mis en suspension dans un mélange eau/pentane pour fournir, après filtration et séchage, le produit attendu.
*RMN ¹H (400 MHz LDMSO-d₆) :* δ (ppm) = 8,15 (s, 2H) ; 8,07 (d, 1H, *J=* 8 Hz) ; 7,51 (d, 1H, *J=* 7 Hz) ; 7,28 (d, 1H, *J=* 8 Hz) ; 7,22 (t, 1H, *J=* 7 Hz) ; 2,20 (s, 3H) ; 1,60 (s, 9H).

### STADE C : 2-[3-(Méthoxycorbonyl)pyridin-2-yl]-3-méthylindole-1-corboxylate de tert-butyle

Le produit est obtenu suivant le même procédé que celui décrit au stade C de la préparation 14 en utilisant une solution de l'acide boronique décrit au stade précédent.
*RMN ¹H (400 MHz; CDCl₃):* δ (ppm) = 8,81 (dd, 1H, *J=* 1,5 4,8 Hz) ; 8,34 (dd, 1H, *J=* 1,8 8,1 Hz) ; 8,19 (d, 1H, *J*=9,0 Hz) ; 7,55 (d, 1H, *J* = 2,1 Hz) ; 7,45 (dd, 1H, *J* = 4,8 7,8 Hz) ; 7,31 (dd, 1H, *J* = 2,1 9,0 Hz) ; 6,57 (s, 1H) ; 3,72 (s, 3H) ; 1,29 (s, 9H).

### STADE D : Iodure de [2-[1-(tert-butoxycarbonyl)-3-méthyl-1H-indol-2-yl]-3-(méthoxycarbonyl)-1-méthyl]pyridinium

Un mélange de 1,8 g du composé obtenu au stade C et de 6,9 ml d'iodométhane est agité à température ambiante pendant 3 jours. Le précipité obtenu est repris par de l'éther diéthylique et filtré. Le résidu est lavé deux fois avec de l'éther diéthylique, puis une dernière fois avec du pentane. Le résidu est séché sous vide pour conduire au produit attendu.
*RMN ¹H (400 MHz ; DMSO-d₆):* δ (ppm) = 9,46 (d, 1H, *J* = 6,5 Hz) ; 9,08 (d, 1H, *J* = 8 Hz) ; 8,45 (dd, 1H, *J =* 6,5 et 8 Hz) ; 8,20 (d, 1H, *J* = 8 Hz) ; 7,76 (d, 1H, *J =* 8 Hz) ; 7,54 (t, 1H, *J=* 8 Hz) ; 7,40 (t, 1H, *J=* 8 Hz) ; 4,16 (s, 3H) ; 3,67 (s, 3H) ; 2,03 (s, 3H) ; 1,31 (s, 9H).

### STADE E : 2-[3-(Méthoxycarbonyl)-1-méthyl-1,4,5,6-tétrahydropyridin-2-yl]-3-méthylindole-1-carboxylate de tert-butyle

Un mélange de 2,1 g du composé obtenu au stade D , de 12,8 ml de triéthylamine et de 187 mg d'oxyde de platine dans 32 ml de méthanol est agité sous atmosphère d'hydrogène pendant 3 h. Le mélange est alors filtré sur célite et le filtrat concentré. Le résidu est repris par de l'éther diéthylique et un précipité blanc apparaît. Ce dernier est éliminé par filtration. La phase organique est séchée sur sulfate de sodium, filtrée et concentrée sous pression réduite. Le résidu obtenu est purifié par chromatographie sur colonne de silice éluée avec un mélange acétate d'éthyle/cyclohexane (30/70) pour conduire au produit attendu.
*Point de fusion : 124 °C.*

### PREPARATION 20 : 2-[3-(Méthoxycarbonyl)-1-méthyl-1,4,5,6-tétrahydropyridin-2-yl]-5-méthylindole-1-carboxylate de tert-butyle

Le produit est obtenu suivant le même procédé que celui décrit à la préparation 19 en remplaçant le 5-méthylindole par le 3-méthylindole.
*RMN ¹H (400 MHz ; CDCl₃):* δ (ppm) = 8,08 (d, 1H, *J=* 8 Hz) ; 7,27 (s, 1H) ; 7,09 (d, 1H, *J*= 8 Hz) ; 6,26 (s, 1H) ; 3,31 (s, 3H) ; 3,20-3,24 (m, 2H) ; 2,64-2,70 (m, 1H) ; 2,60 (s, 3H) ; 2,40 (s, 3H) ; 2,28-2,32 (m, 1H) ; 1,88-2,02 (m, 2H) ; 1,55 (s, 9H).

### PREPARATION 21: (2RS,3SR)-2-[(SR)-5-Méthoxy-1-nitrosoindolin-2-yl]-1-méthyl pipéridine-3-carboxylate de méthyle

### STADE A : (2RS,3SR)-2-[(SR)-5-Méthoxy-1-nitrosoindolin-2-yl]pipéridine-3-carboxylate de méthyle

À une solution de 1,92 g du composé du stade D de la préparation 14 dans 8 ml de dichlorométhane, sont ajoutés 7,60 ml d'acide trifluoroacétique. Le mélange est laissé sous agitation pendant 6 heures à température ambiante, puis concentré sous pression réduite. Le résidu est repris par de l'éther diéthylique et le sel, qui a précipité, est filtré sur fritté puis lavé avec de l'éther diéthylique. Ce solide est alors dissous dans de l'acide acétique glacial (9,5 ml) et de l'eau (9,5 ml). Une solution contenant 339 mg de nitrite de sodium dans 4,3 ml d'eau est alors ajoutée goutte à goutte, à 0 °C, au mélange réactionnel. Après 30 minutes d'agitation à 0 °C, du dichlorométhane (20 ml) et de l'eau (20 ml) sont ajoutés au mélange réactionnel. Du carbonate de potassium est ensuite ajouté jusqu'à pH basique de la phase aqueuse. Les phases sont séparées et la phase aqueuse est extraite avec du dichlorométhane (2 × 25 ml). Les phases organiques sont rassemblées, séchées sur sulfate de sodium, filtrées et concentrées sous pression réduite. Le solide jaune obtenu est alors recristallisé dans un mélange pentane - éthanol pour conduire au produit attendu.
*Point de fusion:* 152-153 °C.

### STADE B : (2RS,3SR)-2-1(SR)-5-Méthoxy-1-nitrosoindolin-2-yl]-1-méthylpipéridine-3-carboxylate de méthyle

À une solution contenant 0.39 g du composé du stade précédent dans 12 ml d'acétonitrile, est ajoutée à température ambiante une solution aqueuse de 0,13 ml de formaldéhyde 37%. La solution est ensuite agitée à température ambiante pendant 40 minutes puis de l'acide acétique (1,35 ml) est ajouté. Après 10 minutes d'agitation, 88 mg de borohydrure de sodium sont ajoutés par petites portions. Le mélange est ensuite agité à température ambiante pendant une heure. De l'eau (30 ml) est ensuite ajoutée et le mélange réactionnel est alcalinisé avec du carbonate de potassium. La phase aqueuse est extraite avec de l'acétate d'éthyle (3 × 20 ml) puis les phases organiques sont rassemblées, séchées sur sulfate de sodium, filtrées et concentrées sous pression réduite. Le résidu obtenu est ensuite purifié par chromatographie sur colonne de silice en utilisant comme éluant un mélange acétate d'éthyle / méthanol (98 / 2) pour conduire au produit attendu.
*Point de fusion* 99-100 °C.

### PREPARATION 22: (2SR, 3SR)-1-[(RS)-1-Aminoindolin-2-yl]-1-méthylpipéridine-3-carboxylate de méthyle

### STADE A : (RS)-2-[(2SR,3SR)-3-(Méthoxycarbonyl)pipéridin-2-yl]indoline-1-carboxylate de tert-butyle

0,54 g de sodium est ajouté par petites portions à 25 ml de méthanol à 0 °C sous atmosphère d'azote. Après dissolution complète du sodium, une solution de 1,68 g du composé du stade A de la préparation 2 dans 25 ml de méthanol est ajoutée à température ambiante. Le mélange est alors porté au reflux pendant 15 heures. Le milieu réactionnel est ensuite ramené à température ambiante puis concentré sous pression réduite. De l'eau est ajoutée et la phase aqueuse est extraite par de l'acétate d'éthyle (3 × 20 ml). Les phases organiques sont rassemblées, séchées sur sulfate de sodium, filtrées et concentrées sous pression réduite. Le résidu est purifié par chromatographie sur gel de silice en utilisant comme éluant un mélange cyclohexane / acétate d'éthyle (70 / 30) pour conduire au produit attendu.
*Point de fusion :* 109 °C.

### STADE B : (2SR, 3SR)-2-[(RS)-Indolin-2-yl]pipéridine-3-carboxylate de méthyle

À une solution de 0,70 g du composé du stade précédent dans 15 ml de dichlorométhane est ajouté de l'acide trifluoroacétique (15 ml). Le mélange est agité à température ambiante pendant une heure puis concentré sous pression réduite. Le résidu est repris par du dichlorométhane (15 ml) et par de l'eau (15 ml). Du carbonate de potassium est ajouté jusqu'à pH basique de la phase aqueuse. Les phases sont séparées et la phase aqueuse est extraite par du dichlorométhane (2 × 10 ml). Les phases organiques sont rassemblées, séchées sur sulfate de sodium, filtrées et concentrées sous vide pour fournir le produit attendu utilisé sans purification supplémentaire.
*Point de fusion :* 99-102 °C.
*Infrarouge (ν_{cm-1}) :* 3353 (ν_{N-H}) ; 2948 et 2869 (ν_{C-H}) 1725 (ν_{C=O}) ; 1486 (vc=c).

### STADE C : (2SR, 3SR)-2-[(RS)-1-Nitrosoindolin-2-yl]pipéridine-3-carboxylate de méthyle

À une solution de 0,43 g du composé du stade précédent dans 8 ml d'acide acétique et 8 ml d'eau à 0 °C, est ajoutée une solution de 110 mg de nitrite de sodium dans 3 ml d'eau. Le mélange est agité à cette température pendant 30 minutes puis du dichlorométhane (20 ml) et de l'eau (10 ml) sont ajoutés. Du carbonate de potassium est alors ajouté jusqu'à pH basqique de la phase aqueuse. Les phases sont séparées. La phase aqueuse est extraite par du dichlorométhane (2 × 10 ml). Les phases organiques sont rassemblées, séchées sur sulfate de sodium, filtrées et concentrées sous pression réduite. Le résidu est alors purifié par chromatographie sur gel de silice en utilsant comme éluant un mélange acétate d'éthyle / cyclohexane (60 / 40) pour conduire au produit attendu.
*Point de fusion :* 100-101°C.

### STADE D : (2SR, 3SR)-1-Méthyl-2-[(RS)-1-nitrosoindolin-2-yl]pipéridine-3-carboxylate de méthyle

À une solution de 101 mg du composé du stade précédent dans 2 ml d'acétonitrile, est ajouté 0,04 ml d'une solution aqueuse de formaldéhyde 37%. Le mélange est agité à température ambiante pendant 40 min puis de l'acide acétique (2 ml) est ajouté. Après 10 minutes, 23 mg de borohydrure de sodium sont ajoutés, et l'agitation est maintenue 40 minutes avant d'y ajouter de l'eau (10 ml) puis du carbonate de potassium jusqu'à l'obtention d'un pH basique. La phase aqueuse est extraite par du dichlorométhane à deux reprises. Les phases organiques sont rassemblées, séchées sur sulfate de sodium, filtrées et concentrées sous pression réduite pour conduire au produit attendu.
*Infrarouge (v* cm⁻¹) : 2947 à 2792 (ν_{C-H}) 1730 (ν_{C=O}) ; 1485 (ν_{C=C}) ; 1428 (ν_{N=O}) ; 1154 (ν_{C-N}).

### STADE E : (2SR, 3SR)-2-[(RS)-1-Aminoindolin-2-yl]-1-méthyl-piyéridine-3-carboxylate de méthyle

À une solution de 99 mg du composé du stade précédent dans 4 ml d'éthanol et 2 ml d'eau à 0°C., sont successivement ajoutés du 0,19 g de zinc et 0,28 g de carbonate d'ammonium. Le mélange est agité à cette température pendant 20 minutes puis filtré sur célite qui est lavée avec de l'eau et du dichlorométhane. Les phases du filtrat sont séparées. La phase aqueuse est extraite par du dichlorométhane à deux reprises. Les phases organiques sont rassemblées, séchées sur sulfate de sodium, filtrées et concentrées sous pression réduite pour conduire au produit attendu.
*Point de fusion :* 122-124 °C.

### PREPARATION 23 : (1RS,4αSR,9αRS)-(2-Benzyl-2,3,4,4α,9,9α-hexahydro-1H-β carbolin-1-yl)acétate de méthyle

### STADE A : (RS)-(2-Benzyl-2,3,4,9-tétrahydro-1H-β-carbolin-1-yl)acétate de méthyle

Le composé est obtenu suivant le même procédé que celui utilisé au stade B de la préparation 8 en remplaçant le propionaldéhyde par le benzaldéhyde.

### STADE B : (1RS,4aSR,9aRS)-(1-Benzyl-1,3,4,4a,9,9a-hexahydro-1H-β-carbolin-1-yl)acétate de méthyle

Le composé est obtenu suivant le même procédé que celui utilisé à la préparation 10 en remplaçant le composé du stade B de la préparation 8 par celui du stade A précédent.

### EXEMPLE 1 : (4aSR,11aSR,11bSR)-1-Méthyl 1,2,3,4,4a,11,11a, 11b-octahydro-1,6,6a-triazabenzo[a]fluorén-5-one

À une solution de 563 mg du composé de la préparation 1 dans 20 ml de THF anhydre à 0 °C, est ajoutée une solution de 2,3 ml de complexe borane-THF 1 M dans le THF goutte à goutte sous atmosphère d'azote. L'agitation est maintenue 30 min à 0 °C et le milieu réactionnel est concentré sous pression réduite. Le résidu est dissous dans 20 ml d'acide trifluoroacétique et le mélange, agité 1 h à température ambiante avant d'être concentré sous pression réduite. Le résidu est repris dans du dichlorométhane et de l'eau. Du carbonate de potassium est ajouté jusqu'à pH basique de la phase aqueuse. La phase aqueuse est extraite par du dichlorométhane à deux reprises. Les phases organiques sont rassemblées, séchées sur sulfate de sodium, filtrées et concentrées sous pression réduite. Le résidu est purifié par chromatographie sur colonne de gel de silice (dichlorométhane/méthanol : 9/1) pour donner un mélange contenant le produit réduit. Ce mélange est dissous dans 10 ml d'acide acétique et 10 ml d'eau et refroidi à 0 °C. Une solution de 0,62 g de nitrite de sodium dans 20 ml l'eau est alors ajoutée goutte à goutte. Après 1 h d'agitation à 0 °C, 20 ml de dichlorométhane sont ajoutés, suivis de carbonate de potassium jusqu'à pH basique de la phase aqueuse. La phase aqueuse est extraite par du dichlorométhane. Les phases organiques réunies sont séchées sur sulfate de sodium, filtrées et concentrées sous pression réduite. Le résidu est dissous dans un mélange de 8 ml d'éthanol et de 4 ml d'eau et refroidi à 0 °C. 0,64 g de zinc et 0,94 g de carbonate d'ammonium sont successivement ajoutés et le mélange réactionnel est agité 15 min à 0 °C avant d'être, filtré. Le zinc est alors lavé avec de l'eau puis, du dichlorométhane. Les phases du filtrat sont séparées. La phase aqueuse est extraite par du dichlorométhane. Les phases organiques réunies sont séchées sur sulfate de sodium, filtrées et concentrées sous pression réduite. Le résidu est repris dans 10 ml de dichlorométhane anhydre et 1,3 ml d'une solution de triméthylaluminium 2.0 M dans l'hexane est additionnée à -20 °C sous atmosphère d'azote. L'agitation est maintenue 1 h à -20 °C puis, le milieu réactionnel est porté au reflux pendant 3 h. La solution est alors jetée dans 20 ml d'une solution aqueuse d'hydroxyde de sodium 20 %. La phase aqueuse est extraite par du dichlorométhane à deux reprises. Les phases organiques sont séchées sur sulfate de sodium, filtrées et concentrées sous pression réduite. Une chromatographie sur colonne de gel de silice (cyclohexane/acétate d'éthyle : 3/7) permet d'obtenir 176 mg du produit attendu.
*Point de fusion :* 132 °C. *Spectrométrie de masse* (ES +, m/z) : 258 (M+H)⁺.
*Analyse élémentaire :*

| | *C%* | *H%* | *N%* |
|---|---|---|---|
| *Calculé :* | *70,01* | *7,44* | *16,33* |
| *Trouvé :* | *69, 79* | *7, 64* | *16,13* |

### EXEMPLE 2 : (4aSR, 11aRS,11bSR)-1-Méthyl-1,2,3,4,4a,11,11a,11b-octahydropyrido[2';3':3,4]pyrrolo[1,2-a]indol-5-one

Le produit est obtenu lors de la synthèse du composé de l'exemple 1.

Une chromatographie sur colonne de gel de silice (cyclohexane/acétate d'éthyle : 3/7) puis (dichlorométhane/méthanol : 95/5) permet d'obtenir 134 mg du produit attendu.
*Point de fusion :* 157-159 °C
*Spectrométrie de masse* (ES +, m/z) : 243 (M+H)⁺.
*Analyse élémentaire :*

| | *C%* | *H%* | *N%* |
|---|---|---|---|
| *Calculé :* | *74,35* | *7,49* | *11,56* |
| *Trouvé :* | *74,51* | *7,67* | *11,38* |

### EXEMPLE 3 : (4aSR, 11aSR, 11bRS)-1-Méthyl 1,2,3,4,4a,11,11a,11b-octahydropyrido[2'3':3,4]pyrrolo[1,2-a]indol-5-one

À une solution de 180 mg du composé de la préparation 2 dans 5 ml de dichlorométhane, sont ajoutés 5 ml d'acide trifluoroacétique à température ambiante. Le mélange réactionnel est agité à cette température pendant 2 h avant d'être concentré sous pression réduite. Le résidu est repris avec du dichlorométhane et de l'eau ; du carbonate de potassium est alors ajouté jusqu'à pH basique de la phase aqueuse. Après séparation des phases, la phase aqueuse est alors extraite par du dichlorométhane. Les phases organiques sont réunies, séchées sur sulfate de sodium, filtrées et concentrées sous pression réduite. Une chromatographie sur colonne de gel de silice (acétate d'éthyle/méthanol : 9/1) permet d'obtenir 85 mg du produit attendu.
*Point de fusion :* 125-127 °C.
*Spectrométrie de masse* (ES +, m/z) : 243 (M+H)⁺.
*Analyse élémentaire* :

| | *C%* | *H%* | *N%* |
|---|---|---|---|
| *Calculé :* | *74,35* | *7,49* | *11,56* |
| *Trouvé :* | *74,09* | *7,29* | *11,49* |

### EXEMPLE 4:[(4aSR,11aSR,11bRS)-5-Oxo-3,4,4α,5,6,11,11α,11b-octahydro-2H-1,6,6a-triazabenzo[a]fluorén-1-yl)]acétonitrile

À une solution de 351 mg du composé de la préparation 4 dans 10 ml d'acétonitrile, sont additionnés 0,1 ml de bromoacétonitrile et 0,5 g de carbonate de potassium à température ambiante. Le mélange réactionnel est agité à température ambiante pendant 3 h avant d'ajouter 20 ml d'eau. Après séparation des phases, la phase aqueuse est extraite par de l'acétate d'éthyle (3 × 10 ml). Les phases organiques sont réunies, séchées sur sulfate de sodium, filtrées et concentrées sous pression réduite. Le résidu est alors dissous dans 10 ml d'éthanol et 5 ml d'eau et la solution, refroidie à 0 °C. 0,51 g de zinc et 0,75 g de carbonate d'ammonium sont successivement ajoutés et le milieu réactionnel est agité à 0 °C pendant 15 min avant d'être filtré. Le solide est lavé par de l'eau puis, par du dichlorométhane. Après séparation des phases, la phase aqueuse est extraite par du dichlorométhane. Les phases organiques sont réunies, séchées sur sulfate de sodium, filtrées et concentrées sous pression réduite. Une chromatographie sur colonne de gel de silice (cyclohexane/acétate d'éthyle : 1/1) permet d'isoler 66 mg du produit attendu.
*Point de fusion:* 223 °C.
*Spectrométrie de masse* (ES +, m/z) : 305 (M+Na)⁺.
*Analyse élémentaire* :

| | *C%* | *H%* | *N%* |
|---|---|---|---|
| *Calculé :* | *68,06* | *6,43* | *19,84* |
| *Trouvé :* | *67,93* | *6,38* | *19,75* |

### EXEMPLE 5 : (4aSR,11aSR,11bRS)-1-Allyl-1,2,3,4,4a,11,11a,11b-octahydropyrido[2',3':3.4]pyrrolo[1,2-a]indol-5-one

À une solution de 80 mg du composé de la préparation 5 dans 3 ml de dichlorométhane, est ajouté de l'acide trifluoroacétique à température ambiante. Le mélange est agité à température ambiante pendant 6 h. Le milieu réactionnel est concentré sous pression réduite. Le résidu est repris par du dichlorométhane et de l'eau. Du carbonate de potassium est ajouté jusqu'à pH basique de la phase aqueuse. Après séparation des phases, la phase aqueuse est extraite par du dichlorométhane. Les phases organiques sont rassemblées, filtrées et concentrées sous pression réduite. Une chromatographie sur colonne de gel de silice (cyclohexane/acétate d'éthyle : 1/1) permet d'isoler 36 mg du produit attendu.
*Point de fusion :* 145 °C.

*Spectrométrie de masse* (ES +, m/z) : 291 (M+Na)⁺.
*Analyse élémentaire :*

| | *C%* | *H%* | *N%* |
|---|---|---|---|
| *Calculé :* | *76,09* | *7,51* | *10,44* |
| *Trouvé :* | *75,84* | *7,76* | *10,33* |

### EXEMPLE 6 : (4aSR, 11aRS,11bSR)-1-Allyl-1,2,3,4,4a,11,11a,11b-octahydropyrido[2 3'.3':3,4]pyrrolo[1,2-a]indol-5-one

Le produit est obtenu selon le procédé de l'exemple 5 en remplaçant le composé de la préparation 5 par le composé de la préparation 6.
*Point de fusion :* 163 °C.
*Spectrométrie de masse* (ES +, m/z) : 291 (M+Na)⁺.
*Analyse élémentaire :*

| | *C%* | *H%* | *N%* |
|---|---|---|---|
| *Calculé :* | *76,09* | *7,51* | *10,44* |
| *Trouvé :* | *76,00* | *7,61* | *10,37* |

### EXEMPLE 7:(4aSR,11aSR,11bRS)-1-Méthyl-1,2,3,4,4a,11,11a,11b-octahydropyrido[2',3':3,4]pyrrolo[1,2-a]indol-5-one

Le produit est obtenu selon le procédé de l'exemple 4 en remplaçant le bromoacétonitrile par l'iodométhane. Une chromatographie sur colonne de gel de silice (acétate d'éthyle/méthanol : 98/2) permet d'obtenir 124 mg du produit attendu.
*Point de fusion :* 231°C.
*Spectrométrie de masse* (ES +, m/z) : 537 (2M+Na)⁺; 280 (M+Na)⁺.
*Analyse élémentaire :*

| | *C %* | *H %* | *N %* |
|---|---|---|---|
| *Calculé :* | *70,01* | *7,44* | *16,33* |
| *Trouvé :* | *70,08* | *7,56* | *16,30* |

### EXEMPLE 8 : (4aSR,11aSR, 11bRS)-1,2,3,4,4a,11,11a,11b-Octahydro-1,6,6a-triazabenzo[a]fluorén-5-one

À une solution de 0,56 g du composé de la préparation 4 dans 18 ml d'éthanol et 9 ml d'eau à 0 °C, sont successivement ajoutés 1,14 g de zinc et 1,67 g de carbonate d'ammonium. Le mélange est agité à 0 °C pendant 20 min puis, filtré. Le solide est lavé avec de l'eau et du dichlorométhane. Les phases du filtrat sont séparées. La phase aqueuse est extraite par du dichlorométhane à deux reprises. Les phases organiques sont réunies, séchées sur sulfate de sodium et concentrées sous pression réduite. Le résidu est purifié par chromatographie sur colonne de gel de silice (dichlorométhane/méthanol 9/1) pour conduire à un mélange de composés. Ce mélange est alors dissous dans 10 ml de dichlorométhane anhydre et refroidi à -20 °C sous atmosphère d'azote. A cette solution, est additionné 1,2 ml d'une solution 2 M de triméthylaluminium. Le mélange réactionnel est agité à -20 °C pendant 90 min puis, porté au reflux pendant 16 h avant d'être refroidi et jeté dans 24 ml d'une solution aqueuse d'acide chlorhydrique 1 M. Les phases sont séparées. Du carbonate de potassium est ajouté à la phase aqueuse jusqu'à l'obtention d'un pH basique. Cette solution est alors extraite par du dichlorométhane à deux reprises. La phase organique est séchée sur sulfate de sodium, filtrée et concentrée sous pression réduite. Une chromatographie sur colone de gel de silice (acétate d'éthyle/méthanol : 9/1) permet d'obtenir 158 mg du produit attendu.
*Point de fusion:* 211 °C.
*Spectrométrie de masse (ES +, m*/*z) :* 266 (M+Na)⁺ ; 244 (M+H)⁺.
*Analyse élémentaire* :

| | *C%* | *H%* | *N%* |
|---|---|---|---|
| *Calculé:* | *69,11* | *7,04* | *17,24* |
| *Trouvé:* | *68,89* | *7,21* | *17,11* |

### EXEMPLE 9 : (4aSR,11aSR,11bRS)-1,2,3,4,4a,11,11a,11b-Octahydropyrido[2',3':3,4]pyrrolo[1,2-a]indol-5-one

Un mélange de 0,99 g du composé du stade A de la préparation 4 et de 2,22 g de carbonate de potassium dans 25 ml d'acétonitrile est porté au reflux pendant 48 h. 20 ml d'eau sont alors ajoutés et les phases sont séparées. La phase aqueuse est extraite par du dichlorométhane à deux reprises. Les phases organiques sont réunies, séchées sur sulfate de sodium, filtrées et concentrées sous pression réduite. Le résidu est alors repris avec 20 ml d'éther diéthylique et filtré pour donner 0,60 g du produit attendu.
*Point de fusion :* 194-196 °C.
*Spectrométrie de masse* (ES +, m/z) : 251 (M+Na)⁺ ; 229 (M+H)⁺.
*Analyse élémentaire :*

| | *C%* | *H%* | *N%* |
|---|---|---|---|
| *Calculé :* | *73,66* | *7,06* | *12,17* |
| *Trouvé :* | *73,41* | *7,17* | *12,19* |

### EXEMPLE 10 : [(4aSR,11aSR,11bRS)-5-Oxo-3,4,4a,11,11a,11b-hexahydro-2H,5H-pyrido[2',3':3,4]pyrrolo[1,2-a]indol-1-yl]acétonitrile

À une solution de 100 mg du composé de l'exemple 9 dans 10 ml d'acétonitrile, sont successivement ajoutés à température ambiante 0,08 ml de bromoacétonitrile et 0,2.g de carbonate de potassium. Le mélange réactionnel est agité à température ambiante pendant 4 h. 10 ml d'eau sont alors additionnés et les phases, séparées. La phase aqueuse est extraite par de l'acétate d'éthyle. Les phases organiques sont réunies, séchées sur sulfate de sodium, filtrées et concentrées sous pression réduite. Une chromatographie sur colonne de gel de silice (acétate d'éthyle/cyclohexane : 6/4) permet d'obtenir 85 mg du produit attendu.
*Point de fusion :* 172-173 °C.
*Spectrométrie de masse* (ES +, m/z) : 290 (M+Na)⁺.
*Analyse élémentaire* :

| | *C%* | *H%* | *N%* |
|---|---|---|---|
| *Calculé :* | *71,89* | *6,41* | *15,72* |
| *Trouvé:* | *71,79* | *6,50* | *15,66* |

### EXEMPLE 11 : (4aSR,11aRS,11bRS)-1-Méthyl-1,2,3,4,4a,11,11a,11b-octahydropyrido[2',3':3,4]pyrrolo[1,2-a]indol-5-one

À 76 mg d'une suspension d'hydrure de sodium 60% dans l'huile dans 5 ml de THF sous atmosphère d'azote, est additionnée à température ambiante une solution de 0,26 g du composé de la préparation 7 dans 5 ml de THF. Le mélange est agité à température ambiante pendant 1 h. De l'eau est ajoutée. Après séparation des phases, la phase aqueuse est extraite par de l'acétate d'éthyle. Les phases organiques sont réunies, séchées sur sulfate de sodium, filtrées et concentrées sous pression réduite. Le résidu est alors purifié par chromatographie sur colonne de gel de silice (cyclohexane/acétate d'éthyle : 6/4) pour donner 120 mg d'un solide qui est repris dans 4 ml de THF et 4 ml d'eau. 100 mg d'hydroxyde de lithium monohydraté est ajouté et le mélange est agité 18 h à température ambiante avant d'ajouter de l'eau. La solution est extraite par l'acétate d'éthyle. La phase organique est lavée avec une solution saturée d'hydrogénocarbonate de sodium, séchée sur sulfate de sodium, filtrée et concentrée sous pression réduite pour donner 90 mg du produit attendu.
*Point de fusion :* 135-136°C. *Spectrométrie de masse* (ES +, m/z) : 265 (M+Na)⁺; 243 (M+H)⁺.
*Analyse élémentaire* :

| | *C%* | *H%* | *N%* |
|---|---|---|---|
| *Calculé :* | *74,35* | *7,49* | *11,56* |
| *Trouvé :* | *74,18* | *7,61* | *11,43* |

### EXEMPLE 12 : (4aSR,11aSR,11bRS)-1-(2-Hydroxyéthyl)-1,2,3,4,4a,11,11a,11b-octahydropyrido[2',3' 3,4]pyrrolo[1,2-a]indol-5-one

Le produit est obtenu selon le procédé de l'exemple 10 en remplaçant le bromoacétonitrile par du bromoéthanol. Une chromatographie sur colonne de gel de silice (acétate d'éthyle/méthanol : 93/7) permet d'obtenir 75 mg du produit attendu.
*Point de fusion :* 158-159 °C.
*Spectrométrie de masse* (ES +, m/z) : 295 (M+Na)⁺.
*Analyse élémentaire* :

| | *C%* | *H%* | *N%* |
|---|---|---|---|
| *Calculé :* | *70,56* | *7,40* | *10,29* |
| *Trouvé :* | *70,69* | *7,43* | *10,20* |

### EXEMPLE 13 : (4aSR, 11aSR,11bRS)-1-Prop-2-ynyl-1,2,3,4,4a,11,11a,11b-octahydropyrido[2'3':3,4]pyrro/o[1,2-a]indo/-5-one

Le produit est obtenu selon le procédé de l'exemple 10 en remplaçant le bromoacétonitrile par du bromure de propargyle. Une chromatographie sur colonne de gel de silice (cyclohexane/acétate d'éthyle : 1/1) permet d'obtenir 88 mg du produit attendu.
*Point de fusion* : 149 °C.
*Spectrométrie de masse* (ES +, m/z) : 289 (M+Na)⁺.
*Analyse élémentaire* :

| | *C %* | *H %* | *N %* |
|---|---|---|---|
| *Calculé :* | *76,66* | *6,81* | *10,52* |
| *Trouvé :* | *76,68* | *6,89* | *10,46* |

### EXEMPLE 14 : (4aSR,11aSR,11bRS)-1-[2-(Pipéridin-1-yl)éthyl]-1,2,3,4,4a,11,11a,11b octahydropyrido[2',3' :3,4]pyrrolo[1,2-a]indol-5-one

À une solution de 154 mg du composé de l'exemple 12 dans 5 ml de dichlorométhane sous atmosphère d'azote, sont ajoutés à 0°C 0,16 ml de triéthylamine puis, 0,06 ml de chlorure de mésyle. Le mélange est agité 30 min à 0°C puis, 1 h à température ambiante avant d'ajouter 10 ml d'une solution saturée en hydrogénocarbonate de sodium. Après séparation des phases, la phase aqueuse est extraite deux fois par du dichlorométhane. Les phases organiques sont réunies, séchées sur sulfate de sodium, filtrées et concentrées sous pression réduite. Le résidu est dissous dans 5 ml d'acétonitrile et 0,17 ml de pipéridine est ajoutée. Le mélange réactionnel est agité à température ambiante pendant 48 h. De l'eau est ajoutée. Après séparation des phases, la phase aqueuse est extraite par du dichlorométhane à deux reprises. Les phases organiques sont réunies, séchées sur sulfate de sodium, filtrées et concentrées sous pression réduite. Une chromatographie sur colonne de gel de silice (dichlorométhane/méthanol : 93/7) pour d'obtenir 63 mg du produit attendu.
*Point de fusion :* 124°C.

*Spectrométrie de masse* (ES +, m/z) : 362 (M+Na)⁺ ; 340 (M+H)⁺ ; 255.
*Analyse élémentaire* :

| | *C%* | *H%* | *N%* |
|---|---|---|---|
| *Calculé :* | *74,30* | *8,61* | *12,38* |
| *Trouvé :* | *74,17* | *8,65* | *11,30* |

### EXEMPLE 15 : (3aSR,6aRS,10cRS)-4-Propyl-(3a,4,5,6,6a,10c-hexahydro)-3H-1,4,10b-triazafluoranthén-2-one

À 220 mg d'une solution du composé de la préparation 9 dans 5 ml de THF anhydre à 0 °C, sont ajoutés 60 mg d'hydrure de sodium à 60 %. La solution est agitée 30 minutes à 0 °C puis, laissée sous agitation 3 heures à température ambiante. La solution est diluée par de l'eau et par du dichlorométhane. La phase organique est récupérée tandis que la phase aqueuse est extraite par du dichlorométhane. Les phases organiques réunies sont séchées sur sulfate de sodium, filtrées puis, évaporées sous pression réduite. Une chromatographie éclair sur gel de silice (CH₂Cl₂/CH₃OH 97/3) permet d'obtenir 120 mg du produit attendu.
*Point de fusion :* 187 °C.
*Spectrométrie de masse* (ESI +, m/z): 272,2 (M + H)⁺.
*Analyse élémentaire :*

| | *C%* | *H%* | *N%* |
|---|---|---|---|
| *Calculé :* | *70,82* | *7,80* | *15,49* |
| *Trouvé :* | *70,74* | *7,89* | *15,40* |

### EXEMPLE 16 : (3aRS,10bSR,10cRS)-3-Propyl-2,3,3a,4,10b,10c-hexahydro-1H benzo[b]pyrido[2,3,4-gh]pyrrolizin-5-one

À une solution de 65 mg du composé de la préparation 10 dans 2 ml de THF anhydre, sont ajoutés à 0 °C 9 mg d'hydrure de sodium à 60 %. Après 30 minutes d'agitation à 0 °C, la solution est agitée 2 heures à température ambiante. La suspension est diluée par de l'eau puis, extraite par du dichlorométhane. La phase aqueuse est extraite deux fois par du dichlorométhane. Les phases organiques réunies sont séchées sur sulfate de sodium, filtrées puis évaporées sous pression réduite. Une chromatographie éclair sur gel de silice (cyclohexane/acétate d'éthyle : 6/4) permet d'obtenir 40 mg du produit attendu.
*Point de fusion :* 103 °C.
*Spectrométrie de masse* (ESI +, m/z): 257,1 (M + H)⁺; 279,1 (M + Na)⁺.
*Analyse élémentaire :*

| | *C%* | *H%* | *N%* |
|---|---|---|---|
| *Calculé :* | *74,97* | *7,86* | *10,93* |
| *Trouvé :* | *74,89* | *7,96* | *10,79* |

### EXEMPLE 17 : (3aRS,6aSR,10cRS)-4-Propyl 3a,4,5,6,6a,10c-hexahydro-3H-1,4,10b-triazafluoranthén-2-one

À une solution de 105 mg du composé de la préparation 12 dans 3,5 ml d'EtOH à 0 °C, sont ajoutés 2 ml d'eau, 290 mg de carbonate d'ammonium et 200 mg de zinc. Le mélange réactionnel est agité 20 min à 0°C. La suspension est filtrée sur fritté (l'insoluble est lavé par du dichlorométhane et par de l'eau). La phase organique est récupérée tandis que la phase aqueuse est extraite par du dichlorométhane. Les phases organiques réunies sont séchées sur sulfate de sodium, filtrées et évaporées sous pression réduite. Une chromatographie éclair, sur gel de silice (cyclohexane/acétate d'éthyle : 7/3) permet d'obtenir 70 mg du produit attendu.
*Point de fusion:* 98 °C.
*Spectrométrie de masse* (ESI +, m/z): 272,2 (M + H)⁺.
*Analyse élémentaire* :

| | *C%* | *H%* | *N%* |
|---|---|---|---|
| *Calculé :* | *70,82* | *7,80* | *15,49* |
| *Trouvé :* | *70,73* | *7,79* | *15,23* |

### EXEMPLE 18 : (3aRS,6aRS,10cRS)-4-Propyl-3a,4,5,6,6a,10c-hexahydro-3H-1,4,10b-triazafluoranthén-2-one

À une solution de 90 mg du composé de la préparation 13 dans 3 ml de dichlorométhane anhydre à -20 °C, sont ajoutés 365 ml de triméthylaluminium en solution 2M dans l'hexane. La solution est agitée 1h 30 sans contrôle de la température puis, diluée avec 2 ml de dichlorométhane anhydre. Le mélange réactionnel est agité 12 h au reflux, à nouveau dilué par du dichlorométhane puis, versé dans une solution aqueuse d'hydroxyde de sodium à 20 % (p/v). La phase organique est récupérée tandis que la phase aqueuse est extraite par du dichlorométhane. Les phases organiques réunies sont séchées sur sulfate de sodium, filtrées et évaporées sous pression réduite. Une chromatographie éclair sur gel de silice (acétate d'éthyle/ méthanol : 97/3) permet d'obtenir 50 mg du produit attendu.
*Point de fusion :* 143 °C. *Infrarouge* (v_{cm-1}) : 3254; 3133 (ν _{N-H}); 3004 (ν_{C-H}); 2952; 2924; 2905; 2870 (ν_{C-H}); 1636 (v _{C=O}); 1607 (v _{C=C}); 1459; 1443 (δ_{C-H}); 1362; 1348 (ν_{C-N}).
*Analyse élémentaire :*

| | *C%* | *H%* | *N%* |
|---|---|---|---|
| *Calculé :* | *70,82* | *7,80* | *15,49* |
| *Trouvé :* | *70,73* | *7,91* | *15,34* |

### EXEMPLE 19 : (4aSR,11aRS,11bSR)-1-Allyl-9-méthoxy-1,2,3,4,4a,11,11a,11b-octahydro-pyrido[2',3':3,4]pyrrolo[1,2-a]indol-5-one

### ETAPE 1 : (2SR)-5-Méthoxy-2-[(2RS,3SR)-1-allyl-3-(méthoxvcarbonyl)pipéridin-2-yl] indoline-1-carboxviate de tert-butyle

À une solution de 2,52 g du composé de la préparation 14 dans 20 ml d'acétonitrile, sont additionnés à température ambiante 4,46 g de carbonate de potassium et 1,68 ml de bromure d'allyle. Le mélange est agité à température ambiante pendant 12 heures puis, de l'eau (30 ml) est ajoutée. Les phases sont séparées et la phase aqueuse est extraite avec de l'acétate d'éthyle (3 × 25 ml). Les phases organiques sont rassemblées, séchées sur sulfate de sodium, filtrées et concentrées sous pression réduite pour fournir le dérivé *N*-allylé sous la forme d'une huile.
*Infrarouge* (ν_{cm-1}) : 2933 (ν_{C-H}) ; 1728 (ν_{C=O}) ; 1692 (ν_{C=O}).

### ETAPE 2 : (2RS)-5-Méthoxy-2-[(2SR,3SR)-1-allyl-3-(méthoxycarbonyl)pipéridin-2-yl] indoline-1-carboxylate de tert-butyle

À une solution de méthanolate de sodium [préparée avec 0,72 g de sodium dissous dans 31 ml de méthanol fraîchement distillé, est additionnée, à température ambiante, une solution de 2,68 g du composé obtenu à l'étape précédente dans du tétrahydrofurane fraîchement distillé (31 ml). Le mélange réactionnel est porté à reflux pendant 3 heures ; de l'eau (30 ml) est ensuite additionnée puis le mélange est concentré sous pression réduite. Le résidu est ensuite repris avec de l'acétate d'éthyle (30 ml) et avec de l'eau (50 ml) ; les phases sont séparées et la phase aqueuse est extraite avec de l'acétate d'éthyle (2 × 30 ml). Les phases organiques sont rassemblées, séchées sur sulfate de sodium, filtrées et concentrées sous pression réduite pour fournir le produit d'isomérisation sous la forme d'une huile.
*Infrarouge* (ν_{cm-1}) : 2931 (ν_{C-H}) ; 1730 (ν_{C=O}) ; 1692 (ν_{C=O}).

### ETAPE 3 : (4aSR,11aRS,11bSR)-1-Allyl-9-méthoxy-1,2,3,4,4a,11,11a,11b-octahydropyrido[2',3':3,4]pyrrolo[1,2-a]indol-5-one

À une solution de 2,48 g du composé obtenu à l'étape 2 dans 9 ml de dichlorométhane, sont ajoutés 9 ml d'acide trifluoroacétique. Le mélange est laissé sous agitation pendant 6 heures à température ambiante puis, concentré sous pression réduite. Le résidu est repris par du dichlorométhane (20 ml) et de l'eau (30 ml). Du carbonate de potassium est ensuite ajouté jusqu'à pH basique. Les phases sont séparées et la phase aqueuse est extraite avec du dichlorométhane (2 × 20 ml). Les phases organiques sont rassemblées, séchées sur sulfate de sodium, filtrées et concentrées sous pression réduite. Le solide obtenu est filtré puis lavé avec du pentane, et finalement recristallisé dans u mélange pentane/éther diéthylique pour fournir 1,52 g du produit attendu.
*Point de fusion : 198-199 °C.*

### EXEMPLE 20 : (4aSR,11aRS,11bSR)-9-Méthoxy-1-propyl-1,2,3,4,4a,11,11a,11b-octahydropyrido[2',3':3.4]pyrrolo[1,2-a]indol-5-one

À une solution de 300 mg du composé de l'exemple 19 dans 15 ml de méthanol est ajoutée une quantité catalytique de palladium sur charbon 10%. Le ballon est ensuite placé sous pression atmosphérique d'hydrogène ; une agitation est maintenue pendant 12 heures à température ambiante. Le mélange réactionnel est alors filtré sur célite qui est lavée avec du méthanol. Après évaporation du solvant sous pression réduite, le résidu obtenu est purifié par chromatographie sur colonne de silice en utilisant comme éluant un mélange acétate d'éthyle / méthanol (95 / 5). Le produit attendu est obtenu sous la forme d'un solide recristallisé dans un mélange pentane - dichlorométhane.
*Point de fusion: 170* °C.

### EXEMPLE 21: (4aSR,11aRS,11bSR)-9-Méthoxy-1-méthyl-1,2,3,4,4a,11,11a,11b-octahydro-pyrido[2',3':3,4]pyrrolo[1,2-a]indol-5-one

### ETAPE 1: mélange de (4aSR,11aRS,11bSR)-9-Méthoxy-1,2,3,4,4a,11,11a,11b-octahydropyrido[2',3':3,4]pyrrolo[1,2-a]indol-5-one et de (4aSR,11aSR,11bRS)-9-méthoxy-1,2,3,4,4a,11,11a,11b-octahydropyrido[2',3':3,4]pyrrolo[1,2-a]indol-5-one

Un ballon contenant 0,45 g du composé de l'exemple 19, 40 mg de palladium sur charbon 10%, 0,2 ml d'acide acétique glacial et 1,60 ml d'eau est porté à reflux pendant 12 heures. Après refroidissement, le mélange réactionnel est ensuite filtré sur célite qui est lavée avec du dichlorométhane (50 ml) et avec de l'eau (80 ml). Après neutralisation du milieu réactionnel au moyen de carbonate de potassium, les phases sont séparées et la phase aqueuse est extraite par du dichlorométhane (2 × 50 ml). Les phases organiques sont ensuite rassemblées, séchées sur sulfate de sodium et évaporées sous pression réduite. Le mélange des deux diastéréomères (inséparables) est purifié par chromatographie sur colonne de silice en utilisant comme éluant un mélange dichlorométhane / méthanol (95 / 5).

### ETAPE 2 : (4aSR, 11aRS,11bSR)-9-Méthoxy-1-méthyl-1,2,3,4,4a,11,11a,11b-octahydropyrido[2',3 :3,4]pyrrolo[1,2-a]indol-5-one

À une solution de 190 mg du mélange de diastéréoisomères précédent dans 8 ml d'acétonitrile, est ajoutée à température ambiante 82 µl d'une solution aqueuse de formaldéhyde 37%. La solution est ensuite agitée à température ambiante pendant 40 minutes puis de l'acide acétique (0,85 ml) est ajouté. Après 10 minutes d'agitation, du borohydrure de sodium (56 mg ; 1,47 mmol) est alors ajouté par petites portions. Le mélange réactionnel est ensuite agité à température ambiante pendant une heure. Puis, de l'eau (30 ml) est ajoutée et le mélange réactionnel est alcalinisé avec du carbonate de potassium. La phase aqueuse est extraite avec de l'acétate d'éthyle (3 × 20 ml) puis les phases organiques sont rassemblées, séchées sur sulfate de sodium, filtrées et concentrées sous pression réduite. Le résidu obtenu est ensuite purifié par chromatographie sur colonne de silice en utilisant comme éluant un mélange acétate d'éthyle / méthanol (95 / 5) pour conduire au produit attendu, qui est recristallisé dans un mélange pentane - éther diéthylique.
*Point de fusion 178 °C.*

### EXEMPLE 22 : (4aSR,11aSR,11bRS)-9-Méthoxy-1-méthyl-1,2,3,4,4a,11,11a,11b-octahydropyrido[2',3':3,4]pyrrolo[1,2-a]indol-5-one

Le composé est obtenu lors de la synthèse du composé de l'exemple 21 à l'issue de l'étape de chromatographie sur colonne de silice en utilisant comme éluant un mélange acétate d'éthyle / méthanol (95 /5). Le produit attendu est ensuite recristallisé dans un mélange pentane - éther diéthylique.
*Point de fusion : 164 °C.*

### EXEMPLE 23 : (4aSR,11aSR,11bRS)-9-Méthoxy-1,2,3,4,4a,11,11a,11b-octahydro-pyrido[2',3':3,4]pyrrolo[1,2-a]indol-5-one

À une solution de 3,70 g du composé de la préparation 14 dans 15 ml de dichlorométhane sont ajoutés 15 ml d'acide trifluoroacétique. Le mélange réactionnel est laissé sous agitation pendant 6 heures à température ambiante puis concentré sous pression réduite. Le résidu est repris par du dichlorométhane (20 ml) et de l'eau (30 ml). Du carbonate de potassium est ensuite ajouté jusqu'à pH basique. Les phases sont séparées et la phase aqueuse est extraite par du dichlorométhane (2 × 20 ml). Les phases organiques sont rassemblées, séchées sur sulfate de sodium, filtrées et concentrées sous pression réduite. Le solide obtenu est filtré et lavé avec du pentane puis finalement recristallisé dans un mélange pentane - éther diéthylique pour conduire au produit attendu.
*Point de fusion: 187* °C.

### EXEMPLE 24 : (4aSR,11aSR,11bRS)-9-Fluoro-1,2,3,4,4a,11,11a,11b-octahydropyrido[2',3':3,4]pyrrolo[1,2-a]indol-5-one

À une solution de 1 g du composé de la préparation 16 dans 4 ml de dichlorométhane sont ajoutés 4 ml d'acide trifluoroacétique. Le mélange est laissé sous agitation pendant 6 heures à température ambiante puis concentré sous pression réduite. Le résidu est repris par du dichlorométhane (20 ml) et de l'eau (30 ml). Du carbonate de potassium est ensuite ajouté jusqu'à pH basique. Les phases sont séparées et la phase aqueuse est extraite avec du dichlorométhane (2 x 20 ml). Les phases organiques sont rassemblées, séchées sur sulfate de sodium, filtrées et concentrées sous pression réduite. Ce résidu est ensuite dissous dans du tétrahydrofurane fraîchement distillé (10 ml) puis additionné, à température ambiante, goutte à goutte et sous atmosphère d'argon, à une suspension de 320 mg d'hydrure de sodium à 60% dans du tétrahydrofurane fraîchement distillé (15 ml). Le mélange est agité à température ambiante pendant 3 heures puis de l'eau (30 ml) et de l'acétate d'éthyle (20 ml) sont ajoutés. Les phases sont séparées et la phase aqueuse est extraite par de l'acétate d'éthyle (2 × 20 ml). Les phases organiques sont rassemblées, séchées sur sulfate de sodium et évaporées sous pression réduite. Le résidu obtenu est purifié par chromatographie sur colonne de silice en utilisant comme éluant un mélange acétate d'éthyle / méthanol (95 / 5) puis finalement recristallisé dans un mélange pentane/éther diéthylique pour fournir le produit attendu.
*Point de fusion : 231 °C.*

### EXEMPLE 25: (4aSR,11aSR,11bRS)-9-Fluoro-1-méthyl1,2,3,4,4a,11,11a,11b-octahydropyrido[2',3':3,4]pyrrolo[1,2-a]indol-5-one

À une solution de 1,47 g du composé de la préparation 16 dans 40 ml d'acétonitrile, est ajoutée à température ambiante une solution aqueuse de formaldéhyde à 37% (0,42 ml). La solution est agitée à température ambiante pendant 40 minutes puis de l'acide acétique (4 ml) est ajouté. Après 10 minutes d'agitation, 280 mg de borohydrure de sodium sont additionnés par petites portions. Le mélange est ensuite agité à température ambiante pendant une heure. De l'eau (30 ml) est ensuite additionnée et le mélange réactionnel est alcalinisé avec du carbonate de potassium. La phase aqueuse est extraite avec de l'acétate d'éthyle (3 × 20 ml) puis les phases organiques sont rassemblées, séchées sur sulfate de sodium, filtrées et concentrées sous pression réduite. Le résidu obtenu est alors dissous dans du dichlorométhane (6 ml) et de l'acide trifluoroacétique (6 ml ; 76,70 mmol) est ajouté. Le mélange est laissé sous agitation pendant 6 heures à température ambiante puis concentré sous pression réduite. Le résidu est repris par du dichlorométhane (20 ml) et de l'eau (30 ml). Du carbonate de potassium est ensuite ajouté jusqu'à pH basique. Les phases sont séparées et la phase aqueuse est extraite avec du dichlorométhane (2 × 20 ml). Les phases organiques sont rassemblées, séchées sur sulfate de sodium, filtrées et concentrées sous pression réduite. Le résidu est purifié par chromatographie sur colonne de silice en utilisant comme éluant un mélange acétate d'éthyle / méthanol (95 / 5), puis recristallisé dans un mélange pentane/éther diéthylique pour fournir le composé attendu.
*Point de fusion : 158 °C.*

### EXEMPLE 26 : (4aSR,11aRS,11bRS)-9-Fluoro-1-méthyl-1,2,3,4,4a,11,11a,11b-octohydro5H-pyrido[2',3':3,4]pyrrolo[1,2-a]indol-5-one

À une solution contenant de 618 mg du composé de la préparation 17 dans 20 ml d'acétonitrile, est ajoutée à température ambiante une solution aqueuse de formaldéhyde à 37% (0,17 ml). La solution est agitée à température ambiante pendant 40 minutes puis de l'acide acétique (2 ml) est ajouté. Après 10 minutes d'agitation, 115 mg de borohydrure de sodium sont additionnés par petites portions. Le mélange est ensuite agité à température ambiante pendant une heure. De l'eau (15 ml) est ensuite additionnée et le mélange réactionnel est alcalinisé avec du carbonate de potassium. La phase aqueuse est extraite avec de l'acétate d'éthyle (3 × 10 ml) puis les phases organiques sont rassemblées, séchées sur sulfate de sodium, filtrées et concentrées sous pression réduite. Le résidu obtenu est alors dissous dans du dichlorométhane (3 ml) et de l'acide trifluoroacétique (3 ml ; 76,70 mmol) est ajouté. Le mélange est laissé sous agitation pendant 6 heures à température ambiante puis concentré sous pression réduite. Le résidu est repris par du dichlorométhane (10 ml) et de l'eau (15 ml). Du carbonate de potassium est ensuite ajouté jusqu'à pH basique. Les phases sont séparées et la phase aqueuse est extraite avec du dichlorométhane (2 × 100 ml). Les phases organiques sont rassemblées, séchées sur sulfate de sodium, filtrées et concentrées sous pression réduite. Ce résidu est ensuite dissous dans du tétrahydrofurane fraîchement distillé (10 ml) puis additionné, à température ambiante, goutte à goutte et sous atmosphère d'argon, à une suspension de 103 mg d'hydrure de sodium à 60% dans 15 ml de tétrahydrofurane fraîchement distillé. Le mélange est agité à température ambiante pendant une heure puis de l'eau (30 ml) et de l'acétate d'éthyle (20 ml) sont ajoutés. Les phases sont séparées et la phase aqueuse est extraite par de l'acétate d'éthyle (2 × 20 ml). Les phases organiques sont rassemblées, séchées sur sulfate de sodium et évaporées sous pression réduite. Le résidu obtenu est purifié par chromatographie sur colonne de silice en utilisant comme éluant un mélange acétate d'éthyle / méthanol (95 / 5) puis recristallisé dans un mélange pentane - éther diéthylique pour fournir le produit attendu.

### EXEMPLE 27: (4aSR,11aSR,11bRS)-9-Chloro-1-méthyl-1,2,3,4,4a,11,11a,11b-octahydropyrido[2',3':3,4]pyrrolo[1,2-a]indol-5-one

### ETAPE 1 : (2SR)-5-Chloro-2-[(2RS,3SR)-3-(méthoxycarbonyl)pipéridin-2-yl]indoline-1-carboxylate de tert-butyle

Un mélange de 4 g du composé obtenu à la préparation 18 et de 4,24 g de rhodium 5 % sur alumine dans 40 ml d'acide acétique glacial, est agité à température ambiante sous 16 bars de pression d'hydrogène pendant 16 h. Le milieu réactionnel est alors filtré sur célite. Le filtrat est concentré sous pression réduite et le résidu est repris par du dichlorométhane et de l'eau. Du carbonate de potassium est ajouté jusqu'à ce que le pH de la phase aqueuse soit basique. Les phases sont séparées et la phase aqueuse est extraite à deux reprises par du dichlorométhane. Les phases organiques sont rassemblées, séchées sur sulfate de sodium, filtrées et concentrées sous pression réduite. Le résidu est alors purifié par chromatographie sur gel de silice avec de l'éther diéthylique contenant 2 % de méthanol pour conduire au produit attendu.
*Spectroscopie de masse :* [ES+] m/z = 417 [M+Na]⁺; 395 [M+H]⁺

### ETAPE 2 : (2SR)-5-Chloro-2-((2RS,3SR)-3-(méthoxycarbonyl)-1-méthylpipéridin-2-yl)indoline-1-carboxylate de tert-butyle

À une solution de 1,34 g du composé obtenu à l'étape précédente dans 40 ml d'acétonitrile, est ajoutée à température ambiante une solution aqueuse de formaldéhyde 37 % (0,4 ml). La solution est agitée à température ambiante pendant 40 min puis de l'acide acétique (4 ml) est ajouté. Après 15 min d'agitation, 245 mg de borohydrure de sodium sont ajoutés par petites portions. Le mélange est alors agité pendant 3 h. Après évaporation du résidu, ce dernier est repris par de l'eau et alcalinisé avec du carbonate de potassium. La phase aqueuse est extraite avec de l'acétate d'éthyle puis les phases organiques sont rassemblées, séchées sur sulfate de magnésium, filtrées et concentrées sous pression réduite. Le résidu est purifié par chromatographie sur colonne de silice éluée avec un mélange dichlorométhane/méthanol (98/2) pour conduire au produit attendu.
*Spectroscopie de masse :* [ES+] m/z = 409 [MTH]⁺

### ETAPE 3 : (4aSR,11aSR,11bRS)-9-Chloro-1-méthyl-1,2,3,4,4a,11,11a,11b-octahydropyrido[2':3':3,4]pyrrolo[1,2-a]indol-5-one

À, une solution de 769 mg du composé obtenu à l'étape précédente dans 20 ml de dichlorométhane est ajouté de l'acide trifluoroacétique (20 ml) à température ambiante. Le mélange est agité à cette température pendant 18 h avant d'être concentré sous pression réduite. Le résidu est repris avec de l'acétate d'éthyle et de l'eau ; du carbonate de potassium est alors ajouté jusqu'à pH basique de la phase aqueuse. Les phases sont séparées, la phase aqueuse est alors extraite par de l'acétate d'éthyle. Les phases organiques sont rassemblées, séchées sur sulfate de magnésium, filtrées et concentrées sous pression réduite. Le résidu est purifié par chromatographie sur gel de silice avec du dichlorométhane contenant 1 % de méthanol pour conduire au produit attendu.
*Point de fusion :* 272 °C.

### EXEMPLE 28 : 9-Chloro-1-méthyl-1,2,3,4-tétrahydropyrido[2',3':3,4]pyrrolo[1,2-a]indo/-5-one

### ETAPE 1 : Iodure de [2-[1-(tert-butoxycarbony/)-5-chloro-1H-indol-2-yl]-3-(méthoxy carbonyl)-1-méthyl]pyridinium

Un mélange de 2,242 g du composé de la préparation 18 et de 9 ml d'iodométhane est agité à température ambiante pendant 3 jours. Le mélange est alors concentré sous pression réduite. Le résidu est lavé une première fois avec de l'éther diéthylique. La phase éthérée est séparée, le résidu séché. Le sel de pyridinium attendu est obtenu par précipitation dans un mélange éther diéthylique/acétate d'éthyle sous la forme d'un solide.
*Spectroscopie de masse :* [ES+] m/z = 401 [M-I]⁺

### ETAPE 2 : 5-Chloro-2-[3-(méthoxycarbonyl)-1-méthyl-1,4,5,6-tétrahydropyridin-2-yl]indole-1-carboxylate de tert-butyle

Un mélange de 1,5 g du composé obtenu à l'étape précédente, de 0,88 ml de triéthylamine et de 129 mg d'oxyde de platine dans 15 ml de méthanol, est agité sous atmosphère d'hydrogène pendant 3 h. Le mélange est alors filtré sur célite et le filtrat, concentré. Le résidu est repris par du dichlorométhane et lavé avec de l'eau. La phase organique est séchée sur sulfate de sodium, filtrée et concentrée sous pression réduite. Le résidu obtenu est purifié par chromatographie sur colonne de silice éluée avec un mélange acétate d'éthyle/cyclohexane (30/70) pour conduire au produit attendu.
*Point de fusion :* 109°C.

### ETAPE 3 : 2-(5-Chloro-1H-indol-2-yl)-1-méthyl-1,4,5,6-tétrahydropyridine-3-carboxylate de méthyle

À une solution de 583 mg du composé obtenu à l'étape précédente dans 5 ml de dicholorométhane, est ajouté de l'acide trifluoroacétique (7 ml). Le mélange est laissé sous agitation pendant 6 h à température ambiante puis, concentré sous pression réduite. Le résidu est repris par de l'eau et du dichlorométhane. Une solution saturée de carbonate de potassium est ajoutée pour alcaliniser la phase aqueuse. Cette dernière est extraite avec du dichlorométhane et de l'acétate d'éthyle. Les phases organiques sont rassemblées, séchées sur sulfate de sodium et concentrées sous pression réduite. Le résidu est purifié par chromatographie sur colonne de silice éluée avec un mélange acétate d'éthyle/cyclohexane (30/70) pour conduire au produit attendu.
*Point de fusion :* 176 °C.

### ETAPE 4 : 9-Chloro-1-méthyl-1,2,3,4-tétrahydropyrido[2',3':3,4]pyrrolo[1,2-a]indol-5-one

À une solution de 350 mg du composé obtenu à l'étape précédente dans 50 ml d'éther diéthylique, sont additionnés de l'Aliquat-336 (0,029 mmol) puis une solution d'hydroxyde de sodium 28,4 % (20 ml) jusqu'à la précipitation partielle dans le milieu réactionnel du composé tétracyclique. Le précipité est resolubilisé dans une grande quantité d'acétate d'éthyle puis les phases sont séparées et la phase aqueuse extraite de nouveau avec de l'acétate d'éthyle. Les phases organiques sont séchées sur sulfate de sodium puis évaporées sous vide. Le produit brut est purifié par chromatographie sur colonne de silice éluée avec un mélange acétate d'éthyle/cyclohexane (50/50) pour conduire au produit attendu. Ce dernier est recristallisé dans un mélange éther diéthylique/acétate d'éthyle.
*Point de fusion :* 176°C.

### EXEMPLE 29 : (4aSR,11bRS)-9-Chloro-1-méthyl-1,2,3,4,4a,11b-hexahydropyrido[2',3':3,4]pyrrolo[1,2-a]indol-5-one

À une solution de 168 mg de composé de l'exemple 28 dans l'acide acétique glacial, sont additionnés par petites fractions 194 mg de cyanoborohydrure de sodium à température ambiante. La solution se décolore peu à peu et est laissée sous agitation pendant trente minutes. L'acide acétique est évaporé, le résidu repris dans l'eau puis alcalinisé avec une solution saturée de carbonate de potassium et enfin la phase aqueuse est extraite avec de l'acétate d'éthyle. Le produit brut est purifié par chromatographie sur colonne de silice éluée avec un mélange acétate d'éthyle/cyclohexane (30/70) pour fournir 146 mg du produit attendu. Ce dernier est recristallisé dans un mélange éther de pétrole/pentane.
*Point de fusion:* 118 °C.

### EXEMPLE 30: (4aSR,11aRS,11bRS)-9-Chloro-1-méthyl-1,2,3,4,4a,11,11a,11b-octahydropyrido[2',3':3,4]pyrrolo[1,2-a]indol-5-one

Un mélange de 780 mg du composé de l'exemple 29 et de 936 mg de rhodium 5 % sur alumine dans 20 ml d'acide acétique glacial, est agité à température ambiante sous 14 bars de pression d'hydrogène pendant 16 h. Le milieu réactionnel est alors filtré sur coton, le filtrat concentré sous pression réduite et le résidu est repris avec de l'eau. Du carbonate de potassium est ajouté jusqu'à pH basique de la phase aqueuse. La phase aqueuse est extraite avec de l'acétate d'éthyle, la phase organique séchée sur sulfate de magnésium, filtrée et concentrée sous pression réduite. Le résidu obtenu est purifié par chromatographie sur colonne de silice éluée avec un mélange acétate d'éthyle/cyclohexane (30/70) pour conduire au produit attendu.
*Point de fusion :* 164 °C.

### EXEMPLE 31: 9-Chloro-1,2,3,4-tétrahydropyrido[2',3',3,4]pyrrolo[1,2-a]indol-5-one

À une solution de 2 g du composé de la préparation 18 dans 8 ml de dichlorométhane, est ajouté de l'acide trifluoroacétique (8 ml). Le mélange est laissé sous agitation pendant 24 h à température ambiante puis concentré sous pression réduite. Le résidu est repris avec de l'eau. Une solution saturée de carbonate de potassium est ajoutée pour alcaliniser la phase aqueuse. Cette dernière est extraite avec de l'acétate d'éthyle. Les phases organiques sont rassemblées, séchées sur sulfate de sodium et concentrées sous pression réduite. Le résidu ainsi obtenu est mis en solution dans l'acétate d'éthyle (30 ml). Puis, sont additionnés de l'Aliquat-336 (0,13 mmol) et une solution d'hydroxyde de sodium 28,4 % (30 ml). Le mélange est laissé sous agitation pendant 24 h à température ambiante. Les phases sont séparées et la phase aqueuse extraite avec de l'acétate d'éthyle. La phase organique est séchée sur sulfate de magnésium puis évaporée sous vide. Le produit brut est ensuite purifié par chromatographie sur colonne de silice éluée avec un mélange acétate d'éthyle/cyclohexane (30/70) pour conduire au produit attendu. Ce dernier est finalement recristallisé dans l'acétate d'éthyle.
*Point de fusion:* 233 °C.

### EXEMPLE 32 : 1-Méthyl-1,2,3,4-tétrahydropyrido[2',3':3,4]pyrrolo[1,2-a]indol-5-one

À une solution de 795 mg du composé obtenu au stade D de la préparation 1 dans 7 ml de dichlorométhane, est ajouté de l'acide trifluoroacétique (4 ml). Le mélange est laissé sous agitation pendant 20 h à température ambiante, puis concentré sous pression réduite. Le résidu est repris par de l'eau et du dichlorométhane. Une solution saturée de carbonate de potassium est ajoutée pour alcaliniser la phase aqueuse. Cette dernière est extraite avec du dichlorométhane et de l'acétate d'éthyle. Les phases organiques sont rassemblées, séchées sur sulfate de sodium et concentrées sous pression réduite. Le résidu ainsi obtenu est solubilisé dans l'acétate d'éthyle (10 ml) ; sont additionnés alors l'Aliquat-336 (0,03 mmol) puis une solution d'hydroxyde de sodium 28,4 % (20 ml) jusqu'à observer la précipitation partielle dans le milieu réactionnel du composé tétracyclique. Le précipité est resolubilisé avec une quantité suffisante d'acétate d'éthyle. Les phases sont séparées et la phase aqueuse extraite de nouveau avec de l'acétate d'éthyle. La phase organique est séchée sur sulfate de sodium puis évaporée sous vide. Le produit brut est purifié par chromatographie sur colonne de silice éluée avec un mélange acétate d'éthyle/cyclohexane (30/70) pour conduire au produit attendu. Ce dernier est finalement recristallisé dans un mélange éther diéthylique/acétate d'éthyle.
*Point de fusion :* 202 °C.

### EXEMPLE 33 : (4aSR,11bRS)-1-Méthyl-1,2,3,4,4a,11b-hexahydropyrido[2',3':3,4] pyrrolo[1,2-a]indol-5-one

À une solution de 296 mg du composé précédent dans l'acide acétique glacial, est additionné, par petites fractions, le cyanoborohydrure de sodium (390 mg) à température ambiante. La solution se décolore peu à peu et est laissée sous agitation pendant 30 min. L'acide acétique est évaporé, le résidu repris dans l'eau puis alcalinisé avec une solution saturée de carbonate de potassium et enfin la phase aqueuse est extraite avec de l'acétate d'éthyle. Le produit brut est purifié par chromatographie sur colonne de silice éluée avec un mélange acétate d'éthyle/cyclohexane (50/50) pour conduire au produit attendu. Ce dernier est recristallisé dans un mélange éther de pétrole/pentane.
*Point de fusion:* 92 °C.

### EXEMPLE 34 : 1,2,3,4-Tétrahydropyrido[2',3';3,4]pyrrolo[1,2-a]indol-5-one

### ETAPE 1: 2-[3-(Méthoxycarbonyl)-1,4,5,6-tétrahydropyridin-2-yl]indole-1-carboxylate de tert-butyle

Un mélange de 2 g du composé obtenu au stade D de la préparation 1 et de 2 g de rhodium 5 % sur alumine dans 70 ml d'acétate d'éthyle est agité sous atmosphère d'hydrogène pendant 3 jours. Le mélange est alors filtré sur célite. La phase organique est séchée sur sulfate de magnésium, filtrée et concentrée sous pression réduite. Le résidu obtenu est purifié par chromatographie sur colonne de silice éluée avec un mélange acétate d'éthyle/cyclohexane (30/70) pour conduire au produit attendu.
*Point de fusion:* 170 °C.

### ETAPE 2 : 1,2,3,4-Tétrahydropyrido[2';3';3,4]pyrrolo[1,2-a]indol-5-one

À une solution de 1,4 g du composé obtenu. à l'étape précédente dans 10 ml de dichlorométhane, est ajouté de l'acide trifluoroacétique (10 ml). Le mélange est laissé sous agitation pendant 24 h à température ambiante puis, concentré sous pression réduite. Le résidu est repris avec de l'eau. Une solution saturée de carbonate de potassium est ajoutée pour alcaliniser la phase aqueuse. Cette dernière est extraite avec de l'acétate d'éthyle. Les phases organiques sont rassemblées, séchées sur sulfate de sodium et concentrées sous pression réduite. Le résidu ainsi obtenu est mis en solution dans 30 ml d'acétate d'éthyle. Y sont additionnés l'aliquat-336 (0,10 mmol) puis une solution d'hydroxyde de sodium 28,4 % (25 ml). Le mélange biphasique est laissé sous agitation pendant 16 h à température ambiante. Les phases sont séparées et la phase aqueuse, extraite avec de l'acétate d'éthyle. La phase organique est séchée sur sulfate de magnésium puis évaporée sous vide. Cette dernière est ajoutée à température ambiante à une suspension de 53 mg d'hydrure de sodium dans 6 ml de tétrahydrofurane. Le mélange est agité pendant trois heures puis hydrolysé et la phase aqueuse extraite avec de l'acétate d'éthyle. Après évaporation, le produit brut est purifié par chromatographie sur colonne de silice éluée avec un mélange acétate d'éthyle/cyclohexane (30/70) pour conduire au produit attendu. Ce dernier est finalement recristallisé dans l'acétate d'éthyle.
*Point de fusion :* 284 °C.

### EXEMPLE 35: 1,11-Diméthyl-1,2,3,4-tétrohydropyrido[2',3':3,4]pyrrolo[1,2-a]indol-5-one

À une solution de 1,2 g du composé de la préparation 19 dans 25 ml de dichlorométhane, est ajouté de l'acide trifluoroacétique (25 ml). Le mélange est laissé sous agitation pendant 3 h à température ambiante puis concentré sous pression réduite. Le résidu est repris par de l'éther diéthylique refroidi dans un bain de glace. Le précipité formé est filtré, lavé abondamment par de l'éther diéthylique refroidi, puis récupéré et séché sous vide. Le solide obtenu est ensuite mis en suspension dans de l'acétate d'éthyle (80 ml), puis est additionnée à 0 °C une solution d'hydroxyde de sodium 28,4 % (20 ml). L'Aliquat-336 est ajouté après retour à température ambiante jusqu'à la précipitation dans le milieu réactionnel du composé tétracyclique. Le mélange réactionnel est maintenu sous agitation pendant 2 h. Les phases sont séparées et la phase aqueuse extraite avec de l'acétate d'éthyle. La phase organique est séchée sur sulfate de sodium, puis évaporée sous vide. Le produit brut est purifié par chromatographie sur colonne de silice éluée avec un mélange acétate d'éthyle/cyclohexane (50/50) pour conduire au produit attendu. Ce dernier est finalement recristallisé dans un mélange éther diéthylique/acétate d'éthyle.
*Point de fusion : 182 °C.*

### EXEMPLE 36 : (11SR, 11aRS)-1,11-Diméthyl-1,2,3,4,11,11a-hexahydropyrido [2',3':3,4]pyrrolo[1,2-a]indol-5-one

À une solution de 400 mg du composé de l'exemple 35 dans 60 ml d'acétate d'éthyle, sont additionnés 450 mg de rhodium 5 % sur alumine. Le mélange est agité à température ambiante sous pression normale d'hydrogène pendant 22 h. Le milieu réactionnel est alors filtré sur célite, le filtrat concentré sous pression réduite. Le résidu est purifié par chromatographie sur une colonne de silice éluée avec un mélange acétate d'éthyle/cyclohexane (50/50) pour conduire au produit attendu. Ce dernier est finalement recristallisé dans un mélange éther diéthylique/acétate d'éthyle.
*Point de fusion :* 188 °C.

### EXEMPLE 37: (4aSR,116RS)-1,11-Diméthyl-1,2,3,4,4a,11b-hexahydropyrido [2',3':3,4]pyrro/o[1,2-a]indol-5-one

À une solution de 130 mg du composé de l'exemple 35 dans 40 ml d'acide acétique glacial est additionné par petites fractions le cyanoborohydrure de sodium (227 mg) à température ambiante. La solution se décolore peu à peu et est laissée sous agitation pendant trente minutes. L'acide est évaporé, le résidu repris dans l'eau puis, alcalinisé avec une solution saturée de carbonate de potassium. La phase aqueuse est extraite avec de l'acétate d'éthyle. Le produit brut est purifié par chromatographie sur colonne de silice éluée avec un mélange acétate d'éthyle/cyclohexane (30/70) pour le produit attendu. Ce dernier est recristallisé dans un mélange éther diéthylique/pentane.
*Point de fusion :* 104 °C.

### EXEMPLE 38 : 1,9-Diméthyl-1,2,3,4-tétrahydropyrido[2',3':3,4]pyrrolo[1,2-a]indol-5-one

Le produit est obtenu suivant le même procédé que celui décrit à l'exemple 35 en remplaçant le composé obtenu à la préparation 19 par celui obtenu à la préparation 20.
*Point de fusion : 214 °C.*

### EXEMPLE 39 : (4aSR;11bRS)-1,9-Diméthyl-1,2,3,4,4a,11b-hexahydropyrido [2',3':3,4]pyrrolo[1,2-a]indol-5-one

Le composé est obtenu suivant le même procédé que celui décrit à l'exemple 37 en remplaçant le composé de l'exemple 35 par le composé de l'exemple 38.
*Point de fusion : 132 °C.*

### EXEMPLE 40 : (11aRS)-1,9-Diméthyl-1,2,3,4,11,11a-hexahydropyrido [2',3':3,4] pyrrolo[1,2-a]indol-5-one

Le composé est obtenu suivant le même procédé que celui décrit à l'exemple 36 en remplaçant le composé de l'exemple 35 par le composé de l'exemple 38. Le résidu ainsi obtenu est purifié par chromatographie sur une colonne de silice éluée avec un mélange acétate d'éthyle/cyclohexane (50/50) pour conduire au composé de l'exemple 39, puis avec de l'acétate d'éthyle pour conduire au composé attendu.
*Point de fusion :-185 °C.*

### EXEMPLE 41: 9-Méthoxy-1-méthyl-1,2,3,4-tétrahydro-pyrido[2':3':3.4]pyrrolo[1,2-a]indol-5-one

Le composé est obtenu selon le même procédé que celui décrit à l'exemple 28 en remplaçant le composé de la préparation 18 par le composé obtenu au stade C de la préparation 14.

### EXEMPLE 42 : (4aSR,11bRS)-9-Méthoxy-1-méthyl-1,2,3,4,4a,11b-hexahydropyrido [2',3 ':3,4]pyrrolo[1,2-a]indol-5-one

Le composé est obtenu selon le même procédé que celui décrit à l'exemple 29 en remplaçant le composé de l'exemple 28 par le composé de l'exemple 41.

### EXEMPLE 43 et EXEMPLE 44 : (4aS,11aR,11bS)-1-Allyl-1,2,3,4,4a,11,11a,11b-octahydropyrido[2',3':3,4]pyrrolo[1,2-a]indol-5-one et (4aR,11aS,11bR)-1-allyl-1,2,3,4a,11,11a,11b-octahydropyrido[2':3':3.4]pyrrolo[1,2-a]indol-5-one

Les composés des exemples 43 et 44 sont préparés par dédoublement du mélange racémique obtenu à l'exemple 6 en appliquant les conditions opératoires suivantes :
HPLC analytique, colonne AD-H CHIRALPAK (amylose tris(3,5-diméthylphénylcarbamate), 4,6mm X 150mm, 5µm; HPLC semi-préparative, colonne AD-H CHIRALPAK (amylose tris(3,5-diméthylphénylcarbamate), 10mm X 250mm, 5µm. Solvant : Heptane / Isopropanol / 0,2% Triéthylamine.
*Temps de rétention :* 9,9 minutes et 12,2 minutes.

### EXEMPLE 45 et EXEMPLE 46 : (4aS,11aS,11bR)-1-Méthyl-1,2,3,4,4a,11,11a,11b-octahydropyrido[2',3':3,4]pyrrolo[1,2-a]indol-5-one et (4aR,11aR,11bS)-1-méthyl 1,2,3,4,4a,11,11a,11b-octahydropyrido[2':3':3,4]pyrrolo[1,2-a]indol-5-one

Les composés des exemples 45 et 46 sont préparés par dédoublement du mélange racémique obtenu à l'exemple 3 en appliquant les conditions opératoires décrites aux exemples 43 et 44.
*Temps de rétention : 4,5 minutes ([α]_{D} = +20°) et 6,0 minutes ([a]_{D} = -20°).*

### EXEMPLE 47 : (4aSR,11aRS,11bSR)-1,2,3,4,4a,11,11a,11b-Octahydropyrido [2',3':3,4]pyrrolo[1,2-a]indol-5-one

À une solution de 140 mg du composé de l'exemple 6 dans 150 µl d'acide acétique (2,61 mmol) et 3 ml d'eau, sont ajoutés 134 mg de Pd/C à 10%. Le mélange réactionnel est porté au reflux du solvant pendant 2 heures. Le mélange est filtré sur célite et le solvant est évaporé sous vide. Le résidu est repris par de l'acide chlorhydrique 1M (10 ml) puis est extrait avec du dichlorométhane (3 x 30 ml). La phase aqueuse est alcalinisée avec une solution saturée de carbonate de sodium jusqu'à pH=10 avant d'être à nouveau extraite avec du dichlorométhane (3 x 30 ml). La phase organique est séchée sur sulfate de sodium, filtrée et concentrée sous pression réduite pour conduire au produit attendu. Ce dernier est ensuite recristallisé dans 2 ml d'acétate d'éthyle.
*Point de fusion : 176 °C.*

### EXEMPLE 48 : (RS)-11,11a-Dihydropyrido[2';3':3,4]pyrrolo[1,2-a]indol-5-one

À une solution de 861 mg du composé obtenu à l'étape 1 de l'exemple 34 dans 5 ml de dichlorométhane, est ajouté de l'acide trifluoroacétique (5 ml). Le mélange est laissé sous agitation pendant 24 h à température ambiante puis concentré sous pression réduite. Le résidu est repris avec de l'eau et du dichlorométhane. Une solution saturée de carbonate de potassium est ajoutée pour alcaliniser la phase aqueuse. Cette dernière est extraite avec du dichlorométhane et de l'acétate d'éthyle. Les phases organiques sont rassemblées, séchées sur sulfate de magnésium et concentrées sous pression réduite. Le produit brut ainsi obtenu est mis en solution dans du tétrahydrofuranne, avant d'être ajouté à 328 mg d'une suspension d'hydrure de sodium dans 10 ml de tétrahydrofuranne à température ambiante. Le mélange est laissé sous agitation pendant 4 h. De l'eau est alors ajoutée puis la phase aqueuse est extraiteplusieurs fois avec de l'acétate d'éthyle. La phase organique est séchée sur sulfate de magnésium, filtrée et concentrée sous pression réduite. Le résidu obtenu est purifié par chromatographie sur colonne de silice éluée avec un mélange acétate d'éthyle/cyclohexane (30/70) pour conduire au composé de l'exemple 34 et au produit attendu.
*Point de fusion : 168 °C.*

### EXEMPLE 49: (4aSR, 11aSR,11bRS)-9-Méthoxy-1-méthyl-1,2,3,4,4a,11,11a,11b-octahydro-1,6,6a-triazabenzo[a]fluorén-5-one

À une solution contenant 335 mg du composé de la préparation 21 dans un mélange éthanol (10 ml) - eau (5 ml), sont ajoutés à une température de 0 °C, 591 mg de zinc et 869 mg de carbonate d'ammonium. L'agitation est maintenue à 0 °C pendant 30 minutes, puis le milieu réactionnel est filtré sur célite qui est lavée avec de l'eau et du dichlorométhane. Les phases sont séparées et la phase aqueuse est extraite par du dichlorométhane (3 x 20 ml). Les phases organiques sont alors rassemblées, séchées sur sulfate de sodium, filtrées et concentrées sous pression réduite. Le résidu obtenu est ensuite purifié par chromatographie sur colonne de silice en utilisant comme éluant un mélange acétate d'éthyle / méthanol (95 / 5) pour conduire au produit attendu.
*Point de fusion 195-197 °C.*

### EXEMPLE 50 : (4aSR,11aRS,11bSR)-1-Méthyl-1,2,3,4,4a,11,11a,11b-octahydro-1,6,6a-triazabenzo[a]fluorén-5-one

À une solution de 80 mg du composé de la préparation 22 dans 10 ml de chlorure de méthylène sous atmosphère d'azote à -20 °C, est ajouté 0,33 ml d'une solution de triméthylaluminium 2 M dans l'hexane. Le mélange est agité une heure à -20 °C puis est porté au reflux pendant 4 heures. Après retour à température ambiante, le milieu réactionnel est versé sur une solution aqueuse d'hydroxyde de sodium 20% (10 ml). Les phases sont séparées. La phase aqueuse est extraite par du dichlorométhane à deux reprises. Les phases organiques sont rassemblées, séchées sur sulfate de sodium, filtrées et concentrées sous pression réduite. Le résidu est purifié par chromatographie sur gel de silice en utilisant comme éluant un mélange acétate d'éthyle / méthanol (98 / 2) pour conduire au produit attendu.
*Spectrométrie de masse (ESI +, m*/*z):* 280 (M+Na, 100%) ; 258 (M+H, 6%) ; 150 (7%).

### EXEMPLE 51 et EXEMPLE 52 : (4aS,11aS,11bR)-1-Méthyl-1,2,3,4,4a,11,11a,11b-octahydropyrido[2',3':3,4]pyrrolo[1,2-a]indol-5-one et (4aR,11aR,11bS)-1-méthyl-1,2,3,4,4a,11,11a,11b-octahydropyrido[2'3':3,4]pyrrolo[1,2-a]indol-5-one

Les composés des exemples 51 et 52 sont préparés par dédoublement du mélange racémique obtenu à l'exemple 7.
*Points de fusion : 168 et 169 °C.*

### EXEMPLE 53 : (3aRS,10bSR,10cRS)-3-Benzyl-2,3,3a,4,10b,10c-hexahydrobenzo [b]pyrido [2,3,4-gh]pyrrolizin-5(1H)-one

Le composé est obtenu suivant un procédé analogue à celui utilisé à l'exemple 16 en remplaçant le composé de la préparation 10 par celui de la préparation 23.
*Points de fusion: 139 °C.*

### ETUDE PHARMACOLOGIQUE DES DERIVES DE L'INVENTION

### EXEMPLE A : Induction de tyrosine hydroxylase

Il est recherché, parmi les dérivés, ceux qui sont capables d'induire une augmentation de la protéine tyrosine hydroxylase (TH) dans le cerveau de la souris Balb/C, au niveau du locus coeruleus (LC).

Les animaux utilisés sont des souris mâles de la lignée pure Balb/C (Charles River Laboratories), âgées de 7 à 8 semaines au moment du traitement.

Les souris reçoivent une injection unique par voie intrapéritonéale du composé à tester, dissous dans une solution d'HCl 0,04 M (contrôle correspondant : HCl 0,004 M) si le composé est suffisamment soluble, ou dans l'huile d'olive 90 % / DMSO 10 % (contrôle correspondant : huile d'olive 90 % / DMSO 10 %) pour les composés insolubles en milieu aqueux.

Trois jours après l'injection de chaque molécule, tous les animaux sont sacrifiés par décapitation. Les cerveaux extraits sont ensuite congelés dans de l'azote liquide et conservés à -80° C.

Des coupes coronales de 8 microns d'épaisseur sont pratiquées le long de l'axe postéro-antérieur du LC et fixées. Les coupes sont transférées sur membranes Immobilon-P. La TH est mesurée par immunodétection et analyse d'image.

### Résultats :

Les résultats d'induction de la TH au niveau du LC sont présentés dans le tableau I suivant.

**TABLEAU I**

| **Mesure de la quantité de TH au niveau des différentes coupes du LC numérotées de 1 à 8 dans le sens antérieur vers postérieur, après administration i.p. (20 mg/kg)** *Les résultats sont exprimés en % par rapport à la valeur moyenne du groupe témoin¹* | | | | | | | | |
|---|---|---|---|---|---|---|---|---|
| % | 1 | 2 | 3 | 4 | 5 | 6 | 7 | 8 |
| Ex.3 | 104 | 97 | 69 | 58 | 49 | 31 | 10 | 8 |
| Ex.6 | 65 | 62 | 50 | 53 | 41 | 27 | 9 | 6 |
| Ex.7 | 59 | 57 | 57 | 51 | 28 | 23 | 13 | 4 |
| Ex. 8 | 59 | 61 | 59 | 50 | 33 | 17 | 9 | 5 |
| Ex.10 | 58 | 50 | 49 | 47 | 34 | 26 | 18 | 1 |
| Ex. 15 | 79 | 75 | 72 | 79 | 33 | 27 | 7 | 5 |
| Ex. 17 | 73 | 72 | 56 | 38 | 34 | 36 | 13 | 3 |
| Ex. 19 | 49 | 48 | 55 | 50 | 21 | 15 | 6 | 9 |
| Ex. 24 | 51 | 71 | 63 | 55 | 44 | 33 | 6 | 3 |
| Ex. 27 | 55 | 67 | 54 | 46 | 26 | 29 | 3 | 5 |
| Ex. 38 | 64 | 99 | 76 | 62 | 27 | 14 | -3 | 5 |
| Ex.40 | 72 | 96 | 66 | 66 | 58 | 34 | 39 | 4 |
| Ex.43 | 69 | 88 | 87 | 60 | 36 | 34 | 9 | 4 |
| Ex.44 | 83 | 99 | 78 | 58 | 38 | 29 | 18 | 3 |
| Ex. 52 | 69 | 86 | 79 | 77 | 40 | 22 | 12 | 1 |
| Ex. 53 | 82 | 68 | 64 | 71 | 53 | 52 | 32 | 18 |

| | | | | | | | | |
|---|---|---|---|---|---|---|---|---|
| ¹animaux traités par le même véhicule | | | | | | | | |

### EXEMPLE B : Affinité pour les récepteurs

L'affinité pour les récepteurs est déterminée selon les méthodes habituelles de relation entre le ligand spécifique et le récepteur, qui peut être d'origine animale ou un recombinant humain. Elle a été déterminée par la méthode du déplacement du ligand spécifique marqué par le composé étudié, et exprimée par la constante de dissociation Kᵢ.
L'affinité réceptorielle pour 28 récepteurs classiques a ainsi été étudiée. L'étude montre que l'induction de TH observée ne passe pas par une affinité vis-à-vis des récepteurs habituellement touchés par les produits psychotropes, comme les récepteurs adrénergiques alpha (de type α₂), 5HT (de type 5HT_{2A}), dopaminergiques (de type D₁ et D₂).
Quelques dérivés montrent une affinité non négligeable pour les récepteurs sigma (σ) (ligand halopéridol) ou muscarinique (M).

**TABLEAU II**

| % d'inhibition concentration (M) | α₂ | | 5HT2A | | D₁ | | D₂ | | M | | σ | |
|---|---|---|---|---|---|---|---|---|---|---|---|---|
| | 10⁻⁷ | 10⁻⁵ | 10⁻⁷ | 10⁻⁵ | 10⁻⁷ | 10⁻⁵ | 10⁻⁷ | 10⁻⁵ | 10⁻⁷ | 10⁻⁵ | 10⁻⁷ | 10⁻⁵ |
| Exemple 3 | - | - | - | 84 | - | 39 | - | - | - | - | - | - |
| Exemple 19 | - | - | - | - | - | - | - | - | - | - | 10 | 36 |
| Exemple 27 | | 10 | | 84 | | | | | | | | 10 |
| Exemple 45 | | 23 | 13 | 92 | | 49 | | | | | | |

### EXEMPLE C : Prédiction de passage de la barrière hémato-encéphalinue (BHE)

Les substances sont incubées à 20 µM dans le compartiment supérieur d'une double cuve séparé du compartiment inférieur soit par un filtre de polycarbonate seul, soit par le même filtre recouvert de cellules endothéliales confluentes de capillaires bovins. L'évaluation de la cinétique de perméabilité est réalisée par quantification LC-MS-MS de la substance inchangée dans le compartiment inférieur après 10, 20, 30, 40 et 60 minutes.
Les dérivés testés présentent un passage généralement élevé de la BHE favorisant la cible neurologique. Les résultats sont rendus sous forme de classes : élevé, intermédiaire, faible. Ainsi, l'exemple 17 présente un passage élevé de la BHE.

### EXEMPLE D : Prédiction de stabilité métabolique

La prédiction de stabilité métabolique est testée par incubation des dérivés à la concentration de 10⁻⁷ M en présence de microsomes hépatiques de souris, rat ou d'homme (0,33 mg prot/ml). Après addition de NADPH (nicotinamide adénine dinucléotide phosphate, forme réduite), des prélèvements sont réalisés à 0, 5, 15, 30 et 60 minutes. La réaction enzymatique est arrêtée avec le méthanol (VN). La protéine est précipitée par centrifugation et le surnageant analysé par LC-MS-MS.
La bonne stabilité métabolique de ces dérivés permet d'envisager un traitement per os.

**TABLEAU III**

| **% de prédiction de stabilité métabolique sur microsomes hépatiques** | | | |
|---|---|---|---|
| | Souris | Rat | Homme |
| Exemple 3 | 61 | 31 | 59 |
| Exemple 8 | 86 | 52 | 80 |
| Exemple 47 | 99 | 85 | 86 |

### EXEMPLE E : Composition pharmaceutique

Formule de préparation pour 1000 comprimés dosés à 10 mg

| | |
|---|---|
| Composé de l'exemple 8 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 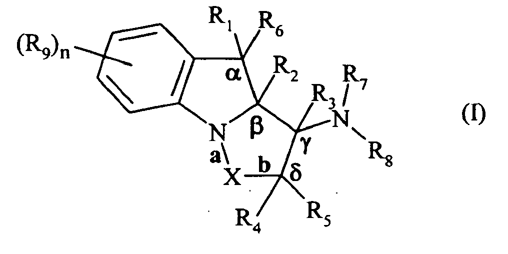 dans laquelle:
- X représente CO ou 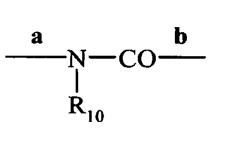
R₁ représente un atome d'hydrogène, un groupement alkyle (C₁-C₆) linéaire ou ramifié, aminoalkyle (C₁-C₆) linéaire ou ramifié, hydroxyalkyle (C₁-C₆) linéaire ou ramifié, arylalkyle (C₁-C₆) linéaire ou ramifié,
R₂ représente un atome d'hydrogène, un groupement alkyle (C₁-C₆) linéaire ou ramifié, aminoalkyle (C₁-C₆) linéaire ou ramifié, hydroxyalkyle (C₁-C₆) linéaire ou ramifié,
ou R₁ et R₂ ensemble avec les atomes de carbone qui les portent forment une liaison carbone carbone,
R₃ représente un atome d'hydrogène, un groupement alkyle (C₁-C₆) linéaire ou ramifié, aminoalkyle (C₁-C₆) linéaire ou ramifié, hydroxyalkyle (C₁-C₆) linéaire ou ramifié,
R₄ représente un atome d'hydrogène, un groupement alkyle (C₁-C₆) linéaire ou ramifié, aminoalkyle (C₁-C₆) linéaire ou ramifié, hydroxyalkyle (C₁-C₆) linéaire ou ramifié,
ou R₃ et R₄ ensemble avec les atomes de carbone qui les portent forment une liaison carbone carbone,
R₅ représente un atome d'hydrogène, un groupement alkyle (C₁-C₆) linéaire ou ramifié,
R₆ représente un atome d'hydrogène, un groupement alkyle (C₁-C₆) linéaire ou ramifié,
R₇, R₈ représentent un atome d'hydrogène, un groupement alkyle (C₁-C₆) linéaire ou ramifié, arylalkyle (C₁-C₆) linéaire ou ramifié, alkényle (C₂-C₆) linéaire ou ramifié, alkynyle (C₂-C₆) linéaire ou ramifié, une chaîne alkyle (C₁-C₆) linéaire ou ramifiée substituée par un ou plusieurs hydroxy, cyano, alkoxy (C₁-C₆) linéaire ou ramifié, NR₁₃R₁₄, une chaîne alkényle (C₁-C₆) linéaire ou ramifiée substituée par les mêmes substituants que ceux définis pour la chaîne alkyle ou, une chaîne alkynyle (C₁-C₆) linéaire ou ramifiée substituée par les mêmes substituants que ceux définis pour la chaîne alkyle,
ou,
soit R₅ et R₈ ensemble avec les atomes de carbone et d'azote qui les portent, forment un hétérocycle à 5, 6 ou 7 chaînons, éventuellement substitué par un groupement R₁₂,
soit R₆ et R₇ ensemble avec les atomes de carbone et d'azote qui les portent, forment un hétérocycle à 5, 6 ou 7 chaînons, éventuellement substitué par un groupement R₁₁,
étant entendu que nécessairement un, mais un seul des deux groupements "R₅ et R₈" ou "R₆ et R₇" ensemble avec les atomes de carbone et d'azote qui les portent, forment un hétérocycle à 5, 6 ou 7 chaînons, éventuellement substitué par un groupement R₁₁,
R₉ représente hydrogène, halogène, alkyle (C₁-C₆) linéaire ou ramifié, alkoxy (C₁-C₆) linéaire ou ramifié, hydroxy, cyano, nitro, polyhalogénoalkyle (C₁-C₆) linéaire ou ramifié, NR₁₅R₁₆, ou une chaîne alkyle (C₁-C₆) linéaire ou ramifiée substituée par un ou plusieurs halogène, un ou plusieurs hydroxy, alkoxy (C₁-C₆) linéaire ou ramifié, ou NR₁₅R₁₆,
n représente un entier 0, 1, 2, 3 ou 4,
R₁₀ représente hydrogène, alkyle (C₁-C₆) linéaire ou ramifié, alkényle (C₂-C₆) linéaire ou ramifié, arylalkyle (C₁-C₆) linéaire ou ramifié, polyhalogénoalkyle (C₁-C₆) linéaire ou ramifié, ou une chaîne alkyle (C₁-C₆) linéaire ou ramifié substituée par un ou plusieurs atomes d'halogène, un ou plusieurs groupements hydroxy, alkoxy (C₁-C₆) linéaire ou ramifié, ou NR₁₅R₁₆,
R₁₁, R₁₂, identiques ou différents, représentent un groupement -COOT ou -CH₂O-U dans lesquels T et U, identiques ou différents, représentent un atome d'hydrogène ou un groupement alkyle (C₁-C₆) linéaire ou ramifié,
R₁₃, R₁₄, identiques ou différents, représentent un atome d'hydrogène ou un groupement alkyle (C₁-C₆) linéaire ou ramifié ou, forment ensemble avec l'atome d'azote qui les porte, un hétérocycle de 4 à 8 chaînons éventuellement substitué, contenant éventuellement une double liaison au sein de l'hétérocycle et contenant éventuellement au sein du système cyclique un second hétéroatome choisi parmi l'atome d'oxygène et l'atome d'azote,
R₁₅, R₁₆, identiques ou différents, représentent un atome d'hydrogène ou un groupement alkyle (C₁-C₆) linéaire ou ramifié, alkényle (C₂-C₆) linéaire ou ramifié,
leurs énantiomères, diastéréoisomères, N-oxydes, ainsi que leurs sels d'addition à un acide ou à une base pharmaceutiquement acceptable,
étant entendu que :
- la (3a*SR*,4*SR*)-3-benzyl-4-éthyl-2,3,3a,4-tétrahydrobenzo[*b*]pyrido[2,3,4-*gh*]pyrrolizin-5(1*H*)-one,
- la pyrido[3',2':4, 5]pyridazino[1,6-*a*]indol-5(6*H*)-one,
- la 6-méthylpyrido[3',2':4, 5]pyridazino[1,6-*a*]indol-5(6*H*)-one,
- la 6-méthyl-1,2,3,4-tétrahydropyrido[3',2':4, 5]pyridazino[1,6-*a*]indol-5(6*H*)-one,
- la (4a*R*,12b*R*)-6-méthyl-1,2,3,4,4a,12b-hexahydropyrido[3',2':4, 5]pyridazino[1,6-*a*] indol-5(6*H*)-one,
- la 1-méthyl-3a,10b-dihydro[1,2,3]triazolo[4',5' :3,4]pyrrolo[1,2-*a*]indol-4(1*H*)-one,
- la 1-benzyl-3a,10b-dihydro[1,2,3]triazolo[4',5' :3,4]pyrrolo[1,2-*a*]indol-4(1*H*)-one, ne font pas partie de l'invention,
par aryle, on entend un groupement phényle ou naphtyle, les deux étant éventuellement substitués par un ou plusieurs atomes d'halogène, groupements nitro, amino, alkyle (C₁-C₆) linéaire ou ramifié, ou alkoxy (C₁-C₆) linéaire ou ramifié,
par hétérocycle à 5, 6 ou 7 chaînons, on entend un groupement monocyclique saturé ou insaturé contenant 5, 6 ou 7 côtés, et possédant un ou deux hétéroatomes choisis parmi azote et oxygène. On peut nommer la pyrrolidine, la pipéridine, l'azépane et la pyridine,
par hétérocycle de 4 à 8 chaînons éventuellement substitué, contenant éventuellement une
ou plusieurs doubles liaisons au sein de l'hétérocycle et contenant éventuellement au sein du système cyclique, un second hétéroatome choisi parmi l'atome d'oxygène ou l'atome d'azote, on peut nommer à titre non limitatif la pyrrolidine, la pipéridine, l'azépane, la pipérazine, la morpholine, ces hétérocycles pouvant éventuellement être substitués, y compris sur le deuxième atome d'azote de la pipérazine par un ou plusieurs groupements, identiques ou différents, choisis parmi alkyle (C₁-C₆) linéaire ou ramifié, hydroxyalkyle (C₁-C₆) linéaire ou ramifié, alkoxy (C₁-C₆)alkyle(C₁-C₆) linéaire ou ramifié, aminoalkyle (C₁-C₆) linéaire ou ramifié, la partie amino étant éventuellement substituée par un ou deux groupements identiques ou différents, alkyle (C₁-C₆) linéaire ou ramifié,
par α, β, γ et δ on entend les centres chiraux éventuellement présents sur les composés de formule (I).
leurs énantiomères et diastéréoisomères ainsi que leurs sels d'addition à un acide ou à une base pharmaceutiquement acceptable.

2. Composés de formule (I) selon la revendication 1 pour lesquels X représente CO, leurs énantiomères et diastéréoisomères ainsi que leurs sels d'addition à un acide ou à une base pharmaceutiquement acceptable.

3. Composés de formule (I) selon la revendication 1 pour lesquels R₁, R₂, R₃ et R₄ représentent chacun un atome d'hydrogène, leurs énantiomères et diastéréoisomères ainsi que leurs sels d'addition à un acide ou à une base pharmaceutiquement acceptable.

4. Composés de formule (I) selon la revendication 1 pour lesquels R₁ et R₂ ensemble avec les atomes de carbone qui les portent forment une liaison carbone carbone, leurs énantiomères et diastéréoisomères ainsi que leurs sels d'addition à un acide ou à une base pharmaceutiquement acceptable.

5. Composés de formule (I) selon la revendication 1 pour lesquels R₃ et R₄ ensemble avec les atomes de carbone qui les portent forment une liaison carbone carbone, leurs énantiomères et diastéréoisomères ainsi que leurs sels d'addition à un acide ou à une base pharmaceutiquement acceptable.

6. Composés de formule (I) selon la revendication 1 pour lesquels n représente un entier 1, leurs énantiomères et diastéréoisomères ainsi que leurs sels d'addition à un acide ou à une base pharmaceutiquement acceptable.

7. Composés de formule (I) selon la revendication 1 pour lesquels R₉ représente un atome d'hydrogène ou d'halogène, ou bien un groupement alkyle (C₁-C₆) linéaire ou ramifié, ou alkoxy (C₁-C₆) linéaire ou ramifié, leurs énantiomères et diastéréoisomères ainsi que leurs sels d'addition à un acide ou à une base pharmaceutiquement acceptable.

8. Composés de formule (I) selon la revendication 1 pour lesquels R₅ et R₈ ensemble avec les atomes de carbone et d'azote qui les portent, forment un hétérocycle à 5, 6 ou 7 chaînons et plus particulièrement un hétérocycle à 6 chaînons, leurs énantiomères et diastéréoisomères ainsi que leurs sels d'addition à un acide ou à une base pharmaceutiquement acceptable.

9. Composés de formule (I) selon la revendication 1 pour lesquels R₆ et R₇ ensemble avec les atomes de carbone et d'azote qui les portent, forment un hétérocycle à 5, 6 ou 7 chaînons et plus particulièrement un hétérocycle à 6 chaînons, leurs énantiomères et diastéréoisomères ainsi que leurs sels d'addition à un acide ou à une base pharmaceutiquement acceptable.

10. Composés de formule (I) qui sont :
(4a*SR,*11a*SR*,11b*RS*)-1-Méthyl-1,2,3,4,4a, 11,11a, 11b-octahydropyrido[2',3':3,4]pyrrolo [1,2-*a*]indol-5-one,
(4a*SR*,11a*RS*, 11b*SR*)-1-Allyl-1,2,3,4,4a,11,11a,11b-octahydropyrido[2',3':3,4]pyrrolo [1,2-a]indol-5-one,
(4a*SR*,11a*SR*,11b*RS*)-1-Méthyl-1,2,3,4,4a,11,11a,11b-octahydropyrido[2',3':3,4]pyrrolo [1,2-*a*]indol-5-one,
(4a*SR*,11a*SR*,11b*RS*)-1,2,3,4,4a,11,11a,11b-Octahydro-1,6,6a-triazabenzo[a]fluorén-5-one,
(4a*SR*,11a*SR*,11b*RS*)-(5-Oxo-3,4,4a,11,11a,11b-hexahydro-2*H*,5*H*-pyrido[2',3':3,4] pyrrolo[1,2-*a*]indol-1-yl)acétonitrile,
(3a*SR*,6a*RS*,10c*RS*)-4-Propyl-(3a,4,5,6,6a,10c-hexahydro)-3*H*-1,4,10b-triazafluoranthén-2-one,
(3a*RS*,6a*SR*,10c*RS*)-4-Propyl-(3a,4,5,6,6a,10c-hexahydro)-3*H*-1,4,10b-triazafluoranthén-2-one,
(4a*SR*,11a*RS*,11b*SR*)-1-Allyl-9-méthoxy-1,2,3,4,4a,11,11a,11b-octahydropyrido [2',3':3,4]pyrrolo[1,2-*a*]indol-5-one,
(4a*SR*,11a*SR*,11b*RS*)-9-Fluoro-1,2,3,4,4a,11,11a,11b-octahydropyrido[2',3':3,4]pyrrolo [1,2-*a*]indol-5-one,
(4a*SR*,11a*SR*,11b*RS*)-9-Chloro-1-méthyl-1,2,3,4,4a,11,11a,11 b-octahydropyrido [2',3':3,4]pyrrolo[1,2-*a*]indol-5-one,
1,9-Diméthyl-1,2,3,4-tétrahydropyrido[2',3':3,4]pyrrolo[1,2-*a*]indol-5-one,
(11a*RS*)-1,9-Diméthyl-1,2,3,4,11,11a-hexahydropyrido[2',3':3,4] pyrrolo[1,2-*a*]indol-5-one,
(4a*S*,11a*R*,11b*S*)-1-Allyl-1,2,3,4,4a,11,11a,11b-octahydropyrido[2',3':3,4]pyrrolo[1,2-*a*] indol-5-one,
(4a*R*,11a*S*,11b*R*)-1-Allyl-1,2,3,4,4a,11,11a,11b-octahydropyrido[2',3':3,4]pyrrolo[1,2-*a*] indol-5-one,
(3a*RS*, 10b*SR*,10c*RS*)-3-Benzyl-2,3,3a,4,10b,10c-hexahydrobenzo[*b*]pyrido [2,3,4-*gh*] pyrrolizin-5(1*H*)-one,
(4a*S*,11aS,11b*R*)-1-Méthyl-1,2,3,4,4a,11,11a,11b-octahydropyrido [2',3':3,4]pyrrolo[1,2-*a*]indol-5-one,
(4a*SR*,11a*RS*,11b*SR*)-1,2,3,4,4a,11,11a,11b-Octahydropyrido [2',3':3,4]pyrrolo[1,2-a] indol-5-one,
(4aR,11aR,11bS)-1-Méthyl-1,2,3,4,4a,11,11a,11b-octahydropyrido[2',3':3,4]pyrrolo[1,2-a]indol-5-one,
leurs énantiomères et diastéréoisomères ainsi que leurs sels d'addition à un acide ou à une base pharmaceutiquement acceptable.

11. Procédé de préparation des composés de formule (I) **caractérisé en ce que** l'on utilise comme produit de départ un composé de formule (II) : 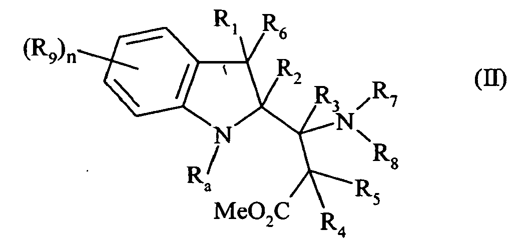 dans laquelle R₁, R₂, R₃, R₄, R₅, R₆, R₇, R₈, R₉ et n sont tels que définis dans la formule (I), Rₐ représente un atome d'hydrogène, un groupement nitroso, amino ou carboxylate de *tert-*butyle,
composé de formule (II) que l'on soumet à une réaction de cyclisation en milieu basique pour conduire aux composés de formule (I), qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation..

12. Procédé de préparation des composés de formule (I/a), cas particulier des composés de formule (I) : 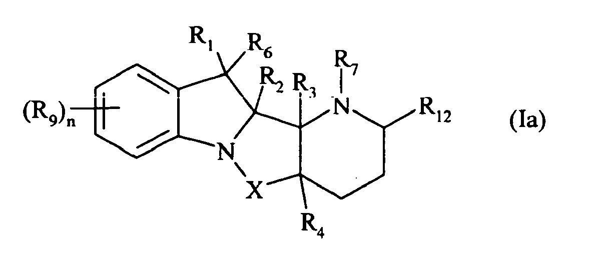 dans laquelle R₁, R₂, R₃, R₄, R₆, R₇, R₉, R₁₂ et n sont tels que définis dans la formule (I),
**caractérisé en ce que** l'on utilise comme produit de départ un composé de formule (III) : 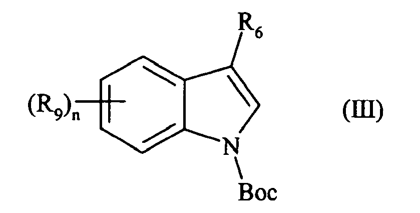 dans laquelle R₆, R₉ et n sont tels que définis dans la formule (I) et Boc représente le groupement carboxylate de *tert*-butyle, qui est mis en réaction avec du borate de triisopropyle en milieu anhydre pour conduire au composé de formule (IV) : 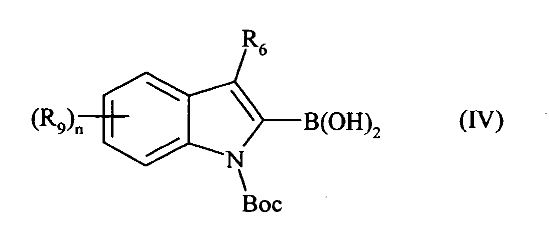 dans laquelle R₆, R₉, n et Boc sont tels que définis précédemment, que l'on fait réagir avec un composé de formule (V) : 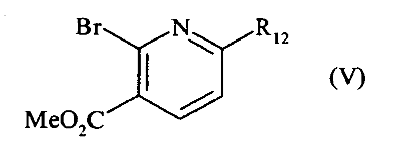 dans laquelle R₁₂ est tel que défini dans la formule (I),
en présence de base et de tétrakis(triphénylphosphine)palladium pour conduire au composé de formule (VI) : 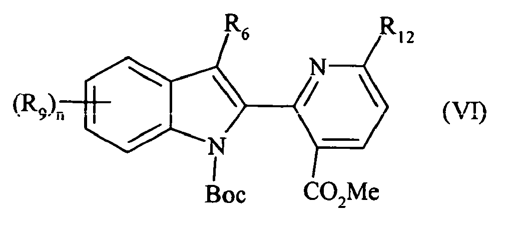 dans laquelle R₆, R₉, R₁₂, n et Boc sont tels que défini précédemment,
composé de formule (VI) que l'on soumet,
- soit à une réaction d'alkylation avec un composé de formule (VII) :
R'₇ - Hal (VII)
dans laquelle R'₇ prend toutes les définitions de R₇ tel que défini dans la formule (I) à l'exception de l'atome d'hydrogène et Hal représente un atome d'halogène,
pour conduire au composé de formule (VIII) : 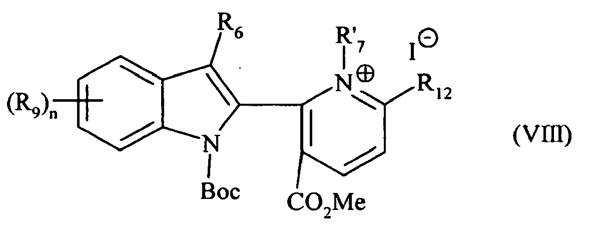 dans laquelle R₆, R'₇, R₉, R₁₂, n et Boc sont tels que définis précédemment,
que l'on soumet à une réaction de réduction partielle avec de l'hydrogène en présence de triéthylamine et d'oxyde de platine pour conduire au composé de formule (IX) : 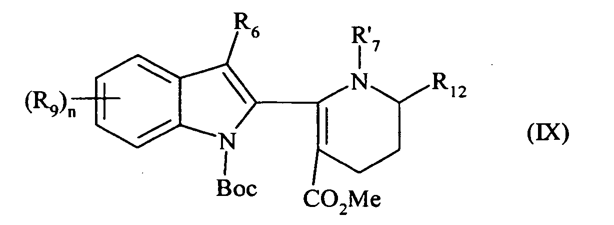 dans laquelle R₆, R'₇, R₉, R₁₂, n et Boc sont tels que définis précédemment,
que l'on soumet à une réduction en présence d'acide chlorhydrique et de cyanoborohydrure de sodium pour conduire au composé de formule (X), de stéréochimie *trans :* 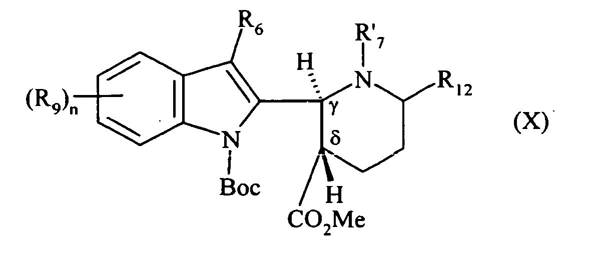 dans laquelle R₆, R'₇, R₉, R₁₂, n et Boc sont tels que définis précédemment, γ et δ sont tels que définis dans la formule (I), et Hγ et Hδ sont de stéréochimie *trans,* qui subit une réaction de déprotection en présence d'acide trifluoroacétique pour conduire au composé de formule (XI), 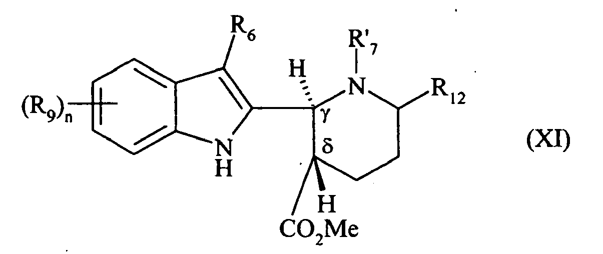 dans laquelle R₆, R'₇, R₉, R₁₂ et n sont tels que définis précédemment,
qui subit une réaction de cyclisation en présence d'un complexe borane-THF et d'acide trifluoroacétique pour conduire au composé de formule (Iₐ₁), cas particulier des composés de formule (I) : 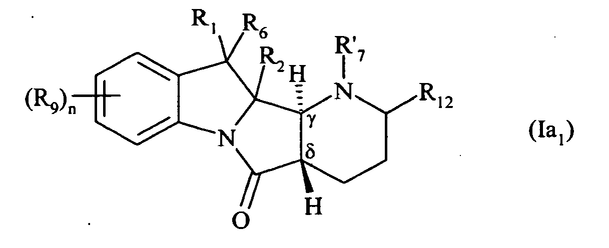 dans laquelle R₆, R'₇, R₉, R₁₂ et n sont tels que définis précédemment, et R₁ et R₂ sont tels que définis dans la formule (I),
- soit à une réaction de réduction avec de l'hydrogène en présence d'acide acétique et d'un catalyseur au rhodium pour conduire aux composés de formules (XIIa) et (XIIb), de stéréochimie (*trans, cis*) et (*cis,cis*) respectivement : 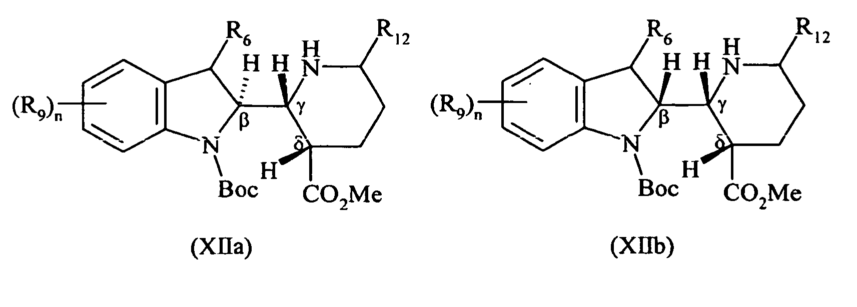
dans lesquelles R₆, R₉, R₁₂, n et Boc sont tels que définis précédemment, β, γ et δ sont tels que définis dans la formule (I) et H_{β} et H_{γ} sont de stéréochimie *trans* dans le composé de formule (XIIa) et de stéréochimie *cis* dans le composé de formule (XIIb) et H_{γ} et H_{δ} sont de stéréochimie *cis* dans les composés de formules (XIIa) et (XIIb),
composés de formule (XIIa) et (XIIb) qui sont :
• soit mis à réagir avec un composé de formule (VII) tel que défini précédemment en présence de base pour conduire aux composés de formules (XIIIa) et (XIIIb) : 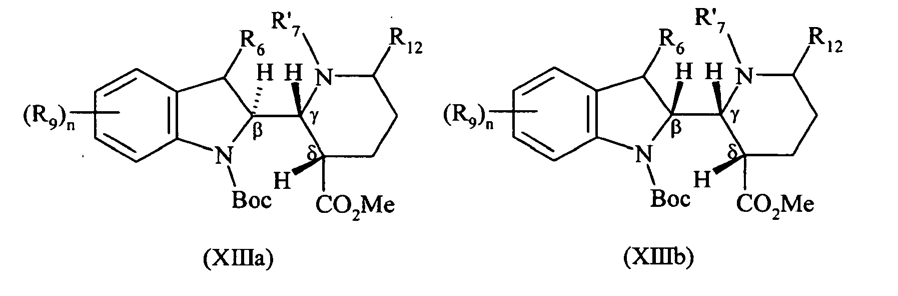
dans laquelle R₆, R'₇, R₉, R₁₂, n et Boc sont tels que définis précédemment,
composés de formules (XIIIa) et (XIIIb) qui sont :
□ soit mis en présence d'acide trifluoroacétique pour conduire aux composés de formules (Iₐ₂) et (XIVb), composés de formule (Iₐ₂), cas particulier des composés de formule (I) : 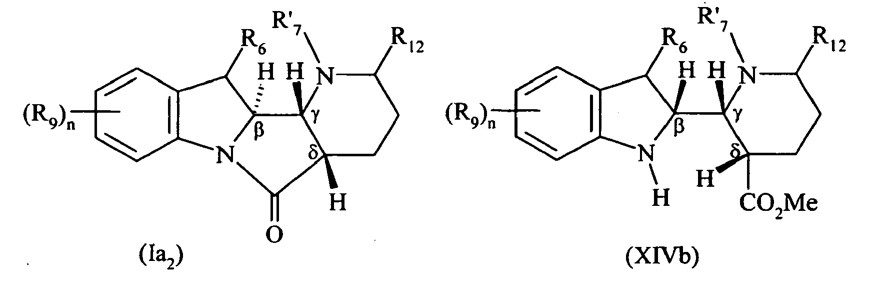
dans lesquelles R₆, R'₇, R₉, R₁₂, et n sont tels que définis précédemment,
composé de formule (XIVb) qui subit une réaction de cyclisation en présence d'hydrure de sodium pour conduire au composé de formule (Iₐ₃), cas particulier des composés de formule (I) : 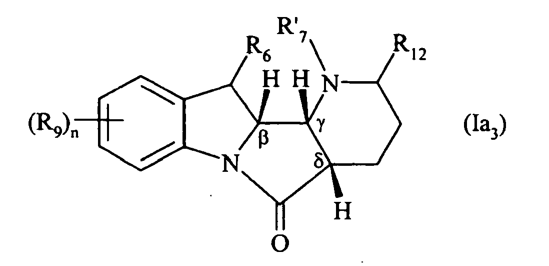 dans lesquelles R₆, R'₇, R₉, R₁₂, et n sont tels que définis précédemment,
composé de formule (XIIIa) qui est :
□ soit mis en présence de méthylate de sodium dans du méthanol pour conduire au composé de formule (XV) : 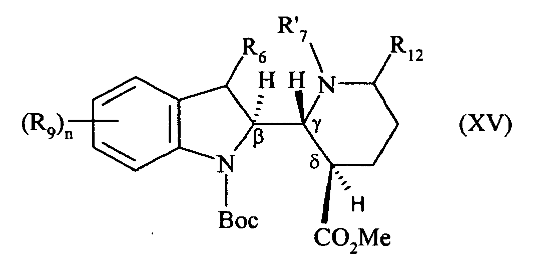
dans lesquelles R₆, R'₇, R₉, R₁₂, n et Boc sont tels que définis précédemment,
qui subit une réaction de cyclisation en présence d'acide trifluoroacétique pour conduire au composé de formule (Iₐ₄), cas particulier des composés de formule (I) : 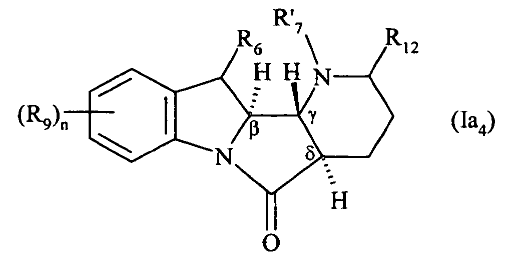 dans laquelle R₆, R'₇, R₉, R₁₂ et n sont tels que définis précédemment,
composé de formule (XIIa) qui est :
• soit soumis aux mêmes conditions que les composés de formules (XIIIa) et (XIIIb) pour conduire au composé de formule (XVI) : 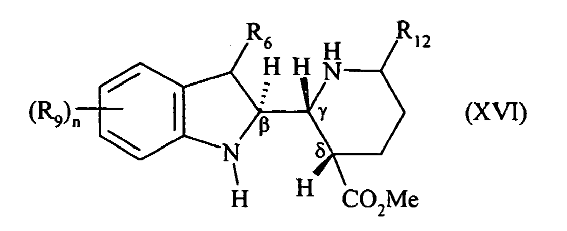
dans laquelle R₆, R₉, R₁₂ et n sont tels que définis précédemment,
composé de formule (XVI) qui est :
* soit dissous dans un mélange d'acide acétique et d'eau et une solution de nitrite de sodium pour conduire au composé de formule (XVII) : 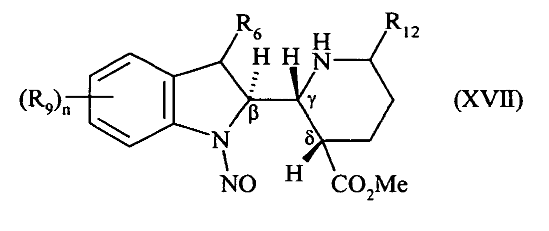
dans laquelle R₆, R₉, R₁₂ et n sont tels que définis précédemment,
qui est successivement mis à réagir avec un composé de formule (VII) tel que défini précédemment, puis du zinc et du carbonate d'ammonium pour conduire au composé de formule (Iₐ₅), cas particulier des composés de formule (I) : 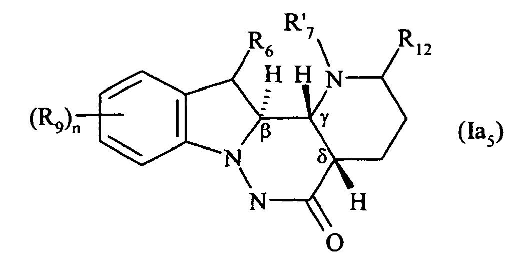 dans laquelle R₆, R'₇, R₉, R₁₂ et n sont tels que définis précédemment,
composé de formule (XVI) qui est :
* soit soumis à une réaction de cyclisation en présence de carbonate de potassium pour conduire au composé de formule (Iₐ₆), cas particulier des composés de formule (I) : 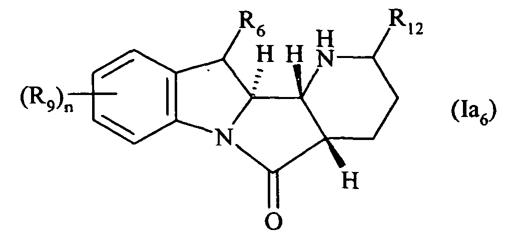
dans laquelle R₆, R₉, R₁₂ et n sont tels que définis précédemment,
qui subit une réaction d'alkylation avec un composé de formule (VII) tel que défini précédemment en milieu basique pour conduire au composé de formule (Iₐ₇), cas particulier des composés de formule (I) : 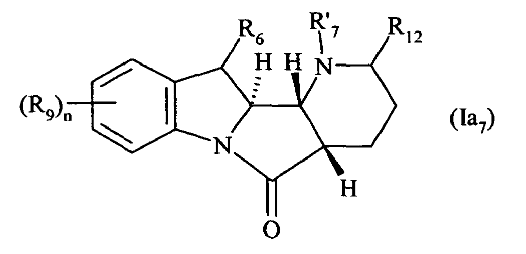 dans laquelle R₆, R'₇, R₉, R₁₂ et n sont tels que définis précédemment,
les composés de formules (Iₐ₁) à (Iₐ₇) forment les composés de formule (Ia), qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

13. Procédé de préparation des composés de formule (Ib), cas particulier des composés de formule (I) : 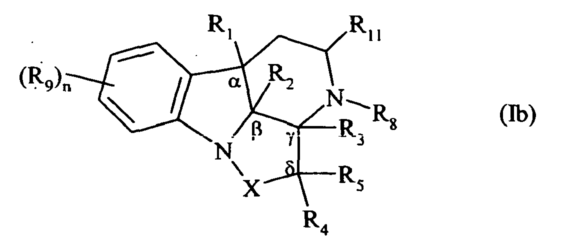 dans laquelle R₁, R₂, R₃, R₄, R₅, R₈, R₉, R₁₁, X et n sont tels que définis dans la formule (I)
**caractérisé en ce que** l'on utilise comme produit de départ un composé de formule (XVIII) : 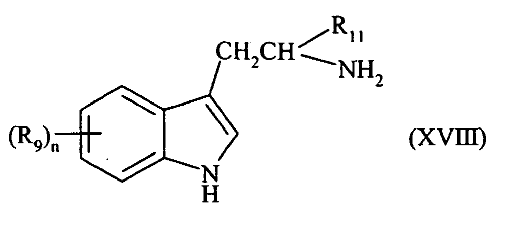 dans laquelle R₉, R₁₁ et n sont tels que définis précédemment,
qui est mis en réaction avec du cyanoacétate de méthyle en présence de Pd/C dans de l'acide acétique glacial pour conduire au composé de formule (XIX) : 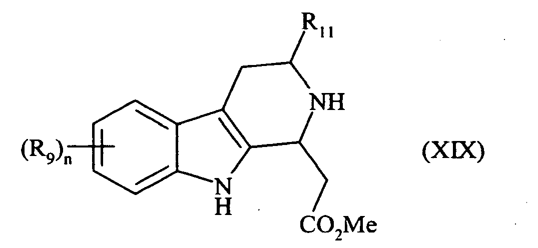 dans laquelle R₉, R₁₁ et n sont tels que définis précédemment,
qui est mis en réaction avec un composé de formule (XX) :
R"₇ - CHO (XX)
dans laquelle R"₇ prend toutes les définitions de R₇ tel que défini dans la formule (I) à l'exception de l'atome d'hydrogène, suivi d'une réduction avec du borohydrure de sodium
ou du cyanoborohydrure de sodium pour conduire au composé de formule (XXI) : 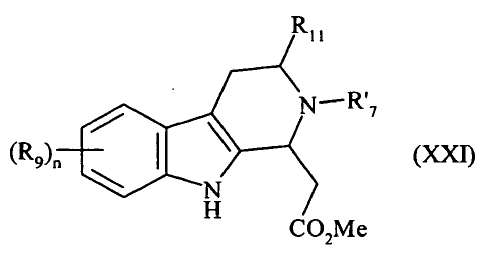 dans laquelle R₉, R₁₁ et n sont tels que définis précédemment et R'₇ prend toutes les définitions de R₇ tel que défini dans la formule (I) à l'exception de l'atome d'hydrogène,
composé de formule (XXI) qui est :
- soit mis à réagir avec un complexe BH₃-THF dans du THF puis mis en solution avec de l'acide trifluoroacétique pour conduire aux composés de formules (XXIIa) et (XXIIb), de stéréochimie (*trans, cis*) et (*trans, trans*) respectivement : 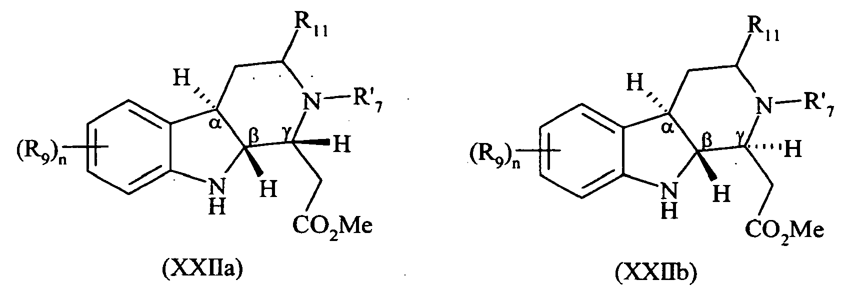
dans lesquelles R'₇, R₉, R₁₁ et n sont tels que définis précédemment, et H_{α} et H_{β} sont de stéréochimie *trans* dans les composés de formules (XXIIa) et (XXIIb) et H_{β} et H_{γ} sont de stéréochimie *cis* dans le composé de formule (XXIIa) et de stéréochimie *trans* dans le composé de formule (XXIIb),
qui sont dissous dans un mélange d'acide acétique et d'eau et une solution de nitrite de sodium pour conduire aux composés de formules (XXIIIa) et (XXIIIb) : 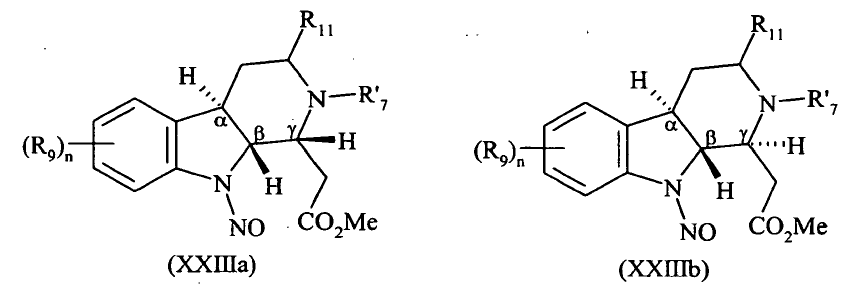 dans lesquelles R'₇, R₉, R₁₁ et n sont tels que définis précédemment,
qui sont mis en présence de carbonate d'ammonium et de zinc pour conduire aux composés de formules (XXIVa) et (XXIVb) : 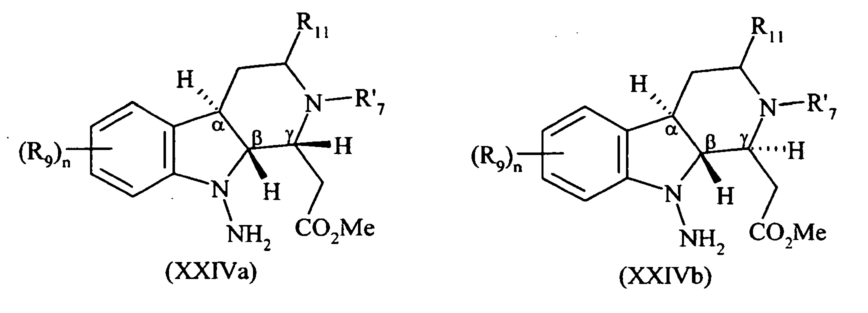 dans lesquelles R'₇, R₉, R₁₁, et n sont tels que définis précédemment,
qui subissent une réaction de cyclisation en présence d'hydrure de sodium ou de triméthylaluminium en solution dans l'hexane pour conduire aux composés de formules (I_{b1}) et (I_{b2}), cas particuliers des composés de formule (I) : 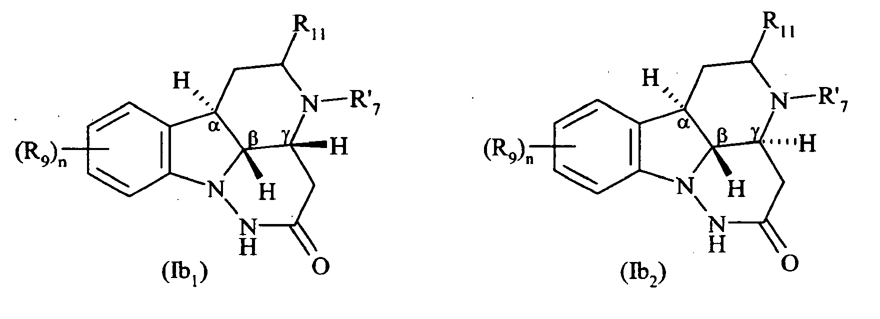 dans lesquelles R'₇, R₉, R₁₁ et n sont tels que définis précédemment,
composé de formule (XXI) qui est :
- soit mis à réagir avec l'anhydride de l'acide di-*tert*-butyloxycarbonique et du diméthylaminopyridine pour conduire au composé de formule (XXV) : 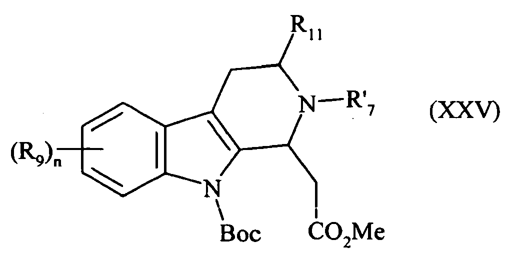
dans laquelle R'₇, R₉, R₁₁ et n sont tels que définis précédemment et Boc représente le groupement carboxylate de *tert*-butyle, qui subit une réaction de réduction en présence d'oxyde de platine et d'hydrogène pour conduire aux composés de formules (XXVIa) et (XXVIb), de stéréochimie (*cis, cis*) et (*cis, trans*) respectivement : 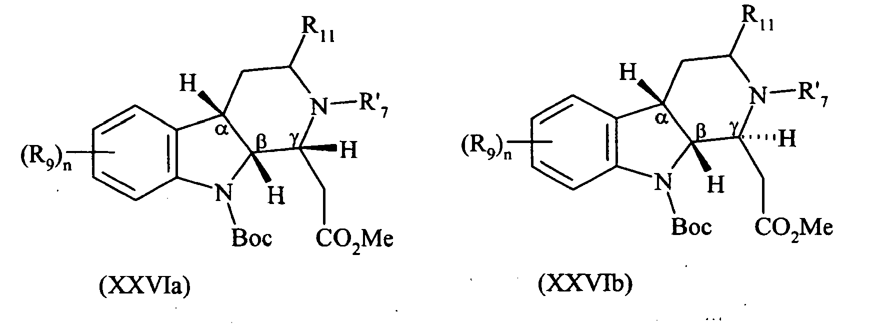 dans lesquelles R'₇, R₉, R₁₁, n et Boc sont tels que définis précédemment,
et H_{α} et H_{β} sont de stéréochimie *cis* dans les composés de formules (XXVIa) et (XXVIb) et H_{β} et Hγ sont de stéréochimie *cis* dans le composé de formule (XXVIa) et de stéréochimie *trans* dans le composé de formule (XXVIb),
qui sont mis en présence d'acide trifluoroacétique dans un milieu anhydre pour conduire aux composés de formules (XXVIIa) et (XXVIIb) : 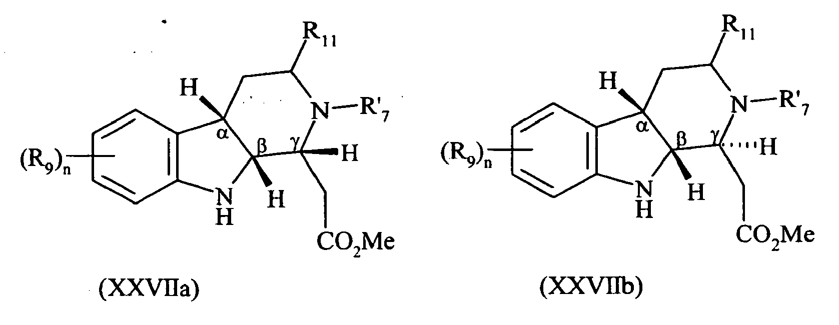 dans lesquelles R'₇, R₉, R₁₁ et n sont tels que définis précédemment,
qui subissent :
• soit une réaction de cyclisation dans les mêmes conditions que les composés de formules (XXIVa) et (XXIVb) pour conduire aux composés de formules (I_{b3}) et (I_{b4}), cas particulier des composés de formule (I) : 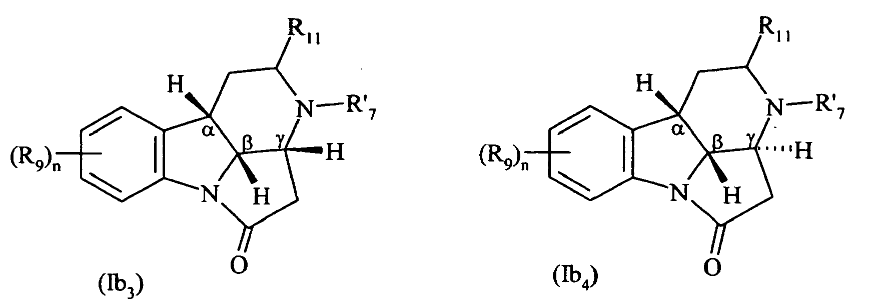
dans lesquelles R'₇, R₉, R₁₁ et n sont tels que définis précédemment,
• soit sont soumis aux mêmes conditions que les composés de formules (XXIIa) et (XXIIb) pour conduire aux composés de formule (XXVIIIa) et (XXVIIIb) : 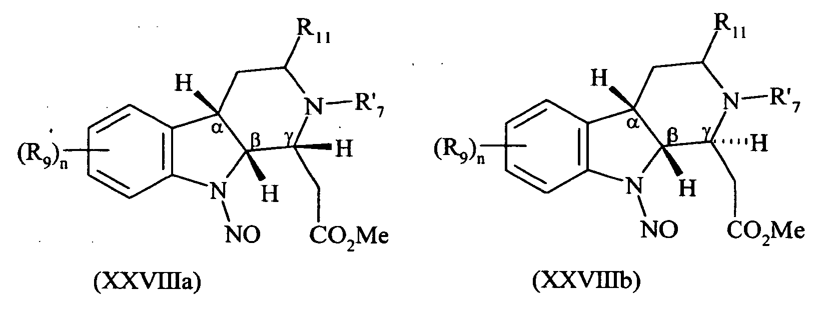
dans lesquelles R'₇, R₉, R₁₁ et n sont tels que définis précédemment,
qui sont soumis aux mêmes conditions que les composés de formules (XXIIIa) et (XXIIIb) pour conduire aux composés de formules (I_{b5}) et (I_{b6}), cas particulier des composés de formule (I) : 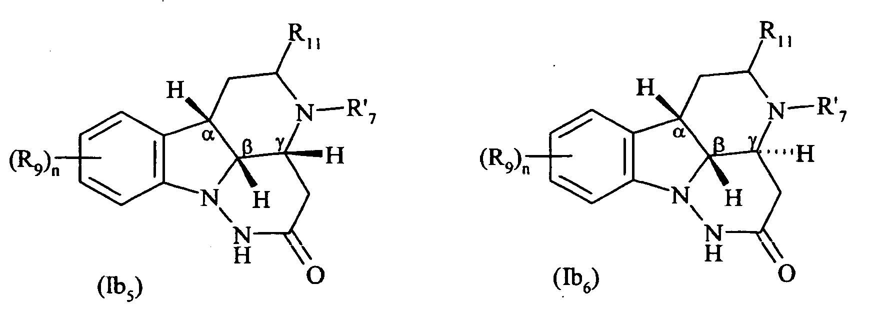 dans lesquelles R'₇, R₉, R₁₁ et n sont tels que définis précédemment,
les composés de formules (I_{b1}) à (I_{b6}) forment les composés de formule (Ib), qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

14. Compositions pharmaceutiques contenant comme principe actif au moins un composé selon l'une quelconque des revendications 1 à 10, en combinaison avec un ou plusieurs excipients ou véhicules inertes, non toxiques, pharmaceutiquement acceptables.

15. Compositions pharmaceutiques selon la revendication 14 utiles dans le traitement des dépressions, de l'anxiété, des troubles de la mémoire au cours de la sénescence et/ou de maladies neurodégénératives, et dans le traitement palliatif de la maladie de Parkinson et pour l'adaptation au stress.

## Claims

1. Compound of formula (I): 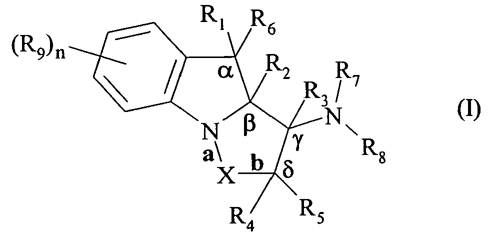 wherein:
- X represents CO or 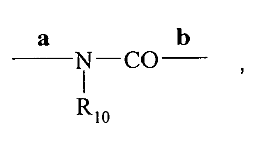
R₁ represents a hydrogen atom, a group linear or branched (C₁-C₆)alkyl, linear or branched (C₁-C₆)aminoalkyl, linear or branched (C₁-C6)hydroxyalkyl, aryl-(C₁-C₆)alkyl in which the
alkyl moiety may be linear or branched,
R₂ represents a hydrogen atom, a group linear or branched (C₁-C₆)alkyl, linear or branched
(C₁-C₆)aminoalkyl, linear or branched (C₁-C₆)hydroxyalkyl,
or R₁ and R₂, together with the carbon atoms carrying them, form a carbon-carbon bond,
R₃ represents a hydrogen atom, a group linear or branched (C₁-C₆)alkyl, linear or branched (C₁-C₆)aminoalkyl, linear or branched (C₁-C₆)hydroxyalkyl,
R₄ represents a hydrogen atom, a group linear or branched (C₁-C₆)_{alkyl}, linear or branched (C₁-C₆)aminoalkyl, linear or branched (C₁-C₆)hydroxyalkyl,
or R₃ and R₄, together with the carbon atoms carrying them, form a carbon-carbon bond,
R₅ represents a hydrogen atom, a linear or branched (C₁-C₆)alkyl group,
R₆ represents a hydrogen atom, a linear or branched (C₁-C₆)alkyl group,
R₇, R₈ represent a hydrogen atom, a group linear or branched (C₁-C₆)alkyl, aryl-(C₁-C₆)alkyl in which the alkyl moiety may be linear or branched, linear or branched (C₂-C₆)alkenyl, linear or branched (C₂-C₆)alkynyl, a linear or branched (C₁-C₆)alkyl chain substituted by one or more hydroxy, cyano, linear or branched (C₁-C₆)alkoxy, NR₁₃R₁₄, a linear or branched (C₁-C₆)alkenyl chain substituted by the same substituents as those defined for the alkyl chain, or a linear or branched (C₁-C₆)alkynyl chain substituted by the same substituents as those defined for the alkyl chain,
or,
either R₅ and R₈, together with the carbon and nitrogen atoms carrying them, form a heterocycle having 5, 6 or 7 ring members, optionally substituted by a group R₁₂,
or R₆ and R₇, together with the carbon and nitrogen atoms carrying them, form a heterocycle having 5, 6 or 7 ring members, optionally substituted by a group R₁₁,
it being understood that of necessity one, but only one, of the two groupings "R₅ and R₈" or "R₆ and R₇" together with the carbon and nitrogen atoms carrying them forms a heterocycle having 5, 6 or 7 ring members, optionally substituted by a group R₁₁,
R₉ represents hydrogen, halogen, linear or branched (C₁-C₆)alkyl, linear or branched (C₁-C₆)alkoxy, hydroxy, cyano, nitro, linear or branched (C₁-C₆)polyhaloalkyl, NR₁₅R₁₆, or a linear or branched (C₁-C₆)alkyl chain substituted by one or more halogens, one or more hydroxy, linear or branched (C₁-C₆)alkoxy, or NR₁₅R₁₆,
n represents an integer 0, 1, 2, 3 or 4,
R₁₀ represents hydrogen, linear or branched (C₁-C₆)alkyl, linear or branched (C₂-C₆)-alkenyl, aryl-(C₁-C₆)alkyl in which the alkyl moiety may be linear or branched, linear or branched (C₁-C₆)polyhaloalkyl, or a linear or branched (C₁-C₆)alkyl chain substituted by one or more halogen atoms, one or more groups hydroxy, linear or branched (C₁-C₆)-alkoxy, or NR₁₅R₁₆,
R₁₁, R₁₂, which may be identical or different, represent a -COOT or -CH₂O-U group wherein T and U, which may be identical or different, represent a hydrogen atom or a linear or branched (C₁-C₆)alkyl group,
R₁₃, R₁₄, which may be identical or different, represent a hydrogen atom or a linear or branched (C₁-C₆)alkyl group or, together with the nitrogen atom carrying them, form an optionally substituted heterocycle having from 4 to 8 ring members optionally containing a double bond in the heterocycle and optionally containing in the ring system a second hetero atom selected from an oxygen atom and a nitrogen atom,
R₁₅, R₁₆, which may be identical or different, represent a hydrogen atom or a group linear or branched (C₁-C₆)alkyl, linear or branched (C₂-C₆)alkenyl,
their enantiomers, diastereoisomers, N-oxides, and also addition salts thereof with a pharmaceutically acceptable acid or base,
wherein:
- (3aS*R*,4*SR*)-3-benzyl-4-ethyl-2,3,3a,4-tetrahydrobenzo[*b*]pyrido[2,3,4-*gh*]pyrrolizin-5(1*H*)-one,
- pyrido[3',2':4,5]pyridazino[1,6-*a*]indol-5(6*H*)-one,
- 6-methylpyrido[3',2':4,5]pyridazino[1,6-*a*]indol-5(6*H*)-one,
- 6-methyl-1,2,3,4-tetrahydropyrido[3',2':4,5]pyridazino[1,6-*a*]indol-5(6*H*)-one,
- (4a*R*,12b*R*)-6-methyl-1,2,3,4,4a,12b-hexahydropyrido[3',2':4,5]pyridazino[1,6-*a*]indol-5(6*H*)-one,
- 1-methyl-3a,10b-dihydro[1,2,3]triazolo[4',5':3,4]pyrrolo[1,2-*a*]indol-4(1*H*)-one,
- 1-benzyl-3a,10b-dihydro[1,2,3]triazolo[4',5':3,4]pyrrolo[1,2-*a*]indol-4(1*H*)-one, do not form part of the invention,
aryl means a phenyl or naphthyl group, both groups optionally being substituted by one or more halogen atoms, nitro, amino, linear or branched (C₁-C₆)alkyl or linear or branched (C₁-C₆)alkoxy groups,
a heterocycle having 5, 6 or 7 ring members means a saturated or unsaturated monocyclic group having 5, 6 or 7 sides and containing one or two hetero atoms selected from nitrogen and oxygen; pyrrolidine, piperidine, azepane and pyridine may be mentioned,
as optionally substituted heterocycle having from 4 to 8 ring members optionally containing one or more double bonds in the heterocycle and optionally containing in the ring system a second hetero atom selected from an oxygen atom and a nitrogen atom, there may be mentioned, without implying any limitation, pyrrolidine, piperidine, azepane, piperazine and morpholine, wherein those heterocycles may optionally be substituted, including on the second nitrogen atom of piperazine, by one or more identical or different groups selected from linear or branched (C₁-C₆)alkyl, linear or branched (C₁-C₆)hydroxyalkyl, linear or branched (C₁-C₆)alkoxy-(C₁-C₆)alkyl and linear or branched (C₁-C₆)aminoalkyl in which the amino moiety may optionally be substituted by one or two identical or different linear or branched (C₁-C₆)alkyl groups,
α, β, γ and δ mean the chiral centres that may be present in the compounds of formula (I). their enantiomers and diastereoisomers and also addition salts thereof with a pharmaceutically acceptable acid or base.

2. Compounds of formula (I) according to claim 1, wherein X represents CO, their enantiomers and diastereoisomers and also addition salts thereof with a pharmaceutically acceptable acid or base.

3. Compounds of formula (I) according to claim 1, wherein R₁, R₂, R₃ and R₄ each represent a hydrogen atom, their enantiomers and diastereoisomers and also addition salts thereof with a pharmaceutically acceptable acid or base.

4. Compounds of formula (I) according to claim 1, wherein R₁ and R₂, together with the carbon atoms carrying them, form a carbon-carbon bond, their enantiomers and diastereoisomers and also addition salts thereof with a pharmaceutically acceptable acid or base.

5. Compounds of formula (I) according to claim 1, wherein R₃ and R₄, together with the carbon atoms carrying them, form a carbon-carbon bond, their enantiomers and diastereoisomers and also addition salts thereof with a pharmaceutically acceptable acid or base.

6. Compounds of formula (I) according to claim 1, wherein n represents an integer 1, their enantiomers and diastereoisomers and also addition salts thereof with a pharmaceutically acceptable acid or base.

7. Compounds of formula (I) according to claim 1, wherein R₉ represents a hydrogen or halogen atom, or a linear or branched (C₁-C₆)alkyl or linear or branched (C₁-C₆)alkoxy group, their enantiomers and diastereoisomers and also addition salts thereof with a pharmaceutically acceptable acid or base.

8. Compounds of formula (I) according to claim 1, wherein R₅ and R₈, together with the carbon and nitrogen atoms carrying them, form a heterocycle having 5, 6 or 7 ring members and more especially a heterocycle having 6 ring members, their enantiomers and diastereoisomers and also addition salts thereof with a pharmaceutically acceptable acid or base.

9. Compounds of formula (I) according to claim 1, wherein R₆ and R₇, together with the carbon and nitrogen atoms carrying them, form a heterocycle having 5, 6 or 7 ring members and more especially a heterocycle having 6 ring members, their enantiomers and diastereoisomers and also addition salts thereof with a pharmaceutically acceptable acid or base.

10. Compounds of formula (I) which are:
(4a*SR*,11 a*SR*,1 1b*RS*)-1-methyl-1,2,3,4,4a,11,11a,11b-octahydropyrido[2',3':3,4]pyrrolo-[1,2-*a*]indol-5-one,
(4a*SR*,11a*RS*,11b*SR*)-1-allyl-1,2,3,4,4a,11,11a,11b-octahydropyrido[2',3':3,4]pyrrolo[1,2-*a*]indol-5-one,
(4a*SR*,11a*SR*,11 b*RS*)-1-methyl-1,2,3,4,4a,11,11a,11b-octahydropyrido[2',3':3,4]pyrrolo-[1,2-*a*]indol-5-one,
(4a*SR*,11a*SR*,11b*RS*)-1,2,3,4,4a,11,11a,11b-octahydro-1,6,6a-triazabenzo[a]fluoren-5-one,
(4a*SR*,11a*SR*,11b*RS*)-(5-oxo-3,4,4a,11,11a,11b-hexahydro-2*H*,5*H*-pyrido[2',3':3,4]-pyrrolo[1,2-*a*]indol-1-yl)acetonitrile,
(3a*SR*,6a*RS*,10c*RS*)-4-propyl-(3a,4,5,6,6a,10c-hexahydro)-3*H*-1,4,10b-triazafluoranthen-2-one,
(3a*RS*,6a*SR*,10c*RS*)-4-propyl-(3a,4,5,6,6a,10c-hexahydro)-3*H*-1,4,10b-triazafluoranthen-2-one,
(4a*SR*,11a*RS*,11b*SR*)-1-allyl-9-methoxy-1,2,3,4,4a,11,11 1a,11b-octahydropyrido[2',3':3,4]-pyrrolo[1,2-*a*]indol-5-one,
(4a*SR*,11a*SR*,11b*RS*)-9-fluoro-1,2,3,4,4a,11,11a,11b-octahydropyrido[2',3':3,4]pyrrolo[1,2-*a*]indol-5-one,
(4a*SR*,11a*SR*,11b*RS*)-9-chloro-1-methyl-1,2,3,4,4a,11,11a,11b-octahydropyrido-[2',3':3,4]pyrrolo[ 1,2-*a*]indol-5-one,
1,9-dimethyl-1,2,3,4-tetrahydropyrido[2',3':3,4]pyrrolo[ 1,2-*a*]indol-5-one,
(11a*RS*)-1,9-dimethyl-1,2,3,4,11,11a-hexahydropyrido[2',3':3,4]pyrrolo[1,2-*a*] indol-5-one,
(4a*S*,11a*R*,11b*S*)-1-allyl-1,2,3,4,4a,11,11a,11b-octahydropyrido[2',3':3,4]pyrrolo[1,2-*a*]-indol-5-one,
(4a*R*,11a*S*,11b*R*)-1-allyl-1,2,3,4,4a,11,11a,11b-octahydropyrido[2',3':3,4]pyrrolo[1,2-*a*]-indol-5-one,
(3a*RS*,10b*SR*,10c*RS*)-3-benzyl-2,3,3a,4,10b,10c-hexahydrobenzo[b]pyrido[2,3,4-*gh*]-pyrrolizin-5(1*H*)-one,
(4a*S*,11a*S*,11b*R*)-1-methyl-1,2,3,4,4a,11,11 a,11b-octahydropyrido[2',3':3,4]pyrrolo[1,2-*a*]indol-5-one,
(4a*SR*,11a*RS*,11b*SR*)-1,2,3,4,4a,11,11a,11b-octahydropyrido[2',3':3,4]pyrrolo[1,2-*a*]indol-5-one,
(4a*R*,11a*R*,11b*S*)-1-methyl-1,2,3,4,4a,11,11 a,11b-octahydropyrido[2',3':3,4]pyrrolo[1,2-*a*]indol-5-one,
their enantiomers and diastereoisomers and also addition salts thereof with a pharmaceutically acceptable acid or base.

11. Process for the preparation of compounds of formula (I), **characterised in that** there is used as starting material a compound of formula (II): 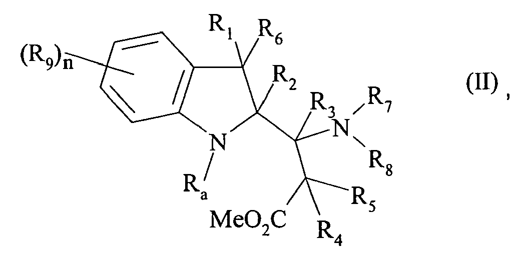 wherein R₁, R₂, R₃, R₄, R₅, R₆, R₇, R₈, R₉ and n are as defined for formula (I), Rₐ represents a hydrogen atom, a nitroso, amino or *tert*-butyl carboxylate group,
which compound of formula (II) is subjected to a cyclisation reaction in basic medium to yield compounds of formula (I), which may be purified according to a conventional separation technique, are converted, if desired, into their addition salts with a pharmaceutically acceptable acid or base and are optionally separated into their isomers according to a conventional separation technique.

12. Process for the preparation of compounds of formula (I/a), a particular case of the compounds of formula (I): 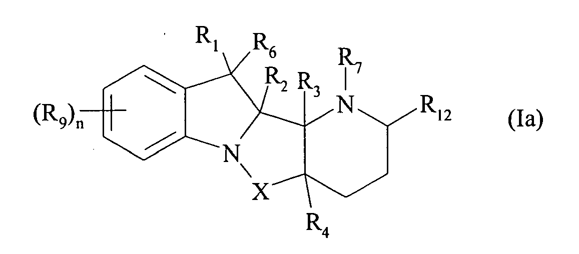 wherein R₁, R₂, R₃, R₄, R₆, R₇, R₉, R₁₂ and n are as defined for formula (I),
**characterised in that** there is used as starting material a compound of formula (III): 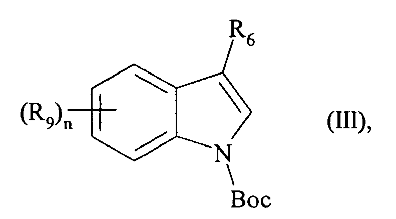 wherein R₆, R₉ and n are as defined for formula (I) and Boc represents a *tert*-butyl carboxylate group, which is reacted with triisopropyl borate in anhydrous medium to yield a compound of formula (IV): 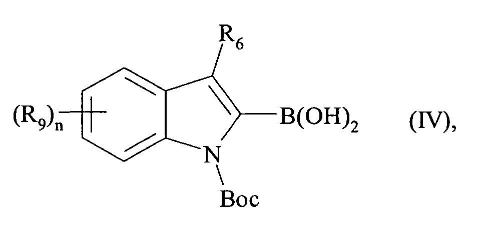 wherein R₆, R₉, n and Boc are as defined hereinbefore, which is reacted with a compound of formula (V): 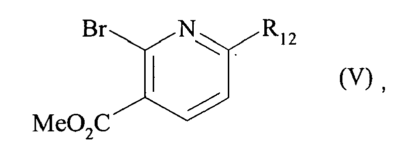 wherein R₁₂ is as defined for formula (I),
in the presence of a base and tetrakis(triphenylphosphine)palladium to yield a compound of formula (VI): 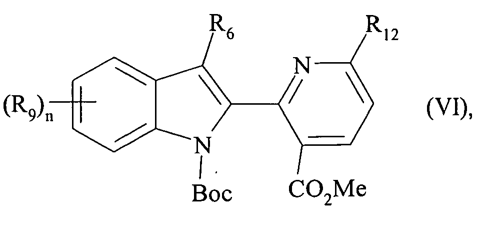 wherein R₆, R₉, R₁₂, n and Boc are as defined hereinbefore,
which compound of formula (VI) is subjected,
- either to an alkylation reaction with a compound of formula (VII):
R'₇-Hal (VII),
wherein R'₇ has all of the meanings given for R₇ as defined for formula (I) with the exception of a hydrogen atom, and Hal represents a halogen atom,
to yield a compound of formula (VIII): 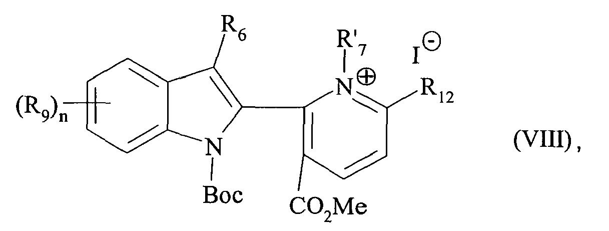 wherein R₆, R'₇, R₉, R₁₂, n and Boc are as defined hereinbefore,
which is subjected to a partial reduction reaction with hydrogen in the presence of triethylamine and platinum oxide to yield a compound of formula (IX): 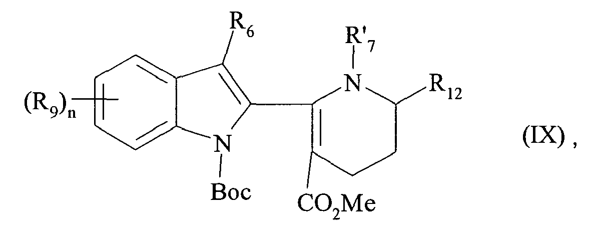 wherein R₆, R'₇, R₉, R₁₂, n and Boc are as defined hereinbefore,
which is subjected to reduction in the presence of hydrochloric acid and sodium cyanoborohydride to yield a compound of formula (X) having *trans* stereochemistry: 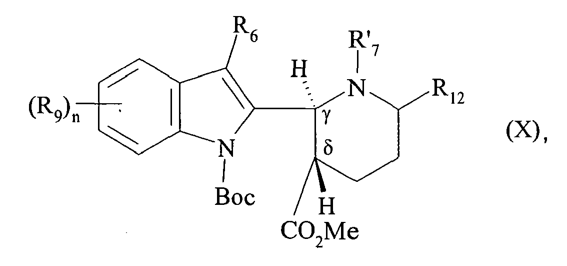 wherein R₆, R'₇, R₉, R₁₂, n and Boc are as defined hereinbefore, γ and δ are as defined for formula (I) and Hγ and Hδ have *trans* stereochemistry, which is subjected to a deprotection reaction in the presence of trifluoroacetic acid to yield a compound of formula (XI), 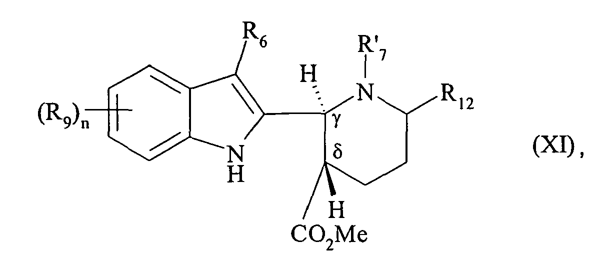 wherein R₆, R'₇, R₉, R₁₂ and n are as defined hereinbefore,
which is subjected to a cyclisation reaction in the presence of a borane-THF complex and trifluoroacetic acid to yield a compound of formula (Iₐ₁), a particular case of the compounds of formula (I): 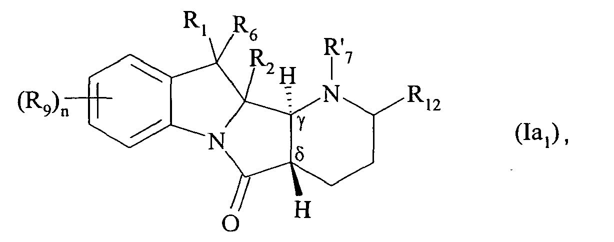 wherein R₆, R'₇, R₉, R₁₂ and n are as defined hereinbefore and R₁ and R₂ are as defined for formula (I),
- or to a reduction reaction with hydrogen in the presence of acetic acid and a rhodium catalyst to yield compounds of formulae (XIIa) and (XIIb) having (*trans, cis*) and (*cis,cis)* stereochemistry, respectively: 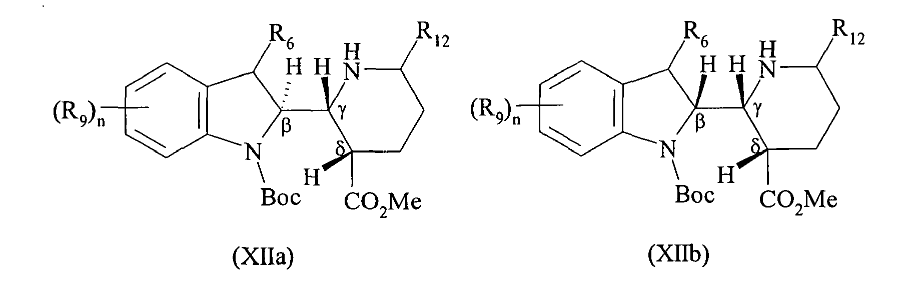
wherein R₆, R₉, R₁₂, n and Boc are as defined hereinbefore, β, γ and δ are as defined for formula (I) and H_{β} and H_{γ} have *trans* stereochemistry in the compound of formula (XIIa) and *cis* stereochemistry in the compound of formula (XIIb) and H_{γ} and H_{δ} have *cis* stereochemistry in the compounds of formulae (XIIa) and (XIIb),
which compounds of formulae (XIIa) and (XIIb) are:
• reacted with a compound of formula (VII) as defined hereinbefore, in the presence of a base, to yield compounds of formulae (XIIIa) and (XIIIb): 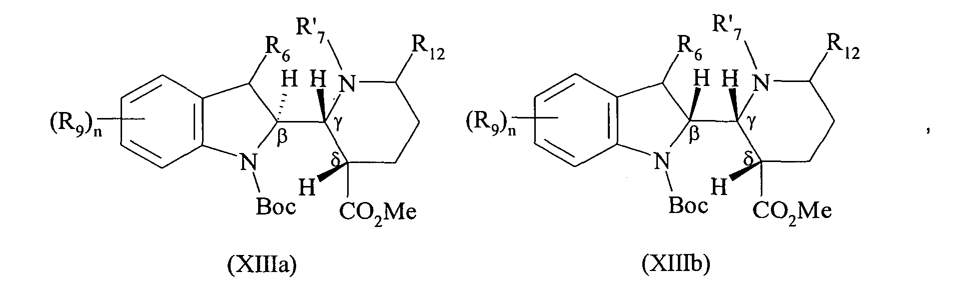
wherein R₆, R'₇, R₉, R₁₂, n and Boc are as defined hereinbefore,
which compounds of formulae (XIIIa) and (XIIIb) are:
□ placed in the presence of trifluoroacetic acid to yield compounds of formulae (Iₐ₂) and (XIVb), compounds of formula (Iₐ₂) being a particular case of the compounds of formula (I): 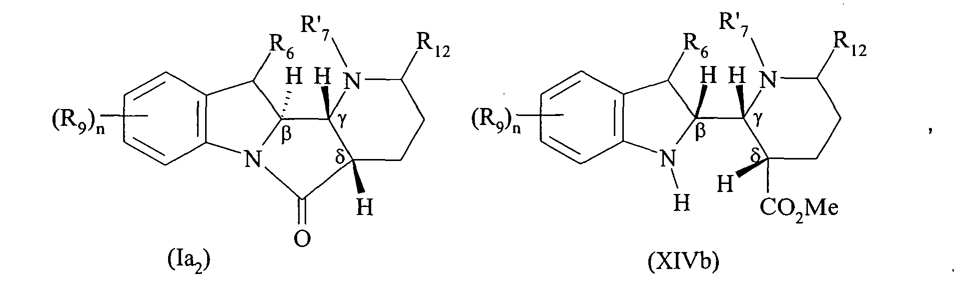
wherein R₆, R'₇, R₉, R₁₂, and n are as defined hereinbefore,
which compound of formula (XIVb) is subjected to a cyclisation reaction in the presence of sodium hydride to yield a compound of formula (Iₐ₃), a particular case of the compounds of formula (I): 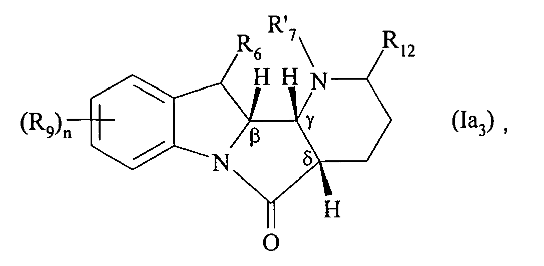 wherein R₆, R'₇, R₉, R₁₂, and n are as defined hereinbefore,
or which compound of formula (XIIIa) is:
□ alternatively placed in the presence of sodium methanolate in methanol to yield a compound of formula (XV): 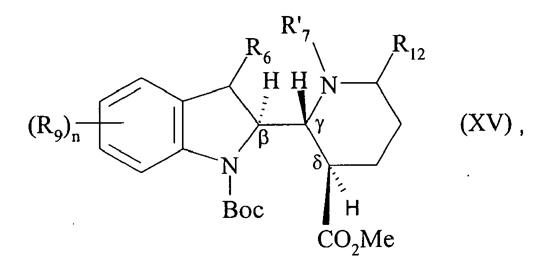
wherein R₆, R'₇, R₉, R₁₂, n and Boc are as defined hereinbefore,
which is subjected to a cyclisation reaction in the presence of trifluoroacetic acid to yield a compound of formula (Iₐ₄), a particular case of the compounds of formula (I): 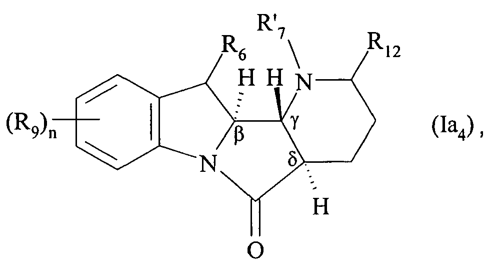 wherein R₆, R'₇, R₉, R₁₂ and n are as defined hereinbefore,
or which compound of formula (XIIa) is:
• alternatively subjected to the same conditions as the compounds of formulae (XIIIa) and (XIIIb) to yield a compound of formula (XVI): 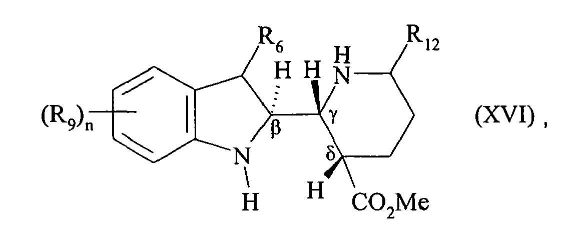
wherein R₆, R₉, R₁₂ and n are as defined hereinbefore,
which compound of formula (XVI) is:
* dissolved in a mixture of acetic acid and water and a sodium nitrite solution to yield a compound of formula (XVII): 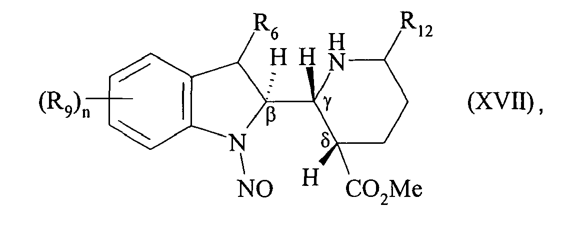
wherein R₆, R₉, R₁₂ and n are as defined hereinbefore,
which is reacted in succession with a compound of formula (VII) as defined hereinbefore, then zinc and ammonium carbonate to yield a compound of formula (Iₐ₅), a particular case of the compounds of formula (I): 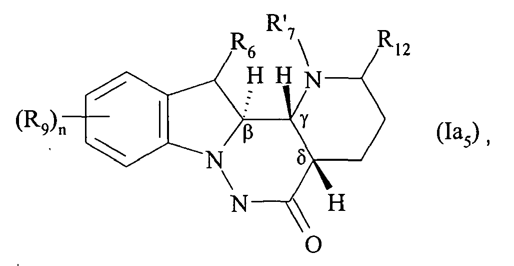 wherein R₆, R'₇, R₉, R₁₂ and n are as defined hereinbefore,
or which compound of formula (XVI) is:
* alternatively subjected to a cyclisation reaction in the presence of potassium carbonate to yield a compound of formula (Iₐ₆), a particular case of the compounds of formula (I): 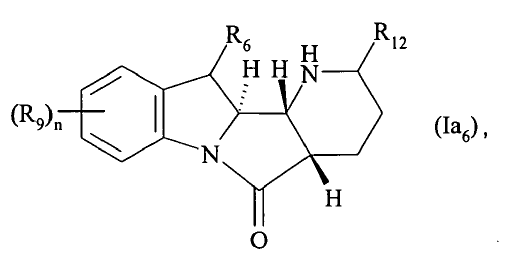
wherein R₆, R₉, R₁₂ and n are as defined hereinbefore,
which is subjected to an alkylation reaction with a compound of formula (VII) as defined hereinbefore, in basic medium, to yield a compound of formula (Iₐ₇), a particular case of the compounds of formula (I): 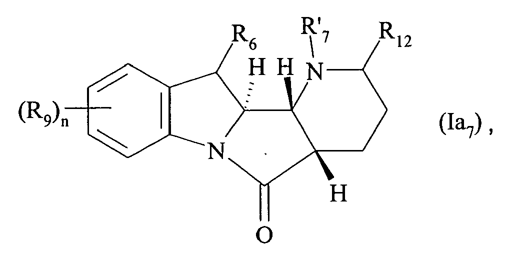 wherein R₆, R'₇, R₉, R₁₂ and n are as defined hereinbefore,
which compounds of formulae (Iₐ₁) to (Iₐ₇) constitute compounds of formula (Ia), which may be purified according to a conventional separation technique, are converted, if desired, into their addition salts with a pharmaceutically acceptable acid or base and are optionally separated into their isomers according to a conventional separation technique.

13. Process for the preparation of compounds of formula (Ib), a particular case of the compounds of formula (I): 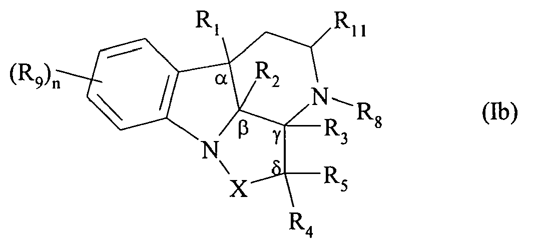 wherein R₁, R₂, R₃, R₄, R₅, R₈, R₉, R₁₁, X and n are as defined for formula (I),
**characterised in that** there is used as starting material a compound of formula (XVIII): 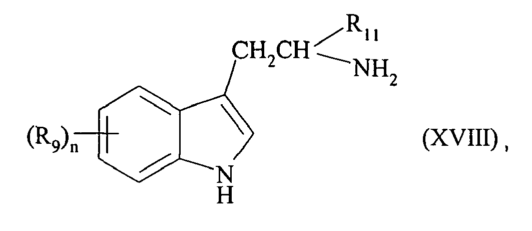 wherein R₉, R₁₁ and n are as defined hereinbefore,
which is reacted with methyl cyanoacetate in the presence of Pd/C in glacial acetic acid to yield a compound of formula (XIX): 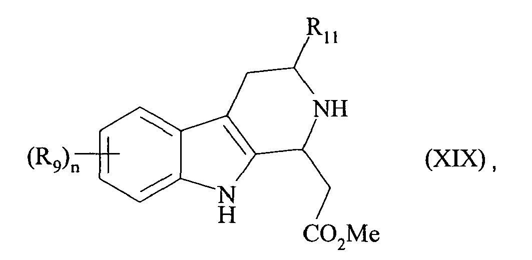 wherein R₉, R₁₁ and n are as defined hereinbefore,
which is reacted with a compound of formula (XX):
R"₇ - CHO (XX),
wherein R"₇ has all of the meanings given for R₇ as defined for formula (I) with the exception of a hydrogen atom, followed by reduction with sodium borohydride or sodium cyanoborohydride to yield a compound of formula (XXI): 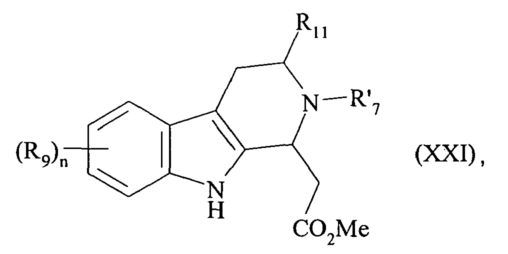 wherein R₉, R₁₁ and n are as defined hereinbefore and R'₇ has all of the meanings given for R₇ as defined for formula (I) with the exception of a hydrogen atom,
which compound of formula (XXI) is:
- reacted with a BH₃-THF complex in THF and then dissolved with trifluoroacetic acid to yield compounds of formulae (XXIIa) and (XXIIb) having (*trans, cis*) and (*trans, trans)* stereochemistry, respectively: 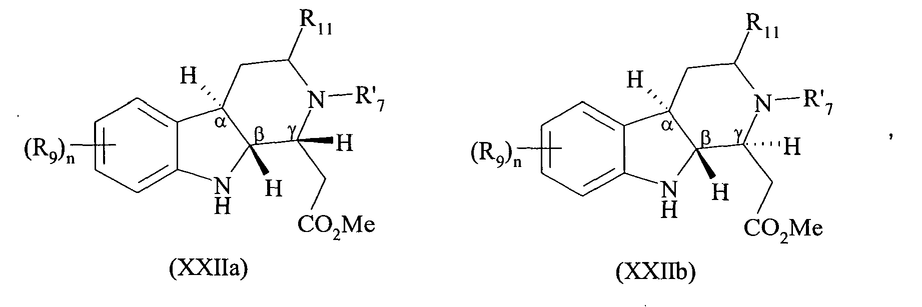
wherein R'₇, R₉, R₁₁ and n are as defined hereinbefore, and H_{α} and H_{β} have *trans* stereochemistry in the compounds of formulae (XXIIa) and (XXIIb) and H_{β} and H_{γ} have *cis* stereochemistry in the compound of formula (XXIIa) and *trans* stereochemistry in the compound of formula (XXIIb),
which are dissolved in a mixture of acetic acid and water and a sodium nitrite solution to yield compounds of formulae (XXIIIa) and (XXIIIb): 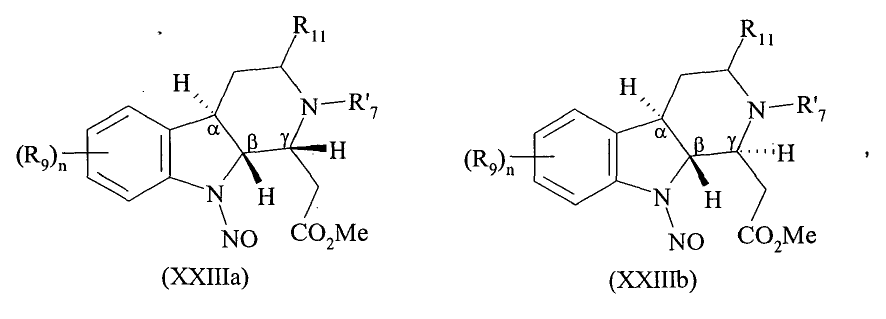 wherein R'₇, R₉, R₁₁ and n are as defined hereinbefore,
which are placed in the presence of ammonium carbonate and zinc to yield compounds of formulae (XXIVa) and (XXIVb): 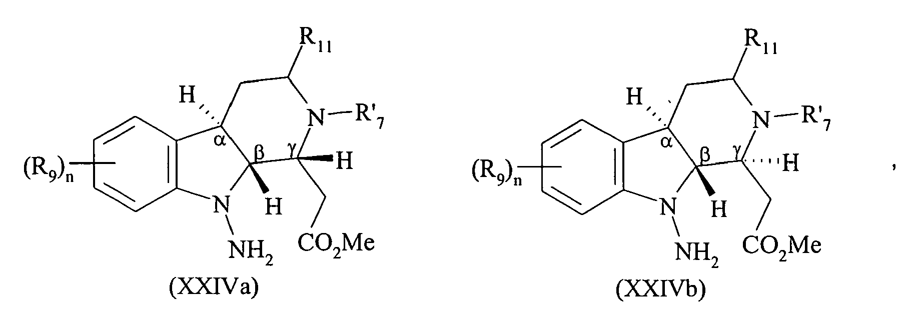 wherein R'₇, R₉, R₁₁ and n are as defined hereinbefore,
which are subjected to a cyclisation reaction in the presence of sodium hydride or trimethylaluminium dissolved in hexane to yield compounds of formulae (I_{b1}) and (I_{b2}), particular cases of the compounds of formula (I): 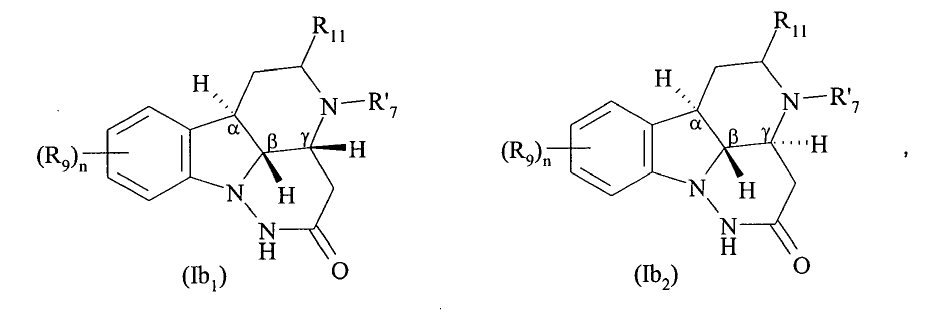 wherein R'₇, R₉, R₁₁ and n are as defined hereinbefore,
or which compound of formula (XXI) is:
- alternatively reacted with di-*tert*-butyloxycarboxylic acid anhydride and dimethylaminopyridine to yield a compound of formula (XXV): 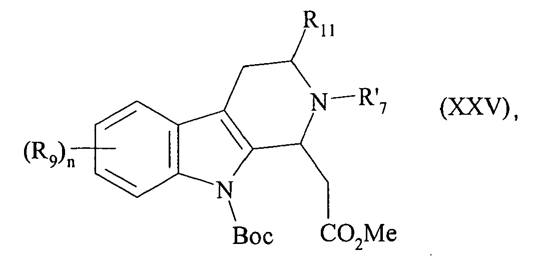
wherein R'₇, R₉, R₁₁ and n are as defined hereinbefore and Boc represents a *tert*-butyl carboxylate group, which is subjected to a reduction reaction in the presence of platinum oxide and hydrogen to yield compounds of formulae (XXVIa) and (XXVIb) having (*cis, cis*) and (*cis, trans*) stereochemistry, respectively: 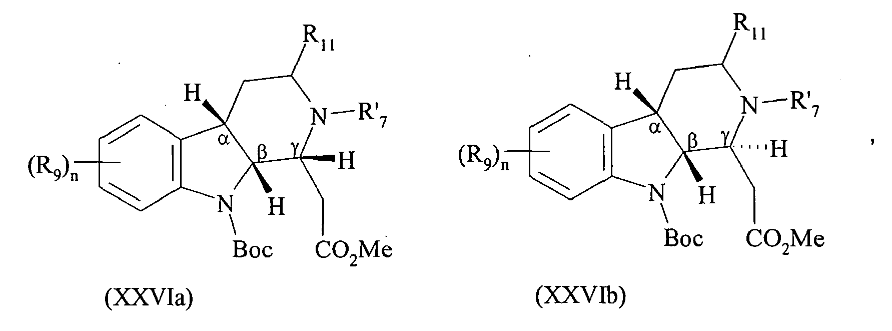 wherein R'₇, R₉, R₁₁, n and Boc are as defined hereinbefore,
and H_{α} and H_{β} have *cis* stereochemistry in the compounds of formulae (XXVIa) and (XXVIb) and H_{β} and H_{γ} have *cis* stereochemistry in the compound of formula (XXVIa) and *trans* stereochemistry in the compound of formula (XXVIb),
which are placed in the presence of trifluoroacetic acid in an anhydrous medium to yield compounds of formulae (XXVIIa) and (XXVIIb): 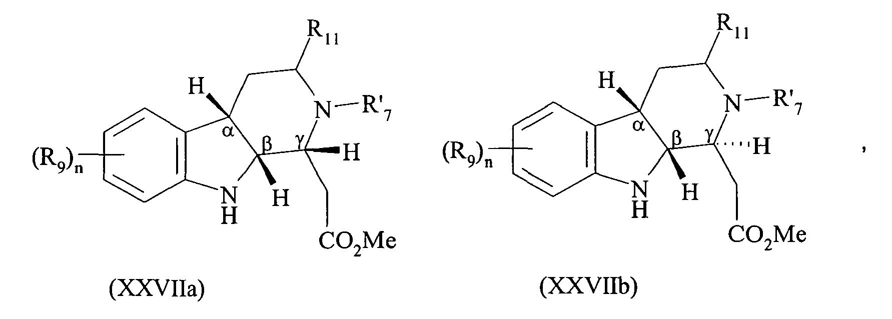 wherein R'₇, R₉, R₁₁ and n are as defined hereinbefore,
which are:
• either subjected to a cyclisation reaction under the same conditions as the compounds of formulae (XXIVa) and (XXIVb) to yield compounds of formulae (I_{b3}) and (I_{b4}), a particular case of the compounds of formula (I): 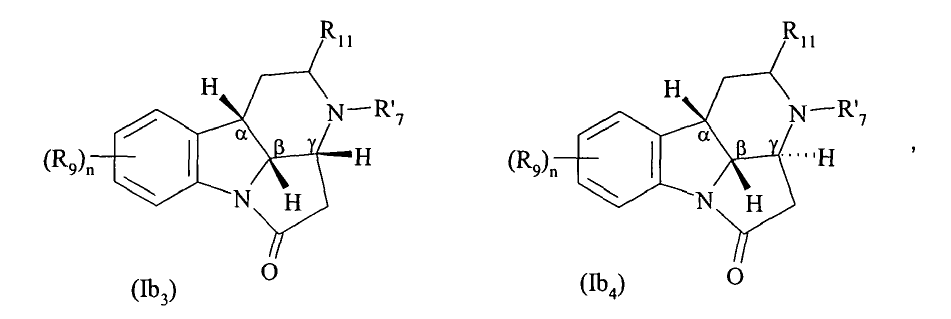
wherein R'₇, R₉, R₁₁ and n are as defined hereinbefore,
• or subjected to the same conditions as the compounds of formulae (XXIIa) and (XXIIb) to yield compounds of formula (XXVIIIa) and (XXVIIIb): 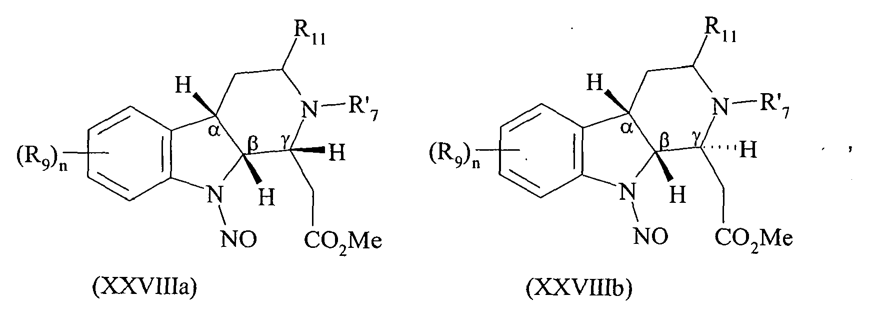
wherein R'₇, R₉, R₁₁ and n are as defined hereinbefore,
which are subjected to the same conditions as the compounds of formulae (XXIIIa) and (XXIIIb) to yield compounds of formulae (I_{b5}) and (I_{b6}), a particular case of the compounds of formula (I): 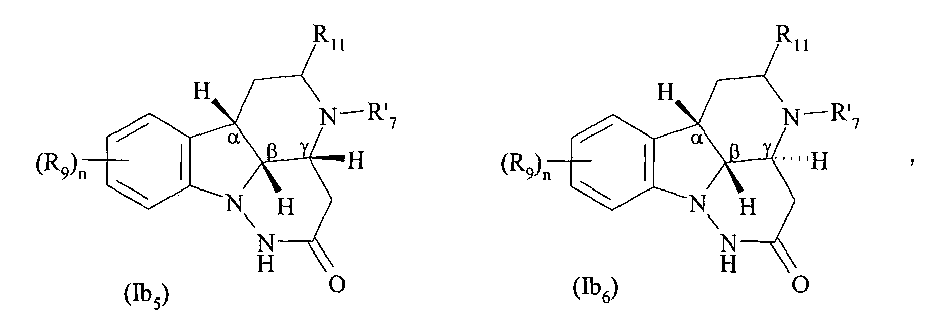 wherein R'₇, R₉, R₁₁ and n are as defined hereinbefore,
which compounds of formulae (I_{b1}) to (I_{b6}) constitute the compounds of formula (Ib), which may be purified according to a conventional separation technique, are converted, if desired, into their addition salts with a pharmaceutically acceptable acid or base and are optionally separated into their isomers according to a conventional separation technique.

14. Pharmaceutical compositions comprising as active ingredient at least one compound according to any one of claims 1 to 10, in combination with one or more pharmaceutically acceptable, inert, non-toxic excipients or carriers.

15. Pharmaceutical compositions according to claim 14 for use in the treatment of depression, anxiety, disorders of memory in the course of ageing and/or neurodegenerative diseases, and in the palliative treatment of Parkinson's disease, and for adaptation to stress.

## Patentansprüche

1. Verbindung der Formel (I): 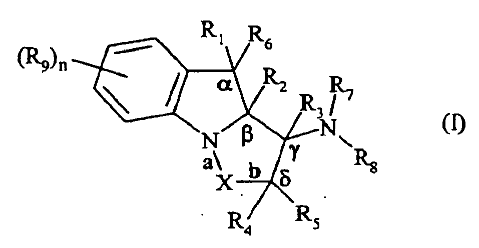 in der:
- X CO oder 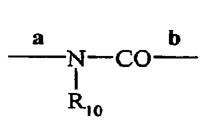 bedeutet,
R₁ ein Wasserstoffatom, eine geradkettige oder verzweigte (C₁-C₆)-Alkylgruppe, geradkettige oder verzweigte (C₁-C₆)-Aminoalkylgruppe, geradkettige oder verzweigte (C₁-C₆)-Hydroxyalkylgruppe oder geradkettige oder verzweigte Aryl- (C₁-C₆)-alkylgruppe bedeutet,
R₂ ein Wasserstoffatom, eine geradkettige oder verzweigte (C₁-C₆)-Alkylgruppe, geradkettige oder verzweigte (C₁-C₆)-Aminoalkylgruppe oder geradkettige oder verzweigte (C₁-C₆)-Hydroxyalkylgruppe bedeutet,
oder R₁ und R₂ gemeinsam mit den sie tragenden Kohlenstoffatomen eine Kohlenstoff-Kohlenstoff-Bindung bilden,
R₃ ein Wasserstoffatom, eine geradkettige oder verzweigte (C₁-C₆)-Alkylgruppe, geradkettige oder verzweigte (C₁-C₆)-Aminoalkylgruppe oder geradkettige oder verzweigte (C₁-C₆)-Hydroxyalkylgruppe bedeutet,
R₄ ein Wasserstoffatom, eine geradkettige oder verzweigte (C₁-C₆)-Alkylgruppe, geradkettige oder verzweigte (C₁-C₆)-Aminoalkylgruppe oder geradkettige oder verzweigte (C₁-C₆)-Hydroxyalkylgruppe bedeutet,
oder R₃ und R₄ gemeinsam mit den sie tragenden Kohlenstoffatomen eine Kohlenstoff-Kohlenstoff-Bindung bilden,
R₅ ein Wasserstoffatom oder eine geradkettige oder verzweigte (C₁-C₆)-Alkylgruppe bedeutet,
R₆ ein Wasserstoffatom oder eine geradkettige oder verzweigte (C₁-C₆)-Alkylgruppe bedeutet,
R₇ und R₈ ein Wasserstoffatom, eine geradkettige oder verzweigte (C₁-C₆)-Alkylgruppe, geradkettige oder verzweigte Aryl-(C₁-C₆)-alkylgruppe, geradkettige oder verzweigte (C₂-C₆)-Alkenylgruppe, geradkettige oder verzweigte (C₂-C₆)-Alkinylgruppe, eine geradkettige oder verzweigte (C₁-C₆)-Alkylkette, die durch eine oder mehrere Hydroxygruppen, Cyanogruppen, geradkettige oder verzweigte (C₁-C₆)-Alkoxygruppen oder Gruppen NR₁₃R₁₄ substituiert ist, eine geradkettige oder verzweigte (C₁-C₆)-Alkenylkette, die durch die gleichen Substituenten wie sie für die Alkylkette definiert worden sind, substituiert ist, oder eine geradkettige oder verzweigte (C₁-C₆)-Alkinylkette, die durch die gleichen Substituenten wie sie für die Alkylkette definiert worden sind, substituiert ist, bedeuten,
oder
entweder R₅ und R₈ gemeinsam mit den sie tragenden Kohlenstoff- und Stickstoffatomen einen Heterocyclus mit 5, 6 oder 7 Kettengliedern bilden, der gegebenenfalls durch eine Gruppe R₁₂ substituiert ist,
oder R₆ und R₇ gemeinsam mit den sie tragenden Kohlenstoff- und Stickstöffatomen einen Heterocyclus mit 5, 6 oder 7 Kettengliedern bilden, der gegebenenfalls durch eine Gruppe R₁₁ substituiert ist,
mit der Maßgabe, dass notwendigerweise eine, jedoch nur eine der beiden Gruppen "R₅ und R₈" oder "R₆ und R₇" zusammen mit den sie tragenden Kohlenstoff- und Stickstoffatomen einen Heterocyclus mit 5, 6 oder 7 Kettengliedern bilden, der gegebenenfalls durch eine Gruppe R₁₁ substituiert ist,
R₉ Wasserstoff, Halogen, geradkettiges oder verzweigtes (C₁-C₆)-Alkyl, geradkettiges oder verzweigtes (C₁-C₆)-Alkoxy, Hydroxy, Cyano, Nitro, geradkettiges oder verzweigtes (C₁-C₆)-Polyhalogenalkyl, NR₁₅R₁₆ oder eine geradkettige oder verzweigte (C₁-C₆)-Alkylkette, die durch ein oder mehrere Halogenatome oder eine oder mehrere Hydroxygruppen, geradkettige oder verzweigte (C₁-C₆)-Alkoxygruppen oder Gruppen NR₁₅R₁₆ substituiert ist, bedeutet,
n eine ganze Zahl mit einem Wert von 0, 1, 2, 3 oder 4 bedeutet,
R₁₀ Wasserstoff, geradkettiges oder verzweigtes (C₁-C₆)-Alkyl, geradkettiges oder verzweigtes (C₂-C₆)-Alkenyl, geradkettiges oder verzweigtes Aryl-(C₁-C₆)-alkyl, geradkettiges oder verzweigtes (C₁-C₆)-Polyhalogenalkyl oder eine geradkettige oder verzweigte (C₁-C₆)-Alkylkette, die durch ein oder mehrere Halogenatome oder eine oder mehrere Hydroxygruppen, geradkettige oder verzweigte (C₁-C₆)-Alkoxygruppen oder Gruppen NR₁₅R₁₆ substituiert ist, bedeutet,
R₁₁ und R₁₂, die identisch oder verschieden sind, eine Gruppe -COOT oder -CH₂O-U bedeuten, worin T und U, die identisch oder verschieden sind, ein Wasserstoffatom oder eine geradkettige oder verzweigte (C₁-C₆)-Alkylgruppe bedeuten,
R₁₃ und R₁₄, die identisch oder verschieden sind, ein Wasserstoffatom oder eine geradkettige oder verzweigte (C₁-C₆)-Alkylgruppe bedeuten oder gemeinsam mit dem sie tragenden Stickstoffatom einen gegebenenfalls substituierten Heterocyclus mit 4 bis 8 Kettengliedern bilden, der gegebenenfalls eine Doppelbindung im Bereich des Heterocyclus aufweist und gegebenenfalls in dem cyclischen System ein zweites Heteroatom aufweist ausgewählt aus dem Sauerstoffatom und dem Stickstoffatom,
R₁₅ und R₁₆, die identisch oder verschieden sind, ein Wasserstoffatom oder eine geradkettige oder verzweigte (C₁-C₆)-Alkylgruppe oder eine geradkettige oder verzweigte (C₂-C₆)-Alkenylgruppe bedeuten,
deren Enantiomere, Diastereoisomere, N-Oxide sowie ihre Additionssalze mit einer pharmazeutisch annehmbaren Säure oder Base,
mit der Maßgabe, dass:
- (3a*SR*,4*SR*)-3-Benzyl-4-ethyl-2,3,3a,4-tetrahydrobenzo[b]pyrido[2,3,4-*gh*]pyrrolizin-5(1*H*)-on,
- Pyrido[3',2':4,5]pyridazino[1,6-a]indol-5(6*H*)-on,
- 6-Methylpyrido[3',2':4,5]pyridazino[1,6-*a*]indol-5(6*H*)-on,
- 6-Methyl-1,2,3,4-tetrahydropyrido[3',2':4,5]pyridazino[1,6-a]indol-5(6*H*)-on,
- (4a*R*,12b*R*)-6-Methyl-1,2,3,4,4a,12b-hexahydropyrido[3',2':4,5]pyridazino[1,6-*a*]indol-5(6*H*)-on,
- 1-Methyl-3a,10b-dihydro[1,2,3]triazolo[4',5':3,4]pyrrolo[1,2-*a*]indol-4(1*H*)-on,
- 1-Benzyl-3a,10b-dihydro[1,2,3]triazolo[4',5':3,4]pyrrolo[1,2-*a*]indol-4(1*H*)-on nicht Gegenstand der Erfindung sind,
man unter Aryl eine Phenyl- oder Naphthylgruppe versteht, welche beiden Gruppen gegebenenfalls durch ein oder mehrere Halogenatome, Nitrogruppen, Aminogruppen, geradkettige oder verzweigte (C₁-C₆)-Alkylgruppen oder geradkettige oder verzweigte (C₁-C₆)-Alkoxygruppen substituiert sind,
man unter einem Heterocyclus mit 5, 6 oder 7 Kettengliedern eine monocyclische gesättigte oder ungesättigte Gruppe mit 5, 6 oder 7 Ringgliedern versteht, der ein oder zwei Heteroatome ausgewählt aus Stickstoff und Sauerstoff aufweist, worunter man Pyrrolidin, Piperidin, Azepan und Pyridin nennen kann,
man als gegebenenfalls substituierten Heterocyclus mit 4 bis 8 Kettengliedern, der gegebenenfalls eine oder mehrere Doppelbindungen im Bereich des Heterocyclus aufweist und gegebenenfalls im cyclischen System ein zweites Heteroatom ausgewählt aus dem Sauerstoffatom oder dem Stickstoffatom enthält, in nicht einschränkender Weise Pyrrolidin, Piperidin, Azepan, Piperazin und Morpholin nennen kann, wobei diese Heterocyclen gegebenenfalls substituiert sein können, auch am zweiten Stickstoffatom des Piperazins durch eine oder mehrere identische oder verschiedene Gruppen ausgewählt aus geradkettigem oder verzweigtem (C₁-C₆)-Alkyl, geradkettigem oder verzweigtem (C₁-C₆)-Hydroxyalkyl, geradkettigem oder verzweigtem (C₁-C₆)-Alkoxy-(C₁-C₆)-alkyl oder geradkettigem oder verzweigtem (C₁-C₆)-Aminoalkyl, wobei der Aminoteil gegebenenfalls durch eine oder mehrere identische oder verschiedene, geradkettige oder verzweigte (C₁-C₆)-Alkylgruppen substituiert sein kann,
man unter α, β, γ und δ die gegebenenfalls an der Verbindung der Formel (I) vorhandenen chiralen Zentren versteht,
deren Enantiomere und Diastereoisomere sowie deren Additionssalze mit einer pharmazeutisch annehmbaren Säure oder Base.

2. Verbindungen der Formel (I) nach Anspruch 1, worin X CO bedeutet, deren Enantiomere und Diastereoisomere sowie deren Additionssalze mit einer pharmazeutisch annehmbaren Säure oder Base.

3. Verbindungen der Formel (I) nach Anspruch 1, worin R₁, R₂, R₃ und R₄ jeweils ein Wasserstoffatom bedeuten, deren Enantiomere und Diastereoisomere sowie deren Additionssalze mit einer pharmazeutisch annehmbaren Säure oder Base.

4. Verbindungen der Formel (I) nach Anspruch 1, worin R₁ und R₂ gemeinsam mit den sie tragenden Kohlenstoffatomen eine Kohlenstoff-Kohlenstoff-Bindung bilden, deren Enantiomere und Diastereoisomere sowie deren Additionssalze mit einer pharmazeutisch annehmbaren Säure oder Base.

5. Verbindungen der Formel (I) nach Anspruch 1, worin R₃ und R₄ gemeinsam mit den sie tragenden Kohlenstoffatomen eine Kohlenstoff-Kohlenstoff-Bindung bilden, deren Enantiomere und Diastereoisomere sowie deren Additionssalze mit einer pharmazeutisch annehmbaren Säure oder Base.

6. Verbindungen der Formel (I) nach Anspruch 1, worin n eine ganze Zahl mit einem Wert von 1 bedeutet, deren Enantiomere und Diastereoisomere sowie deren Additionssalze mit einer pharmazeutisch annehmbaren Säure oder Base.

7. Verbindungen der Formel (I) nach Anspruch 1, worin R₉ ein Wasserstoffatom, ein Halogenatom oder eine geradkettige oder verzweigte (C₁-C₆)-Alkylgruppe oder geradkettige oder verzweigte (C₁-C₆)-Alkoxygruppe bedeutet, deren Enantiomere und Diastereoisomere sowie deren Additionssalze mit einer pharmazeutisch annehmbaren Säure oder Base.

8. Verbindungen der Formel (I) nach Anspruch 1, worin R₅ und R₈ gemeinsam mit den sie tragenden Kohlenstoff- und Stickstoffatomen einen Heterocyclus mit 5, 6 oder 7 Kettengliedern bilden, insbesondere einen Heterocyclus mit 6 Kettengliedern, deren Enantiomere und Diastereoisomere sowie deren Additionssalze mit einer pharmazeutisch annehmbaren Säure oder Base.

9. Verbindungen der Formel (I) nach Anspruch 1, worin R₆ und R₇ gemeinsam mit den sie tragenden Kohlenstoff- und Stickstoffatomen einen Heterocyclus mit 5, 6 oder 7 Kettengliedern und insbesondere einen Heterocyclus mit 6 Kettengliedern bilden, deren Enantiomere und Diastereoisomere sowie deren Additionssalze mit einer pharmazeutisch annehmbaren Säure oder Base.

10. Verbindungen der Formel (I), nämlich:
(4a*SR*,11a*SR*,1 1b*RS*)-1-Methyl-1,2,3,4,4a,11,11a,11b-octahydropyrido[2',3':3,4]-pyrrolo[1,2-*a*]indol-5-on,
(4a*SR*,11a*RS*,11b*SR*)-1-Allyl-1,2,3,4,4a,11,11a,11b-octahydropyrido[2',3':3,4]-pyrrolo[1,2-*a*]indol-5-on,
(4a*SR*,11a*SR*,11b*RS*)-1-Methyl-1,2,3,4,4a,11,11a,11b-octahydropyrido[2',3':3,4]-pyrrolo[1,2-*a*]indol-5-on,
(4a*SR*,11a*SR*,11b*RS*)-1,2,3,4,4a,11,11a,11b-Octahydro-1,6,6a-triazabenzo[a]-fluoren-5-on,
(4a*SR*,11a*SR*,11b*RS*)-(5-Oxo-3,4,4a,11,11a,11b-hexahydro-2*H*,5*H*-pyrido[2',3':3,4]-pyrrolo[ 1,2-a]indol-1-yl)-acetamid,
(3a*SR*,6a*RS*,10c*RS*)-4-Propyl-(3a,4,5,6,6a, 10c-hexahydro-3*H*-1,4, 10b-triazafluoranthen-2-on,
(3a*RS*,6a*SR*,10c*RS*)-4-Propyl-(3a,4,5,6,6a, 10c-hexahydro)-3*H*-1,4, 10b-triazafluoranthen-2-on,
(4a*SR*,11a*RS*,11b*SR*)-1-Allyl-9-methoxy-1,2,3,4,4a,11,11a,11b-octahydropyrido-[2',3':3,4]pyrrolo[1,2-a]indol-5-on,
(4a*SR*,11a*SR*, 11b*RS*)-9-Fluor-1,2,3,4,4a,11, 11a,11b-octahydropyrido[2',3':3,4]-pyrrolo[1,2-*a*]indol-5-on,
(4a*SR*, 11a*SR*,11b*RS*)-9-Chlor-1-methyl-1,2,3,4,4a,11,11a,11b-octahydropyrido-[2',3':3,4]pyrrolo[1,2-a]indol-5-on,
1,9-Dimethyl-1,2,3,4-tetrahydropyrido[2',3':3,4]pyrrolo[1,2-a]indol-5-on,
(11a*RS*)-1,9-Dimethyl-1,2,3,4,11,11a-hexahydropyrido[2',3':3,4]pyrrolo[1,2-*a*]-indol-5-on,
(4a*S*,11a*R*,1b*S*)-1-Allyl-1,2,3,4,4a,11,11a,11b-octahydropyrido[2',3':3,4]-pyrrolo[1,2-*a*]indol-5-on,
(4a*R*,11a*S*,11 b*R*)-1-Allyl-11,2,3,4,4a,11,11a,11b-octahydropyrido[2',3':3,4]-pyrrolo[1,2-*a*]indol-5-on,
(3a*RS*, 10b*SR*,10c*RS*)-3-Benzyl-2,3,3a,4,10b,10c-hexahydrobenzo[b]pyrido[2,3,4-*gh*]pyrrolizin-5(1*H*)-on,
(4aS,11aS,11bR)-1-Methyl-1,2,3.4,4a,11,11a,11b-octahydropyrido[2',3':3,4]-pyrrolo[1,2-a]indol-5-on,
(4a*SR*,11a*RS*,11b*SR*)-1,2,3,4,4a,11,11a,11b-Octahydropyrido[2',3':3,4]pyrrolo[1,2-*a*]indol-5-on,
(4a*R*,11a*R*,11b*S*)-1-Methyl-1,2,3,4,4a,11,11a,11b-octahydropyrido[2',3':3,4]-pyrrolo[1,2-*a*]indol-5-on,
deren Enantiomere und Diastereoisomere sowie deren Additionssalze mit einer pharmazeutisch annehmbaren Säure oder Base.

11. Verfahren zur Herstellung der Verbindungen der Formel (I), **dadurch gekennzeichnet, dass** man als Ausgangsprodukt eine Verbindung der Formel (II) verwendet: 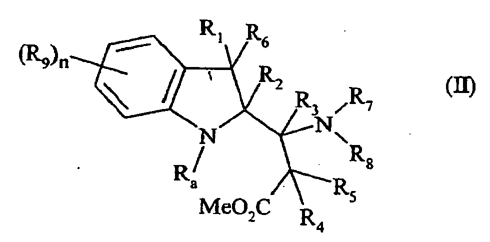 in der R₁, R₂, R₃, R₄, R₅, R₆, R₇, R₈, R₉ und n die bezüglich der Formel (I) angegebenen Bedeutungen besitzen und Rₐ ein Wasserstoffatom, eine Nitrosogruppe, eine Aminogruppe oder eine *tert*.-Butylcarboxylatgruppe darstellt,
welche Verbindung der Formel (II) man einer Cyclisierungsreaktion in basischem Medium unterwirft zur Bildung der Verbindungen der Formel (I), welche mit Hilfe einer klassischen Trennungsmethode gereinigt werden können, welche man gewünschtenfalls in ihre Additionssalze mit einer pharmazeutisch annehmbaren Säure oder Base überführt und welche man gegebenenfalls mit Hilfe einer klassischen Trennmethode in ihre Isomeren auftrennt.

12. Verfahren zur Herstellung der Verbindungen der Formel (Ia), einem Sonderfall der Verbindungen der Formel (I): 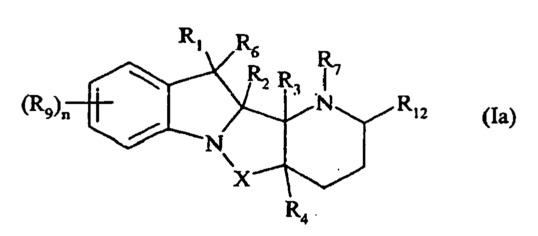 in der R₁, R₂, R₃, R₄, R₆, R₇, R₉, R₁₂ und n die bezüglich der Formel (I) angegebenen Bedeutungen besitzen,
**dadurch gekennzeichnet, dass** man als Ausgangsprodukt eine Verbindung der Formel (III) verwendet: 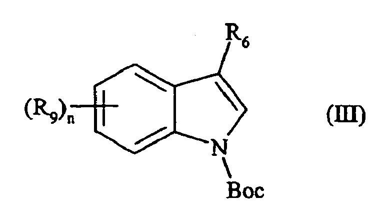 in der R₆, R₉ und n die bezüglich der Formel (I) angegebenen Bedeutungen besitzen und Boc die *tert*.-Butylcarboxylatgruppe bedeutet, welche mit Triisopropylborat in wasserfreiem Medium umgesetzt wird zur Bildung der Verbindung der Formel (IV): 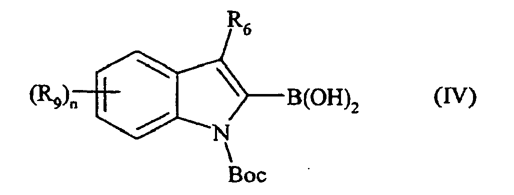 in der R₆, R₉, n und Boc die oben angegebenen Bedeutungen besitzen, welche man mit einer Verbindung der Formel (V): 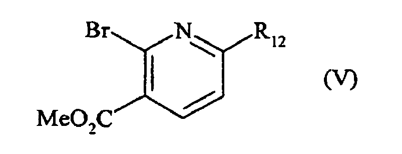 in der R₁₂ die bezüglich der Formel (I) angegebenen Bedeutungen besitzt,
in Gegenwart einer Base und von Tetrakis(triphenylphosphin)palladium umsetzt zur Bildung der Verbindung der Formel (VI): 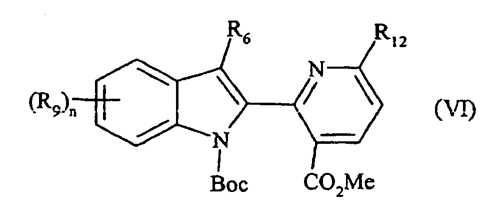 in der R_{6,} R₉, R₁₂, n und Boc die oben angegebenen Bedeutungen besitzen,
welche Verbindung der Formel (VI) man
- entweder einer Alkylierungsreaktion mit einer Verbindung der Formel (VII):
R'₇ - Hal (VII)
in der R'₇ sämtliche Bedeutungen von R₇ aufweist, wie sie bezüglich der Formel (I) definiert worden sind, mit Ausnahme des Wasserstoffatoms und Hal ein Halogenatom bedeutet, unterwirft
zur Bildung der Formel (VIII): 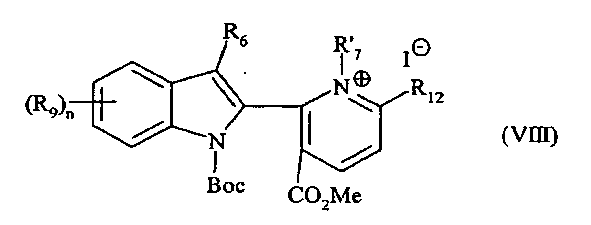 in der R₆, R'₇, R₉, R₁₂, n und Boc die oben angegebenen Bedeutungen besitzen, welche man einer partiellen Reduktionsreaktion mit Wasserstoff in Gegenwart von Triethylamin und Platinoxid unterwirft zur Bildung der Verbindung der Formel (IX): 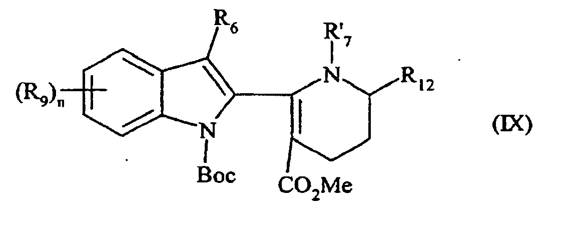 in der R₆, R'₇, R₉, R₁₂, n und Boc die oben angegebenen Bedeutungen besitzen,
welche man einer Reduktion in Gegenwart von Chlorwasserstoffsäure und Natriumcyanborhydrid unterzieht zur Bildung der Verbindung der Formel (X), in der *trans*-Stereochemie: 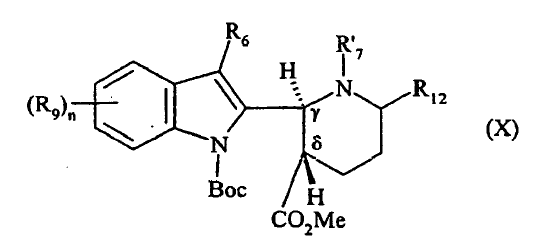 in der R₆, R'₇, R₉, R₁₂, n und Boc die oben angegebenen Bedeutungen besitzen, y und δ die bezüglich der Formel (I) angegebenen Bedeutungen besitzen und Hγ und Hδ in der *trans*-Stereochemie vorliegen, welche man einer Schutzgruppenabspaltung in Gegenwart von Trifluoressigsäure unterwirft zur Bildung der Verbindung der Formel (XI): 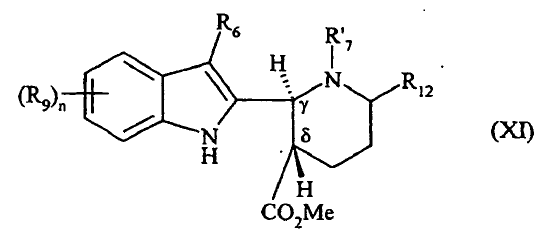 in der R₆, R'₇, R₉, R₁₂ und n die oben angegebenen Bedeutungen besitzen,
welche man einer Cyclisierungsreaktion in Gegenwart eines Boran-THF-Komplexes und von Trifluoressigsäure unterwirft zur Bildung der Verbindung der Formel (Ia₁), einem Sonderfall der Verbindungen der Formel (I): 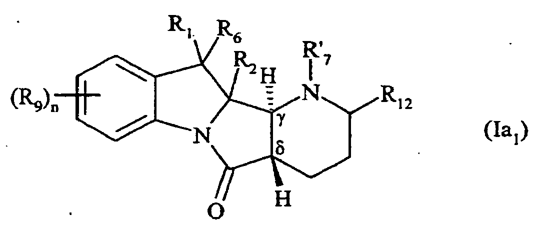 in der R₆, R'₇, R₉, R₁₂ und n die oben angegebenen Bedeutungen besitzen und R₁ und R₂ die bezüglich der Formel (I) angegebenen Bedeutungen aufweisen,
- oder einer Reduktionsreaktion mit Wasserstoff in Gegenwart von Essigsäure und eines Rhodium-Katalysators unterwirft zur Bildung der Verbindungen der Formeln (XIIa) und (XIIb) in der (*trans, cis*)- bzw. (cis,cis)-Stereochemie: 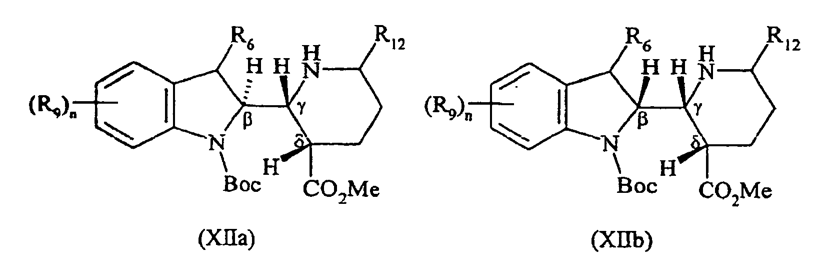
worin R₆, R₉, R₁₂, n und Boc die oben angegebenen Bedeutungen besitzen, β, γ und δ die bezüglich der Formel (I) angegebenen Bedeutungen besitzen und H_{β} und H_{γ} bei der Verbindung der Formel (XIIa) in der *trans-*Stereochemie und in der Verbindung der Formel (XIIb) in der *cis-*Stereochemie vorliegen und H_{γ} und H_{δ} bei den Verbindungen der Formeln (XIIa) und (XIIb) in der *cis-*Stereochemie vorliegen, welche Verbindungen der Formeln (XIIa) und (XIIb):
• entweder mit einer Verbindung der Formel (VII), wie sie oben definiert worden ist, in Gegenwart einer Base umgesetzt werden, zur Bildung der Verbindungen der Formeln (XIIIa) und (XIIIb): 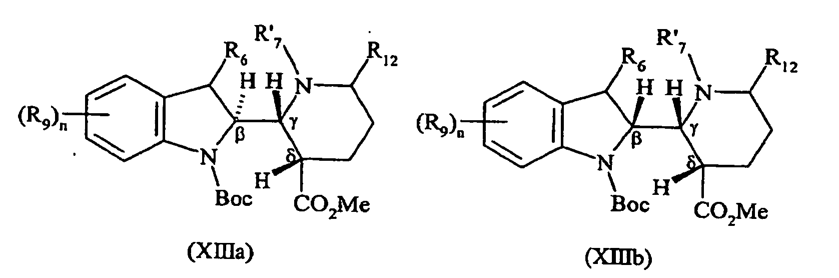
worin R₆, R'₇, R₉, R₁₂, n und Boc die oben angegebenen Bedeutungen besitzen,
welche Verbindungen der Formeln (XIIIa) und (XIIIb):
□ entweder mit Trifluoressigsäure umgesetzt werden zur Bildung der Verbindungen der Formeln (Ia₂) und (XIVb), wobei die Verbindungen der Formel (Ia₂) einen Sonderfall der Verbindungen der Formel (I) darstellen: 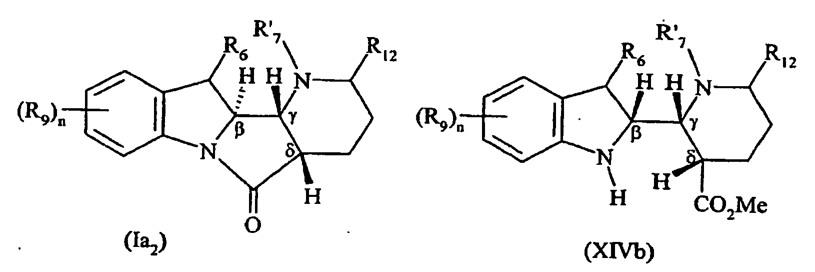
worin R₆, R'₇, R₉, R₁₂ und n die oben angegebenen Bedeutungen besitzen,
welche Verbindung der Formel (XIVb) einer Cyclisierungsreaktion in Gegenwart von Natriumhydrid unterworfen wird zur Bildung der Verbindung der Formel (Ia₃), einem Sonderfall der Verbindungen der Formel (I): 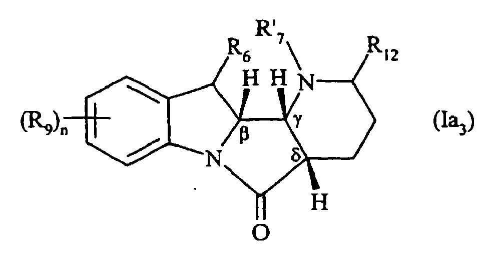 worin R₆, R'₇, R₉, R₁₂ und n die oben angegebenen Bedeutungen besitzen,
□ oder die Verbindung der Formel (XIIIa) mit Natriummethylat in Methanol umgesetzt wird zur Bildung der Verbindung der Formel (XV): 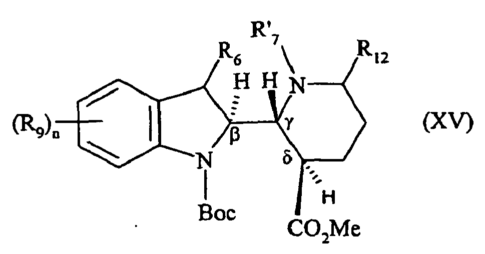
in der R₆, R'₇, R₉, R₁₂, n und Boc die oben angegebenen Bedeutungen besitzen,
welche einer Cyclisierungsreaktion in Gegenwart von Trifluoressigsäure unterworfen wird zur Bildung der Verbindung der Formel (Ia₄), einem Sonderfall der Verbindungen der Formel (I): 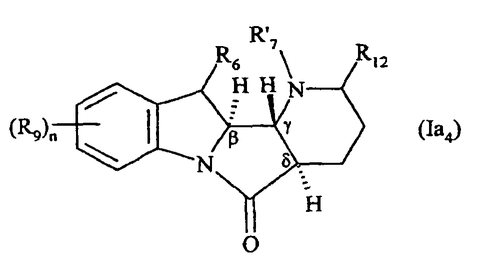 in der R₆, R'₇, R₉, R₁₂ und n die oben angegebenen Bedeutungen besitzen,
• oder die Verbindung der Formel (XIIa) den gleichen Bedingungen unterworfen wird wie die Verbindungen der Formeln (XIIIa) und (XIIIb) zur Bildung der Verbindung der Formel (XVI): 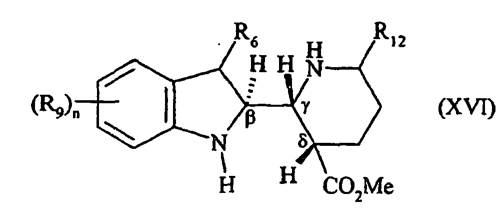
in der R₆, R₉, R₁₂ und n die oben angegebenen Bedeutungen besitzen,
welche Verbindung der Formel (XVI):
* entweder in einer Mischung aus Essigsäure und Wasser und einer Natriumnitritlösung gelöst wird zur Bildung der Verbindung der Formel (XVII): 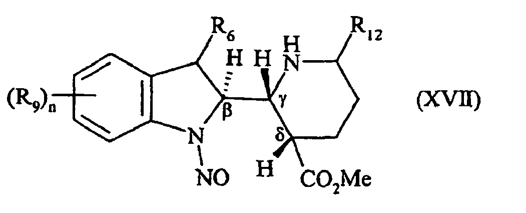 in der R₆, R₉, R₁₂ und n die oben angegebenen Bedeutungen besitzen,
welche nacheinander mit einer Verbindung der Formel (VII), wie sie oben definiert worden ist, und dann mit Zink und Ammoniumcarbonat umgesetzt wird zur Bildung der Verbindung der Formel (Ia₅), einem Sonderfall der Verbindungen der Formel (I): 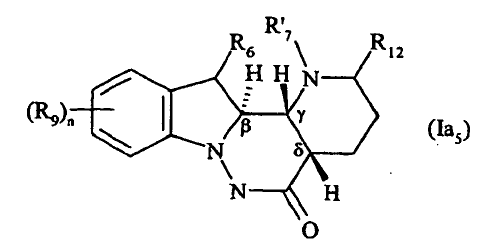 in der R₆, R'₇, R₉, R₁₂ und n die oben angegebenen Bedeutungen besitzen,
* oder die Verbindung der Formel (XVI) einer Cyclisierungsreaktion in Gegenwart von Kaliumcarbonat unterworfen wird zur Bildung der Verbindung der Formel (Ia₆), einem Sonderfall der Verbindungen der Formel (I): 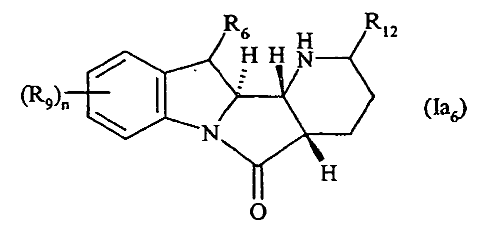 in der R₆, R₉, R₁₂ und n die oben angegebenen Bedeutungen besitzen,
welche einer Alkylierungsreaktion mit einer Verbindung der Formel (VII), wie sie oben definiert worden ist, in basischem Medium unterworfen wird zur Bildung der Verbindung der Formel (Ia₇), einem Sonderfall der Verbindungen der Formel (I): 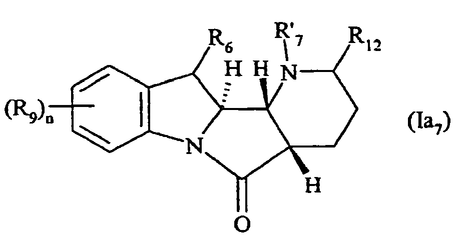 in der R₆, R'₇, R₉, R₁₂ und n die oben angegebenen Bedeutungen besitzen,
welche Verbindungen der Formeln (Ia₁) bis (Ia₇) die Verbindungen der Formel (Ia) bilden, welche mit Hilfe einer klassischen Trennmethode gereinigt werden können, welche man gewünschtenfalls in ihre Additionssalze mit einer pharmazeutisch annehmbaren Säure oder Base überführt und welche man gegebenenfalls mit Hilfe einer klassischen Trennmethode in ihre Isomeren auftrennt.

13. Verfahren zur Herstellung der Verbindungen der Formel (Ib), einem Sonderfall der Verbindungen der Formel (I): 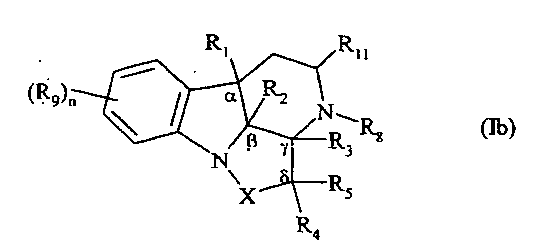 in der R₁, R₂, R₃, R₄, R₅, R₈, R₉, R₁₁, X und n die bezüglich der Formel (I) angegebenen Bedeutungen besitzen,
**dadurch gekennzeichnet, dass** man als Ausgangsprodukt eine Verbindung der Formel (XVIII) verwendet: 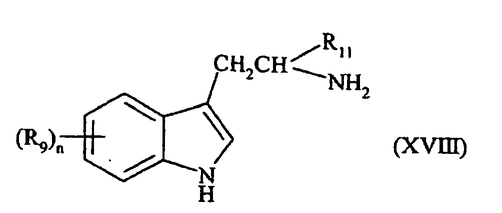 in der R₉, R₁₁ und n die oben angegebenen Bedeutungen besitzen,
welche man mit Cyanessigsäuremethylester in Gegenwart von Pd/C in Eisessig umsetzt zur Bildung der Verbindung der Formel (XIX): 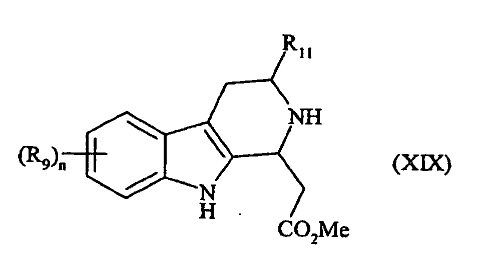 in der R₉, R₁₁ und n die oben angegebenen Bedeutungen besitzen,
welche man mit einer Verbindung der Formel (XX):
R"₇ - CHO (XX)
in der R"₇ sämtliche Bedeutungen von R₇ aufweist, wie sie bezüglich der Formel (I) definiert worden sind, mit Ausnahme des Wasserstoffatoms, umsetzt, gefolgt von einer Reduktion mit Natriumborhydrid oder Natriumcyanborhydrid zur Bildung der Verbindung der Formel (XXI): 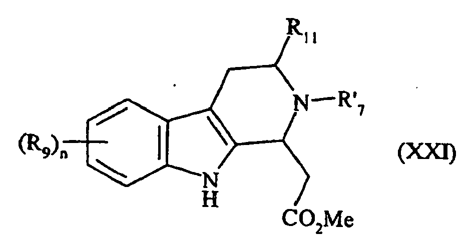 in der R₉, R₁₁ und n die oben angegebenen Bedeutungen besitzen und R'₇ sämtliche Bedeutungen von R₇ aufweist, wie sie bezüglich der Formel (I) definiert worden sind, mit Ausnahme des Wasserstoffatoms,
welche Verbindung der Formel (XXI):
- entweder mit einem BH₃-THF-Komplex in THF umgesetzt und dann in Trifluoressigsäure gelöst wird zur Bildung der Verbindungen der Formeln (XXIIa) und (XXIIb) in der (trans,cis)- bzw. (trans,trans)-Stereochemie: 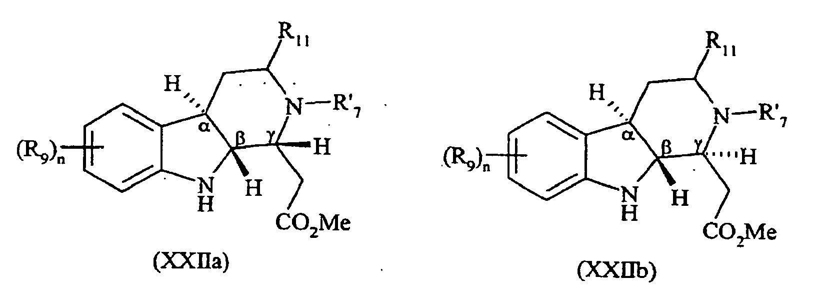
worin R'₇, R₉, R₁₁ und n die oben angegebenen Bedeutungen besitzen und H_{α} und H_{β} bei den Verbindungen der Formeln (XXIIa) und (XXIIb) in der *trans-*Stereochemie und H_{β} und H_{γ} bei der Verbindung der Formel (XXIIa) in der *cis-*Stereochemie und bei der Verbindung der Formel (XXIIb) in der *trans-*Stereochemie vorliegen, welche man in einer Mischung aus Essigsäure und Wasser und einer Natriumnitritlösung löst zur Bildung der Verbindungen der Formeln (XXIIIa) und (XXIIIb): 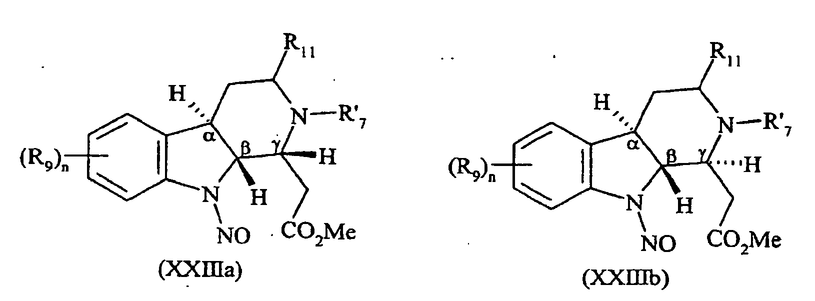 worin R'₇, R₉, R₁₁ und n die oben angegebenen Bedeutungen besitzen, welche man mit Ammoniumcarbonat und Zink umsetzt zur Bildung der Verbindungen der Formeln (XXIVa) und (XXIVb): 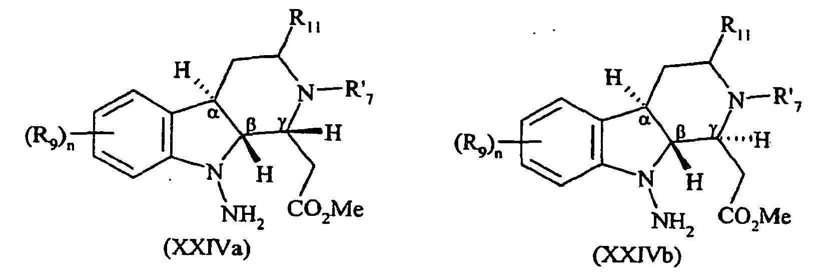 worin R'₇, R₉, R₁₁ und n die oben angegebenen Bedeutungen besitzen,
welche man einer Cyclisierungsreaktion in Gegenwart von Natriumhydrid oder Trimethylaluminium in Lösung in Hexan unterwirft zur Bildung der Verbindungen der Formeln (Ib₁) und (Ib₂), Sonderfällen der Verbindungen der Formel (I): 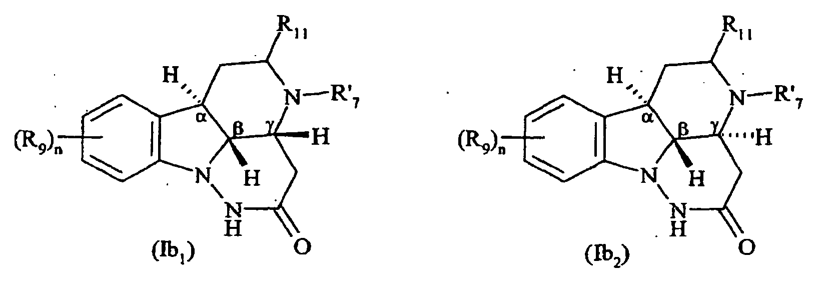 worin R'₇, R₉, R₁₁ und n die oben angegebenen Bedeutungen besitzen,
- oder welche Verbindung der Formel (XXI) mit Di-*tert*.-butyloxycarbonsäureanhydrid und Dimethylaminopyridin umgesetzt wird zur Bildung der Verbindung der Formel (XXV): 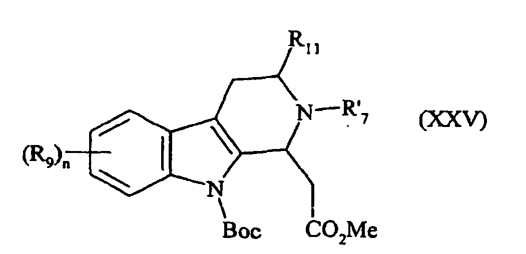
in der R'₇, R₉, R₁₁ und n die oben angegebenen Bedeutungen besitzen und Boc die *tert*.-Butylcarboxylatgruppe bedeutet, welche man einer Reduktionsreaktion in Gegenwart von Platinoxid und Wasserstoff unterwirft zur Bildung der Verbindungen der Formeln (XXVIa) und (XXVIb) in der (cis,cis)- bzw. (cis,trans)-Stereochemie: 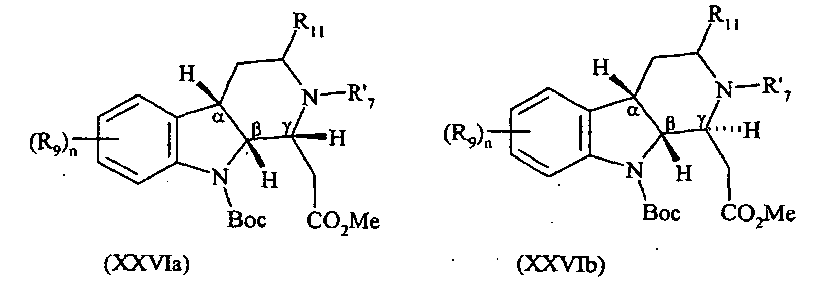 worin R'₇, R₉, R₁₁, n und Boc die oben angegebenen Bedeutungen besitzen
und H_{α} und H_{β} bei den Verbindungen der Formeln (XXVIa) und (XXVIb) in der *cis-*Stereochemie und H_{β} und H_{γ} bei der Verbindung der Formel (XXVIa) in der *cis-*Stereochemie und bei der Verbindung der Formel (XXVIb) in der *trans-*Stereochemie vorliegen,
welche mit Trifluoressigsäure in wasserfreiem Medium umgesetzt werden zur Bildung der Verbindungen der Formlen (XXVIIa) und (XXVIIb): 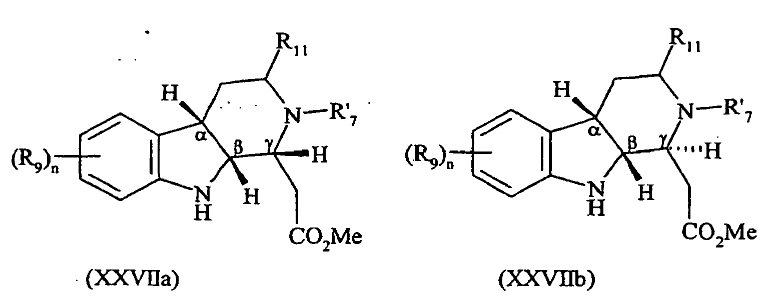 worin R'₇, R₉, R₁₁ und n die oben angegebenen Bedeutungen besitzen, die:
• entweder einer Cyclisierungsreaktion unter den gleichen Bedingungen wie die Verbindungen der Formeln (XXIVa) und (XXIVb) unterworfen werden zur Bildung der Verbindungen der Formeln (Ib₃) und Ib₄), einem Sonderfall der Verbindungen der Formel (I): 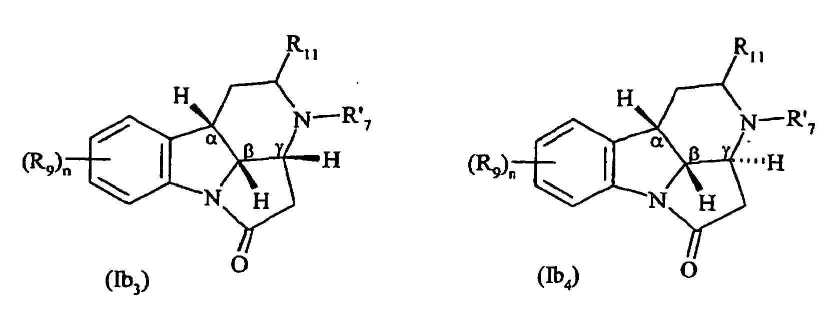
worin R'₇, R₉, R₁₁ und n die oben angegebenen Bedeutungen besitzen,
• oder den gleichen Bedingungen unterworfen werden wie die Verbindungen der Formeln (XXIIa) und (XXIIb) zur Bildung der Verbindungen der Formeln (XXVIIIa) und (XXVIIIb): 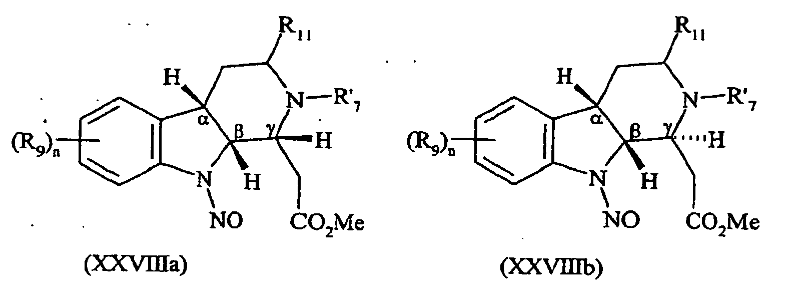
worin R'₇, R₉, R₁₁ und n die oben angegebenen Bedeutungen besitzen,
welche den gleichen Bedingungen unterworfen werden wie die Verbindungen der Formeln (XXIIIa) und (XXIIIb) zur Bildung der Verbindungen der Formeln (Ib₅) und (Ib₆), einem Sonderfall der Verbindungen der Formel (I): 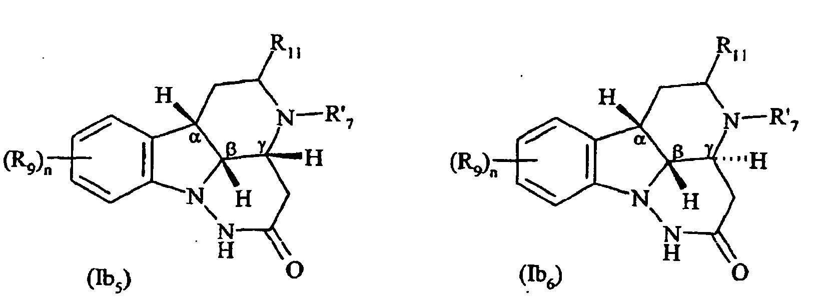 worin R'₇, R₉, R₁₁ und n die oben angegebenen Bedeutungen besitzen,
wobei die Verbindungen der Formeln (Ib₁) bis (Ib₆) die Verbindungen der Formel (Ib) bilden, welche mit Hilfe einer klassischen Trennmethode gereinigt werden können, welche man gewünschtenfalls in ihre Additionssalze mit einer pharmazeutisch annehmbaren Säure oder Base überführt und welche man gegebenenfalls mit Hilfe einer klassischen Trennmethode in ihre Isomeren auftrennt.

14. Pharmazeutische Zubereitungen enthaltend als Wirkstoff mindestens eine Verbindung nach einem der Ansprüche 1 bis 10 in Kombination mit einem oder mehreren inerten, nichttoxischen, pharmazeutisch annehmbaren Hilfsstoffen oder Trägermaterialien.

15. Pharmazeutische Zubereitungen nach Anspruch 14, nützlich zur Behandlung von Depressionen, der Angst, Gedächtnisstörungen als Folge des Alterns und/oder von neurodegenerativen Erkrankungen und zur palliativen Behandlung der Parkinsonschen Krankheit und zur Anpassung an Stress.
